(19) Europäisches Patentamt
European Patent Office
Office européen des brevets

(11) Publication number: **0 491 351 A2**

# (12) EUROPEAN PATENT APPLICATION

(21) Application number: **91121591.1**

(22) Date of filing: **17.12.91**

(51) Int. Cl.[5]: **C12N 15/13**, C07K 15/28, C12N 5/20, C12P 21/08

(30) Priority: **18.12.90 JP 413829/90**
**11.11.91 JP 294464/91**

(43) Date of publication of application:
**24.06.92 Bulletin 92/26**

(84) Designated Contracting States:
**AT BE CH DE DK ES FR GB GR IT LI LU MC NL SE**

(71) Applicant: **TAKEDA CHEMICAL INDUSTRIES, LTD.**
**1-1, Doshomachi 4-chome**
**Chuo-ku, OSAKA(JP)**

(72) Inventor: **Iwasa, Susumu**
**21-2, Ohsumigaoka 1-chome, Tanabe-cho**
**Tsuzuku-gun, Kyoto 610-93(JP)**
Inventor: **Tada, Hiroko**
**30-4, Yuyamadai 1-chome**
**Kawanishi, Hyogo 666-01(JP)**
Inventor: **Watanabe, Takeshi**
**6-19, Kaisei 5-chome**
**Saga, Saga 840(JP)**

(74) Representative: **von Kreisler, Alek, Dipl.-Chem. et al**
**Patentanwälte Von Kreisler-Selting-Werner,**
**Deichmannhaus am Hauptbahnhof**
**W-5000 Köln 1(DE)**

## (54) Chimeric antibodies and their use.

(57) Disclosed are monoclonal chimeric antibodies containing an anti-human fibrin antibody light chain variable region and/or anti-urokinase antibody light chain variable region in combination with an anti-human antibody light chain constant region. Further disclosed are DNAs comprising a DNA coding for said antibody and eukaryotic cells allowing expression of said antibodies. Said chimeric antibodies can be used for producing a thrombolytic protein complex, which is useful as thrombolytic agent, by immunologically binding a urokinase to the urokinase recognition site of said bispecific antibodies.

EP 0 491 351 A2

## BACKGROUND OF THE INVENTION

The present invention relates to a chimeric monoclonal antibody specifically binding to human fibrin, a urokinase and both of them, a deoxyribonucleic acid coding for said antibody, a eukaryotic cell capable of producing said antibody and a thrombolytic complex which comprises said antibodies.

The method developed by Köhler and Milstein for monoclonal antibody (hereinafter also abbreviated as MoAb) production using hybridomas is advantageous in that antibodies showing single specificity can be obtained in large quantities and in a stable manner [Kohler, G. and Milstein, C.: Nature, 256, 495 (1975)]. This technique has been applied widely. Recently, in particular, said technique has contributed much not only to detection and purification of various antigens or development of diagnostic reagents but also to creation of preventive and/or therapeutic agents for various diseases.

However, the use of a MoAb, for example a mouse MoAb, which is a heterologous protein to humans, as a preventive and/or therapeutic agent may result in a reduced therapeutic effect due to the production of antibodies to the mouse MoAb in the human body or is risky in that it may cause a severe allergic response. Therefore, it is much more desirable that a human MoAb is used as a preventive and/or therapeutic agent. However, the technology for preparing human monoclonal antibodies has not advanced so much as that for producing mouse monoclonal antibodies and has so far resulted in only a few actually successful cases. Human monoclonal antibodies are produced by human-human hybridomas, mouse-human heterohybridomas or human lymphocytes transformed by Epstein-Barr virus (hereinafter also abbreviated as EBV), among others. However, the latter two are inferior in the stability of antibody producing ability and in proliferation potency and, accordingly, it is desirable that human-human hybridomas should be used for MoAb production. Generally, however, the efficiency of fusion in human-human hybridoma production is very low and thus, there is a delay in the development of human monoclonal antibodies as drugs.

Nevertheless, as mentioned above it is expected that monoclonal antibodies be applied as drugs. In particular, clinical application thereof has already made marked advances in missile therapy for cancer and antibody targeting thrombolysis therapy [R. K. Oldham et al.: Journal of Biological Response Modifiers, 2, 1 (1983); E. S. Vitteta et al.: Science, 219, 644 (1983); E. Haber et al.: Science, 243, 51 (1989); E. K. Gold et al.: Circulation, 77, 670 (1988)]. In antibody targeting thrombolysis therapy, the use of antibodies capable of carrying thrombolytic proteins, for example enzymes or precursors thereof involved in thromlysis, such as streptokinase (hereinafter also abbreviated as SK), urokinase, tissue plasminogen activator (hereinafter also abbreviated as TPA) and prourokinase, selectively to the sites of thrombosis, especially to fibrin contained in the thrombus, has been investigated [M. S. Runge et al.: Proceedings of the National Academy of Sciences of the U.S.A., 84, 7659 (1989); C. Bode et al.: Journal of Biological Chemistry, 264, 944 (1989)]. A bispecific antibody capable of binding to fibrin at a target thrombotic site while holding a thrombolytic agent has also been developed [T. Kurokawa et al.: Bio/Technology, 7, 1163 (1989); European Unexamined Patent Publication No. 363712].

In these antibody-targeting thrombolytic agents, antibodies which do not react with fibrinogen but bind specifically to fibrin are used so as to alleviate the adverse effects of said thrombolytic proteins [B. L. Pacella, Jr. et al.: Molecular Immunology, 20, 521 (1983); K. Y. Hui et al.: Science, 222, 1129 (1983)]. However, these anti-fibrin specific antibodies as well as anti-thrombolytic protein antibodies such as anti-TPA antibody and anti-UK antibody are of the mouse type and when administered to human patients, they cause immune responses in the recipients and as the administration is repeated, they sustain progressive loss of the targeting effect and tend to undergo rapid clearance from circulation.

Meanwhile, recent advances in the recombinant DNA technology have made it possible to produce chimeric antibodies in which the variable region of an antibody derived from a certain animal species is bound to the constant region of an antibody derived from another animal species [D. R. Shaw et al.: Journal of Immunology, 138, 4534 (1987); L. K. Sun et al.: Proceedings of the National Academy of Sciences of the U.S.A., 84, 214 (1987); M. S. Neuberger et al.: Nature, 314, 268 (1985); S. L. Morrison et al.: Proceedings of the National Academy of Sciences of the U.S.A., 81, 6851 (1984); and elsewhere]. As regards the bispecific antibody, too, a mouse-human chimeric antibody applicable to cancer targeting has been reported [Japanese Unexamined Patent Publication No. 2-145187].

Among the chimeric antibodies produced by using this technology, there are a number of mouse-human chimeric antibodies in which the variable region of a mouse antibody is bound to the constant region of a human antibody. Such chimeric antibodies, in which the Fc portion is predominantly responsible for their immunogenicity is of the human type, which can advantageously be administered to humans.

As mentioned above, administration of a MoAb, which is a heterologous protein to humans, for example a mouse MoAb, as a prophylactic or therapeutic agent may meet with a reduction in therapeutic effect or involve a risk of inducing a severe allergic response due to the production of an antibody to the mouse MoAb in the human body. Therefore, it is by far more desirable that a human MoAb be employed. However, the technology of producing human monoclonal antibodies is trailing far behind the technology for producing mouse monoclonal antibodies and, accordingly, the development of human monoclonal antibodies as drugs is also much behind. Thus, there is a desire in the art to produce antibodies which are clinically applicable and show low immunogenicity should desirably be utilized.

BRIEF DESCRIPTION OF THE DRAWINGS

In the drawings, the symbols used therein are as defined below:

| | |
|---|---|
| E : | EcoRI site |
| H : | HindIII site |
| B : | BamHI site |
| M : | MluI site |
| Hp : | HpaI site |
| P : | PstI site |
| hCK: | Human $\kappa$ chain C region gene exon |
| VFK: | FIB1-11 $\kappa$ chain V region gene exon |
| L : | The leader sequence gene exon of FIB1-11 $\kappa$ chain in Fig. 2., and that of NL-1 H chain in Fig. 5. |
| o : | Promotor of FIB1-11 $\kappa$ in Fig. 2 and that of NL-1 cell H chain in Fig. 5. |
| En : | Human IgG H chain enhancer |
| $hC\gamma_1$: | Human $\gamma_1$, chain constant region exon |
| VHNL-1: | NL-1 H chain V region exon |
| VFH : | FIB1-11 H chain V region cDNA |
| EV : | EcoRV site |
| Bg : | BglII site |
| Bs : | BstPI site |
| Pv : | PvuII site |
| Bc : | BclI site |
| Ps : | PstI site |
| Sp1 : | SplI site |
| Pm : | PmacI site |
| Sp : | SpeI site |
| Xh : | XhoI site |
| C : | ClaI site |
| Sac : | SacI site |
| Sca : | ScaI site |
| S : | SalI site |
| Spe : | SpeI site |
| Xb : | XbaI site |

Fig. 1 shows the nucleic acid sequence of the genomic $V_{FK}$ gene constructed in Example 1-(1) and the amino acid sequence deduced from said gene. Further show the framework region (FR), complementarity determining region (CDR) and leader sequence (Leader) as well as the estimated site of recombination between the $V_K$II gene and $J_{K2}$ gene. The transcription promoter (octamer sequence) is enclosed with a rectangle.

Fig. 2 shows the construction scheme for the mouse-human chimeric antibody light chain expression vector ($pSV_2hF_k$) constructed in Example 1-(3). The antibody gene exon portions are shown by ▭.

Fig. 3 shows the results of PAGE of the amplified cDNA fragment (lane F) obtained in Example 2-(1). Lane M shows molecular weight markers (pBR322/HinfI).

Fig. 4 shows the nucleic acid sequence of the genomic chimeric $V_{FH}$ gene constructed in Example 2-(1) and the amino acid sequence deduced therefrom. It further shows the framework region (FR), complementarity determining region (CDR) and leader sequence (Leader) as well as the site of recombination between $V_{HNL1}$ and $V_{FH}$-cDNA and the estimated sites of $V_H$III-$D_{SP2}$-$J_{H4}$ gene recombination. The PCR primer-derived sequences are underlined.

Fig. 5 shows the construction sheme for the mouse-human chimeric antibody heavy chain expression vector ($pSV_2$-hFH) constructed in Example 2-(3). Further, the antibody gene exon portions are shown by ▭. The $V_{FH}$-cDNA portion obtained by PCR is shown by VFH.

Fig. 6 shows the proliferating ability (●) and antibody producing capacity (o) of the mouse-human chimeric antibody producing cell line FIB1-HO1/X63 obtained in Example 3-(2).

Fig. 7 shows the inhibitory effect of the peptide-BSA conjugate described in Reference Example 2-(1) on the binding of the chimeric antibody FIB1-HO1/X63 (o) prepared in Example 3-(4) and of the mouse antibody FIB1-11 (●) to a fibrin monomer-sensitized microtiter plate.

Fig. 8 shows the construction scheme for the plasmid pTB1410 constructed in Example 4-(1), which contains a mouse-human chimeric anti-human fibrin $\kappa$ chain cDNA. The cDNA portion is represented by ▭.

Fig. 9 shows the nucleic acid sequence of the chimeric $\kappa$ chain cDNA (Igkv) obtained in Example 4-(1) and the amino acid sequence deduced therefrom. It further shows the leader sequence, variable region ($V_K$) and constant region ($C_K$).

Fig. 10 shows the construction scheme for the plasmid pTB1373 constructed in Example 4-(2) and containing a mouse-human chimeric anti-human fibrin heavy chain cDNA. The cDNA portion is represented by ▭.

Fig. 11 shows the nucleic acid sequence of the chimeric H chain cDNA constructed in Example 4-(2) and the amino acid sequence deduced therefrom. It also shows the leader sequence, variable region ($V_H$) and constant region (CH1, hinge, CH2, CH3).

Fig. 12 shows the construction scheme for the mouse-human chimeric anti-human fibrin $\kappa$ chain expression vector (pTB1411) and H chain expression vector (pTB1374) constructed in Example 4-(3). The SR$\alpha$ promoter (SR$\alpha$) derived from pcDL-SR$\alpha$296 is represented by ▭, the poly A additional signal region (poly A) by

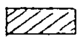 ,

the chimeric H chain cDNA (IhH-FIB) by

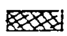 ,

and the chimeric $\kappa$ chain cDNA (Igsk-FIB) by

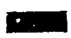 .

Fig. 13 shows the construction scheme for the mouse-human chimeric anti-human fibrin antibody cDNA expression vector (pTB1387) constructed in Example 4-(3). The SR$\alpha$ promoter (SR$\alpha$) is represented by

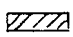 ,

the poly A additional signal region (poly A) by

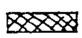 ,

the IgH-FIB cDNA by ▭, the Igk-FIB cDNA by

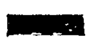

and the gpt gene by

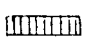 .

Fig. 14 shows the construction scheme for the antibody $V_H$ cDNA cloning vector pTB1420 and antibody $V_K$ cDNA cloning vector pTB1421 constructed in Example 5-(1). The promoter region is represented by

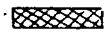 ,

the poly A additional signal region by

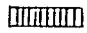 ,

the V region cDNA by

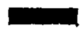 ,

and the regioning antibody cDNA portion by ▭.

Fig. 15 shows the nucleic acid sequence of the anti-UK antibody $V_K$ cDNA obtained in Example 5-(2) and the amino acid sequence deduced therefrom. It also shows the estimated location of the complementarity determining region (CDR), the PCR primer-derived sequence being underlined.

Fig. 16 shows the nucleic acid sequence of the anti-UK antibody $V_H$ cDNA obtained in Example 5-(3) and the amino acid sequence deduced therefrom. It also shows the estimated location of the complementarity determining region (CDR), the PCR primer-derived sequence being underlined.

Fig. 17 shows the construction scheme for the mouse-human chimeric anti-UK antibody expression vector (pTB1458) constructed in Example 5-(4). The SR$\alpha$' promoter and poly A signal region (poly A) is represented by

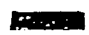 ,

the chimeric antibody $\kappa$ chain cDNA portion by

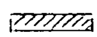 ,

the chimeric antidoby H chain cDNA portion by ▭, and the neomycin resistance gene by

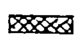 .

Fig. 18 shows the antibody-producing ability of the mouse-human chimeric anti-UK antibody-producing SU/S-9.21 cells obtained in Example 5-(6).

Fig. 19 shows the bispecific antibody-producing ability of the bispecific chimeric antibody-producing SUSF/S-8.4 cells obtained in Example 6-(1).

Fig. 20 shows the results of an in vitro plasma clot lysis test performed with the bispecific chimeric antibody SUSF/S-8.4 obtained in Example 6-(1). The lytic potency of the prourokinase/antibody conjugate is found on the ordinate, with the lytic potency of prourokinase alone (in the absence of the antibody) being taken as 1.

Fig. 21 shows the results of subjecting a conjugate between the bispecific chimeric antibody SUSF/S-8.4 described in Example 6-(1) and prourokinase to an in vivo experiment with a hamster pulmonary embolism model. The result is compared with the results obtained by single administration of prourokinase.

## DISCLOSURE OF THE INVENTION

In this specification, amino acids and peptides are represented by the respective abbreviations adopted by the IUPAC-IUB Commission on Biochemical Nomenclature (CBN). Thus, for instance, the abbreviations given below are used. Where optical isomerism is involved, for example in the case of an amino acid, the L form is referred to unless otherwise specified.

Gln: Glutamine residue
Asp: Aspartic acid residue
Pro: Proline residue

Tyr: Tyrosine residue
Val: Valine residue
Lys: Lysine residue
Glu: Glutamic acid residue
Ala: Alanine residue
Asn: Asparagine residue
Leu: Leucine residue
Phe: Phenylalanine residue
Gly: Glycine residue
His: Histidine residue
Ser: Serine residue
Thr: Threonine residue
Ile: Isoleucine residue
Trp: Tryptophan residue
Arg: Arginine residue
Met: Methionine residue

Unless otherwise specified, the direction of from left to right in a sequence is identical with the direction of from amino terminus to carboxy terminus, and the symbol "-" at either end means a bonding valence.

In this specification, each deoxyribonucleic acid (hereinafter sometimes referred to as DNA) occurring as a polymer or oligomer is represented by a sequence of the following abbreviations:

A: 2'-Deoxyadenylic acid residue
C: 2'-Deoxycytidylic acid residue
G: 2'-Deoxyguanylic acid residue
T: Thymidylic acid residue

Unless otherwise specified, the direction of from left to right in the sequence is the direction of from 5' to 3'.

Under the technological circumstances mentioned above, the present inventors made intensive investigations in an attempt to derive chimeric antibodies from anti-human fibrin specific antibodies, anti-UK (urokinase) antibodies and anti-fibrin anti-UK bispecific antibodies, which are useful in the targeting of thrombolytic proteins. As a result, DNA or RNA was extracted from an anti-human fibrin specific antibody producing hybridoma and an anti-UK antibody producing hybridoma, respectively, and the nucleic acid sequence coding for the variable region of the antibody involved in binding to human fibrin or UK was determined. And then, said sequence was combined with the nucleic acid sequence coding for the constant region of a human antibody to give chimeric antibodies capable of specifically binding to human fibrin and UK, respectively, as well as cells producing the same and DNAs coding for such chimeric antibodies. Furthermore, bispecific chimeric antibody-producing cells were produced by introducing genes coding for these two chimeric antibodies into animal cells.

The present invention further provides:

(1) a chimeric monoclonal antibody which contains an anti-human fibrin antibody light chain variable region containing at least one of the polypeptide chains A, B and C represented by the formulas

A: -Thr Ser Ser Gln Ser Leu Leu Asp Ser Asp Gly Lys Thr Tyr Leu Asn-,[Seq. ID No. 1]

B: -Leu Val Ser Lys Leu Tyr Ser- [Seq. ID No. 2] and

C: -Trp Gln Gly Ile His Phe Pro Tyr-, [Seq. ID No. 3]

respectively, and a human antibody light chain constant region;

(2) a DNA which contains a DNA coding for the above anti-human fibrin antibody light chain variable region; and

(3) a DNA which contains a DNA coding for the above anti-human fibrin antibody light chain variable region and a DNA coding for a human antibody light chain constant region.

The invention further provides:

(4) a chimeric monoclonal antibody which contains an anti-human fibrin antibody heavy chain variable region containing at least one of the polypeptide chains D, E and F represented by the formulas

D: -Asn Tyr Asp Met Ser- [Seq. ID No. 4],
E: -Ser Ile Ser Val Gly Gly Thr Thr Tyr Tyr Pro Asp Ser Met Lys Gly [Seq. ID No. 5], and
F: -Gly Asn Phe Ala Asp Ala Met Asp Tyr- [Seq. ID No. 6],

respectively, and a human antibody heavy chain constant region;
(5) a DNA which contains a DNA coding for the above anti-human fibrin antibody heavy chain variable region; and
(6) a DNA which contains a DNA coding for the above anti-human fibrin antibody heavy chain variable region and a DNA coding for a human antibody heavy chain constant region.
(7) a chimeric monoclonal antibody which contains a UK-recognizing antibody light chain variable region containing at least one of the polypeptide chains G, H and I represented by the formulas

G: -Ser Ala Ser Ser Ser Val Gly Tyr Met Tyr- [Seq. ID No. 13],
H: -Leu Thr Ser Asn Leu Ala Ser- [Seq. ID No. 14] and
I: -Gln Gln Trp Ser Ser Asp Pro Pro Thr-, [Seq. ID No. 15]

respectively, and a human antibody light chain constant region;
(8) a DNA which contains a DNA coding for the above UK-recognizing antibody light chain variable region; and
(9) a DNA which contains a DNA coding for the above UK-recognizing antibody light chain variable region and, further, a DNA coding for a human antibody light chain constant region.
The present invention still further provides:
(10) a chimeric monoclonal antibody which contains a UK-recognizing antibody heavy chain variable region conaining at least one of the polypeptide chains J, K and L represented by the formulas

J: -Ser Asp Tyr Ala Trp Asn- [Seq. ID No. 16],
K: -Tyr Ile Asn Tyr Ser Gly Thr Thr Ser Tyr Asn Pro Ser Leu Lys Ser- [Seq. ID No. 17] and
L: -Leu Gly Asp Phe Asp Ala Gly Asp Tyr Phe Asp Tyr- [Seq. ID No. 18],

respectively, and a human antibody heavy chain constant region;
(11) a DNA which contains a DNA coding for the above UK-recognizing antibody heavy chain variable region; and
(12) a DNA which contains a DNA coding for the above UK-recognizing antibody heavy chain variable region and, a human antibody heavy chain constant region.
The invention further provides:
(13) a bispecific chimeric monoclonal antibody which contains two kinds of light chain and heavy chain variable regions, which specifically recognize human fibrin and a UK, respectively, and further contains the light and heavy chain constant each of which has a human antibody constant.
The invention also provides:
(14) a thrombolytic protein immunocomplex which contains an anti-human fibrin chimeric antibody fragment (1) and an anti-urokinase chimeric antibody fragment (2), wherein a urokinase being immunologically coupled to the chimeric antibody fragment (2).
The invention is further concerned with eukaryotic cells allowing expression of the chimeric monoclonal antibody mentioned above under (1), (4), (7), (10) or (13). Still further, the invention is concerned with a mouse-human chimeric anti-human fibrin antibody-producing mouse myeloma cell transformant designated FIB1-HO1/X63, a mouse-human chimeric anti-human fibrin antibody-producing mouse hybridoma cell transformant designated ss/s-3, a mouse-human chimeric anti-human UK antibody-producing mouse hybridoma cell transformant designated SU/S-9.21 and a mouse-human chimeric anti-human fibrin anti-UK bispecific antibody-producing mouse hybridoma cell transformant designated SUSF/S-8.4.

The anti-human fibrin antibody light chain variable region in the chimeric monoclonal antibody of the present invention may be any one if it contains at least one of the polypeptide chains A, B and C represented by the above formulas A, B and C, respectively. A preferred one is an anti-human fibrin antibody light chain variable region showing high affinity and specificity for human fibrin, in particular an anti-human fibrin antibody light chain variable region containing at least two, in particular all three, of the polypeptide chains A, B and C as complementarity determining regions (hereinafter briefly referred to as CDRs). A more preferred one contains an amino acid sequence represented by the following formula (I):

```
Asp Val Val Met Ala Gln Thr Pro Leu Thr Leu Ser Val Thr
Ile Gly Gln Pro Ala Phe Ile Ser Cys Thr Ser Ser Gln Ser
Leu Leu Asp Ser Asp Gly Lys Thr Tyr Leu Asn Trp Leu Leu
Gln Arg Pro Gly Gln Ser Pro Lys Arg Leu Ile Tyr Leu Val
Ser Lys Leu Tyr Ser Gly Val Pro Asp Arg Phe Thr Gly Ser
Gly Ser Gly Thr Ala Phe Thr Leu Lys Ile Asn Arg Val Glu
Ala Glu Asp Leu Gly Val Tyr Tyr Cys Trp Gln Gly Ile His
Phe Pro Tyr Thr Phe Gly Gly Gly Thr Lys Leu Glu Ile Lys
(I)                                        [Seq. ID No.7]
```

The anti-human fibrin antibody heavy chain variable region in the chimeric monoclonal antibody of the present invention may be any one if it contains at least one of the polypeptide chains D, E and F represented by the above formulas D, E and F, respectively. A preferred one is an anti-human fibrin antibody heavy chain variable region showing high affinity and specificity for human fibrin, in particular an anti-human fibrin antibody heavy chain variable region containing at least two, in particular all three, of the polypeptide chains D, E and F as CDRs. A more preferred one contains an amino acid sequence represented by the following formula (II):

```
Asp Val Gln Leu Trp Glu Ser Gly Gly Gly Leu Val Lys Pro
Gly Gly Ser Leu Lys Leu Ser Cys Ala Ala Ser Gly Phe Thr
Phe Ser Asn Tyr Asp Met Ser Trp Val Arg Gln Thr Pro Glu
Arg Arg Leu Glu Trp Val Ala Ser Ile Ser Val Gly Gly Thr
Thr Tyr Tyr Pro Asp Ser Met Lys Gly Arg Phe Thr Ile Ser
Arg Asp Asn Ala Arg Asn Ile Leu Tyr Leu Gln Leu Ser Ser
Leu Arg Ser Glu Asp Thr Ala Met Tyr Tyr Cys Gly Asn Phe
Ala Asp Ala Met Asp Tyr Trp Gly Gln Gly Thr Leu Val Thr
Val Ser Ser (II)                           [Seq. ID No.8]
```

As the chimeric monoclonal antibody of the present invention, there may be mentioned chimeric monoclonal antibodies containing at least one of the above-mentioned anti-human fibrin antibody heavy chain and light chain variable regions. Among them, those chimeric monoclonal antibodies which contain the above-mentioned anti-human fibrin antibody light chain variable region are preferred. Those chimeric monoclonal antibodies which further contain the above-mentioned anti-human fibrin antibody heavy chain variable region are also preferred. The light chain and heavy chain constant regions in the chimeric monoclonal antibodies of the present invention may be any human antibody light chain and heavy chain constant regions, respectively. Thus, for instance, those human antibody light chain and heavy chain constant regions the amino acid sequences of which are respectively known may be used. By combining these with the above-mentioned anti-human fibrin antibody light chain and heavy chain variable regions, the light and heavy chains of the monoclonal antibodies of the present invention can be obtained. The chimeric monoclonal antibody of the present invention includes chimeric monoclonal antibodies which contain at least one of the thus-obtained light and heavy chains and show affinity and specificity for human fibrin. Among them, those which contain the above-mentioned light and heavy chains are preferred, however.

The term "a urokinase" briefly referred to as "UK" as used herein in the designation "UK-recognizing chimeric antibody" is a general term for single-chain or two-chain urokinase, low molecular urokinase, prourokinase and the like and also includes urokinase precursors and derivatives showing thrombolytic activity and capable of being recognized by antibodies.

The anti-UK recognizing antibody light chain variable region in the chimeric monoclonal antibody of the present invention may be any one if it contains at least one of the polypeptide chains G, H and I represented by the above formulas G, H and I, respectively. A preferred one is an anti-UK recognizing antibody light chain variable region showing high affinity and specificity for a UK, in particular a UK-recognizing antibody light chain variable region containing at least two, in particular all three, of the polypeptide chains G, H and I as CDRs. A more preferred one contains an amino acid sequence represented by the following formula (III):

Asp Ile Gln Leu Thr Gln Ser Pro Ala Leu Met Ser Ala Val
Pro Gly Glu Lys Val Thr Met Thr Cys Ser Ala Ser Ser Ser
Val Gly Tyr Met Tyr Trp Tyr Gln Gln Lys Pro Arg Ser Ser

Pro Lys Pro Trp Ile Ser Leu Thr Ser Asn Leu Ala Ser Gly
Val Pro Ala Arg Phe Ser Gly Ser Gly Ser Gly Thr Ser Tyr
Ser Leu Thr Ile Ser Ser Met Glu Ala Glu Asp Ala Ala Thr
Tyr Tyr Cys Gln Gln Trp Ser Ser Asp Pro Pro Thr Phe Gly
Gly Gly Thr Lys Leu Glu Ile Lys Arg (III)

[Seq. ID No. 19]

The UK-recognizing antibody heavy chain variable region in the chimeric monoclonal antibody of the present invention may be any one if it contains at least one of the polypeptide chains J, K and L represented by the above formulas J, K and L, respectively. A preferred one is a UK-recognizing antibody heavy chain variable region showing high affinity and specificity for a UK, in particular a UK-recognizing antibody heavy chain variable region containing at least two, in particular all three, of the polypeptide chains J, K and L as CDRs. A more preferred one contains an amino acid sequence represented by the following formula (IV):

Glu Val Gln Leu Val Glu Ser Gly Pro Gly Leu Val Lys Pro
Ser Gln Ser Leu Ser Leu Thr Cys Thr Val Thr Gly Tyr Ser
Ile Thr Ser Asp Tyr Ala Trp Asn Trp Ile Arg Gln Phe Pro
Gly Asn Lys Leu Glu Trp Met Gly Tyr Ile Asn Tyr Ser Gly
Thr Thr Ser Tyr Asn Pro Ser Leu Lys Ser Arg Ile Ser Ile
Thr Arg Asp Thr Ser Asn Asn Gln Phe Phe Leu Gln Leu Asn
Ser Val Thr Ser Glu Asp Thr Ala Thr Tyr Tyr Cys Ala Arg
Leu Gly Asp Phe Asp Ala Gly Asp Tyr Phe Asp Tyr Trp Gly
Gln Gly Thr Thr Val Thr Val Ser Ser Ala Ser (IV)
[Seq. ID No. 20]

The DNAs of the present invention which respectively code for the light and heavy chain variable regions should preferably contain also a DNA coding for a leader peptide necessary for the expression of these polypeptides in eukaryotic cells. The DNA sequence coding for the leader polypeptide for the light chain variable region polypeptide should preferably be substantially identical with a DNA sequence of the formula:

Met Met Ser Pro Ala Gln Phe Leu Phe Leu Leu Val Leu Trp
ATG ATG AGT CCT GCC CAG TTC CTG TTT CTG TTA GTG CTC TGG
Ile Arg Glu Thr Asn Gly
ATT CGG GAA ACC AAC GGT

(the 153rd to 201st and 603rd to 613th of nucleic acid sequence of Seq. ID No. 11)

The DNA sequence coding for the leader polypeptide for the heavy chain variable region polypeptide should preferably be substantially identical with a DNA sequence of the formula:

Met Asp Ser Arg Leu Asn Leu Val Phe Leu Val Leu Ile Leu
ATG GAC TCC AGG CTC AAT TTA GTT TTC CTT GTC CTT ATT TTA
Lys Gly Val Gln Cys
AAA GGT GTC CAG TGT

(the 151st to 195th and 314th to 624th of nucleic acid sequence of Seq. ID No. 12)

The DNAs for the leader peptides as disclosed herein contain a translation initiation signal, namely a DNA at which functional polypeptide translation starts, as well.

However, since leader peptides are not involved in binding affinity of fibrin or UK, the use of particular eukaryotic leader peptides is not necessarily required but DNA sequences coding for other eukaryotic leader peptides may also be used for the purpose of the present invention.

If a DNA of the present invention which codes for the light chain or heavy chain variable region, even after modification thereof by means of site-specific mutagenesis, for example, still can be translated into a chimeric antibody substantially equivalent to the antibody of the present invention, the modification of said DNA also falls under the scope of the present invention. Such modification procedure sometimes yield a chimeric antibody with favorably modified affinity and specificity for human fibrin and/or UK.

The DNAs of the present invention which code for the light chain and heavy chain variable regions, respectively, should preferably be taken from genomic DNA derived from a human fibrin-specific MoAb or UK-specific MoAb producing mouse hybridoma. Particularly preferred mouse hybridomas are the mouse hybridoma FIB1-11 (IFO No. 50174, FERM No. BP-2081) and UK 1-3 (IFO No. 50176, FERM BP-2083) which have high specificity and affinity for human fibrin [T. Kurokawa et al.: Bio/Technology, 7, 1163 (1989)] or UK, respectively.

It is also possible, however, to use DNAs obtained from other mammals (e.g. rabbit, goat, horse, ox or cow) if they are substantially equivalent in DNA sequence or in the amino acid sequences of the light chain and heavy chain variable regions after translation to those disclosed herein.

The genomic DNA to be used in the practice of the invention can be prepared for cloning by any of the conventional methods or various other methods known to those skilled in the art [L. G. Davis et al. (ed.): Basic Methods in Molecular Biology, published by Elsevier, New York, 1986; and J. Feder et al.: American Journal of Human Genetics, 37, 635 (1985)].

The mRNA to be used in the practice of the present invention can also be prepared by any of the known methods in conventional use and can be used as a material for cDNA preparation and cloning [J. Sambrook et al. (ed.): Molecular Cloning, A Laboratory Manual, published by Cold Spring Harbor Laboratory Press, New York, 1989].

For example, it can be obtained from a genomic library by methods known to those skilled in the art. Thus, the genomic DNA of the hybridoma cells is first prepared by a standard procedure. For example, the cells are treated with proteinase K in the presence of sodium dodecyl sulfate (SDS) and then extracted with phenol. Further treatment with DNase-free RNase A and the subsequent phenol extraction give the genomic DNA [Masami Muramatsu (ed.): Laboratory Manual of Genetic Engineering, Maruzen, Tokyo, page 59 (1988)]. The genomic DNA containing the desired variable region gene is then isolated by a standard procedure. For example by fragmenting the genomic DNA into restriction fragments using a restriction endonuclease, cloning the resulting fragments in an appropriate recombinant DNA cloning vector, and screening for the DNA sequence disclosed herein using a radiolabeled or enzyme-labeled probe.

The DNA obtained from genomic DNA generally contains introns which do not code for any polypeptide, so that the DNA is modified, for example, by the known method of deletion or substitution [W. Kramer et al.: Nucleic Acids Research, 12, 9441 (1984); and T. A. Kunkel: Proceedings of the National Academy of Sciences of the U.S.A., 82, 488 (1985)].

The DNAs coding for the light chain and heavy chain variable region polypeptides of the chimeric antibody of the present invention can also be obtained from a cDNA library [H. Okayama et al.: Molecular and Cellular Biology, 2, 161 (1982)].

For example, the mRNA is prepared from the hybridoma cells by a standard method, for example by homogenizing the cells in a solution containing guanidine thiocyanate and subjecting the homogenate to ultracentrifugation on a cesium trifluoroacetate-EDTA density gradient to give an RNA pellet, and further subjecting to an oligo-dT column to give a poly(A)$^+$-RNA. Using this poly(A)$^+$-RNA as a template, a first strand cDNA is synthesized by a method known to the skilled artisan using an oligo-dT primer, a random primer or a primer specific to the antibody gene DNA sequence. And, further, this first strand cDNA is used as a template for the synthesis of a second strand cDNA [Masami Muramatsu (ed.): Laboratory Manual of Genetic Engineering, Maruzen, Tokyo, page 70 and page 77 (1988)].

The cDNA obtained in such a method is cloned in an appropriate cloning vector and the clone obtained is screened for a cDNA coding for the variable region disclosed herein using an appropriate probe. After isolation of the desired clone, the cDNA can be treated essentially in the same manner as the genomic DNA.

It is also possible to obtain the DNAs coding for the light chain and heavy chain variable regions of the chimeric antibody of the present invention by specifically amplifying the same using the polymerase chain reaction [R. Orlandi et al.: Proc. Natl. Acad. Sci. U.S.A., 86, 3833 (1989)].

DNAs coding for the light chain and heavy chain variable regions specific to human fibrin or UK can also be synthesized chemically in the conventional manner [N. D. Shina et al.: Nucleic Acids Research, 12, 4359 (1984)]. These synthetic DNAs need not be identical with the DNAs obtained by cloning even if one or more original codons have been replaced by a corresponding degenerate codon or codons provided that when the codon or codons are translated, the same amino acid or acids are given.

The DNAs coding for the human antibody light chain and heavy chain constant regions of the chimeric antibody of the present invention can be cloned from the genomic DNA and cDNAs. They can be chemically synthesized as well.

Chimeric light chain and heavy chain polypeptides with very low antigenicity can be obtained by using the DNAs coding for human antibody constant regions as obtained in the above manner. Such DNAs coding for human antibody constant region polypeptides are preferably obtained from human lymphocytes, for example peripheral blood lymphocytes. Furthermore, as the light chain constant region-encoding DNA, a DNA obtained from the human antibody $\kappa$ chain (kappa chain; hereinafter also abbreviated as $C\kappa$) DNA can be used with particular preference and, as the heavy chain constant region-encoding DNA, a DNA obtained from a human antibody $\gamma$ chain DNA, in particular $\gamma 1$ chain (hereinafter also abbreviated as $C\gamma 1$) DNA [P. A. Heiter et al.: Cell, 22, 197 (1980)].

The thus-obtained DNA constructs can be introduce into an appropriate recombinant DNA cloning vector and a recombinant DNA expression vector by methods know to those skilled in the art [Y. Gluzman (ed.)" Eukaryotic Viral Vectors, published by Cold Spring Harbor Laboratory, New York, 1982; Y. Gluzman (ed.): Eukaryotic Transcription, published by Cold Spring Harbor Laboratory, New York, 1985; European Unexamined Patent Publication No. 380068].

In the practice of the invention, the DNA constructs coding for the light chain and heavy chain polypeptides are introduced into an appropriate eukaryotic host cell as part of an expression vector. These two constructs may be contained in one single expression vector for eukaryotic cells or may be maintained individually in separate expression vectors. It is necessary, however, that, for chimeric polypeptide expression, the vector or vectors should contain sequences for transcription and translation regulation which are functional in the eukaryotic host cell selected. Therefore, chimeric genes each constructed as a large DNA fragment which contains 5' and 3' nontranslational regions and introns as well as all homologous sequences functional in eukaryotic host cells, such as a promoter, enhancer, transcription terminator and polyadenylation site (hereinafter also abbreviated as poly-A site) are preferably used. It is also possible to recombinationally bind the DNA construct coding for the chimeric light chain or heavy chain polypeptide to various heterologous regulatory regions such as well-known SV40 or herpes virus TK sequences containing a viral promoter, enhancer, transcription terminator and poly-A site. The chimeric gene constructs may be bound to synthetic regulatory elements if such elements are functional in eukaryotic host cells and can be fused with said chimeric genes in an appropriate manner. The cDNA clones or synthetic genes, too, can be bound to homologous or heterologous regulatory sequences for their expression as polypeptides.

A number of recombinant expression vectors are known and they can be employed in the practice of the invention. Among them, however, vectors of the pSV2 type are used with particular preference. Said pSV2 type vectors contain a segment constituting the eukaryotic transcription unit of the SV40 genome and can be used for the transformation of mammalian and other eukaryotic host cells by inserting them into the chromosomal DNA of said cells. Various plasmid pSV2 type vectors in which the transcription of an insert gene is ordered by the SV40 promoter, for example the plasmids pSV2-gpt, pSV2-neo, pSV2-dhfr and pSV2-$\beta$-globin, have already been constructed [cf. "Eukaryotic Viral Vectors"]. Expression vectors maintained extra-chromosomally, such as bovine papilloma virus-based expression vectors and Epstein-Barr virus (EBV) expression vectors, can also be used ["Eukaryotic Viral Vectors"; D. Kioosis: EMBO Journal, 6, 355 (1987)].

Promoter-leader peptide sequences are present upstream from the 5' terminus of the respective variable regions and these sequences can be used not only in the expression of homologous immunoglobulin genes but also in the expression of heterologous immunoglobulin genes. For example, the mouse myeloma cell line X63.Ag8.653 derived by transfection of the NL1 cell line heavy promoter-leader sequence with the plasmid pSV2-hFH containing a mouse hybridoma FIB1-11-derived heavy chain variable region gene and a human antibody heavy chain constant region gene shows a high expression level.

Almost all genomic immunoglobulin genes contain an enhancer sequence. Mouse or human immunoglobulin genes enhancers can achieve a high level of expression when an immunoglobulin promoter sequence coexists. These enhancer sequences can be shifted to various sites on the expression vector while maintaining the high expression level.

As preferred examples of the eukaryotic host cell to be used as the host cell for the expression of the chimeric monoclonal antibodies of the present invention, there may be mentioned hybridoma, myeloma, plasmacytoma and lymphoma cells, among others. However, other mammalian eukaryotic host cells can also be used if the eukaryotic host cells can recognize the transcriptional and translational DNA sequences for chimeric gene expression, process the leader peptides and cleave the leader sequences for chimeric protein secretion and if posttranslational chimeric protein modification (e.g. glycosylation) is possible.

The host cells of the present invention can be transformed by methods known to those skilled in the art for transfection. In particular, the electroporation method, protoplast fusion method and calcium phosphate precipitation method, among other, are preferred [F. Toneguzzo: Molecular and Cellular Biology, 6, 703 (1986); and V. Oi et al.: Proceedings of the National Academy of Sciences of the U.S.A., 80, 825 (1983)].

For the expression of the chimeric antibodies of the present invention, the method comprising transfecting host cells successively with two recombinant vectors coding for the immunoglobulin light chain and heavy chain, respectively, is used, for instance. Thus, for example, host cells are first transfected with a DNA construct coding for the chimeric light chain of the present invention and transformant host cells expressing the chimeric light chain polypeptide are then selected by enzyme immunoassay (hereinafter also abbreviated as EIA). Further, the chimeric light chain-expressing host cells thus selected are transfected with a DNA construct coding for a heavy chain, preferably a DNA construct coding for the chimeric heavy chain of the present invention, and host cells expressing the chimeric antibody are selected. Antibodies containing the chimeric heavy chain of the present invention can be obtained in the same manner. It is also possible to introduce a chimeric light chain expression vector and a chimeric heavy chain expression vector simultaneously into host cells. Furthermore, in an alternative method, both DNA constructs coding for the chimeric light chain and heavy chain, respectively, may be inserted into one single expression vector to be used for transfection of host cells to thereby achieve chimeric antibody expression by one transfection procedure. In either case, after transfection, host cells capable of producing the desired chimeric antibody can be identified by an appropriate methods, known to those skilled in the art for example by EIA for detecting specific antibodies to human fibrin or a UK, and grown and maintained.

For the expression of the bispecific chimeric antibodies of the present invention, the method, for example, comprising transfecting host cells successively using a recombinant vector coding for the immunoglobulin light chain and heavy chain of one specific antibody and a recombinant expression vector coding for the immunoglobulin light chain and heavy chain of the other specific antibody is used. Thus, for instance, transfection is performed using a DNA construct coding for the light chain and heavy chain of one specific antibody, and chimeric antibody-expressing transformant host cells are selected using EIA. The chimeric antibody-expressing cells selected are further transfected with a DNA construct coding for the light chain and heavy chain of the other specific antibody and cells expressing the desired bispecific antibody are selected, whereby the bispecific antibody can be obtained.

It is also possible to introduce an expression vector coding for the immunoglobulin light chain and heavy chain of one specific antibody and an expression vector coding for the immunoglobulin light chain and heavy chain of the other specific antibody simultaneously into host cells. Furthermore, according to an alternative method, the bispecific chimeric antibody can be expressed by preparing transformant cells expressing one specific antibody and transformant cells expressing the other specific antibody separately and subjecting these two kinds of transformant cells to cell fusion. In any case, host cells producing the desired bispecific chimeric antibody can be identified for multiplication and maintenance by using known, for example EIA for detecting a bispecific antibody capable of recognizing human fibrin and a UK simultaneously.

The above-mentioned chimeric antibody-producing cells of the present invention can be grown generally in a liquid medium or in the peritoneal cavity of an animal (e.g. in the peritoneal cavity of a mammal such as a mouse) in the conventional manner. The antibody in the culture fluid or ascitic fluid can be purified by a combination of known biochemical techniques. For example, the culture or ascitic fluid is centrifuged, and the supernatant is taken out and subjected to salting out (generally using ammonium sulfate or sodium sulfate). The proteinic precipitate obtained is dissolved in an appropriate solution and, after dialysis, subjected to column chromatography (using e.g. an ion exchange column, gel filtration column, protein A column, hydroxyapatite column or antigen-bound column) for separation and purification of the desired antibody. By such separation and purification procedure as mentioned above, about 1 to 5 mg of a chimeric antibody with a purity of 90% or higher as determined on the protein weight basis can be obtained from one liter of culture supernatant, for instance, while 3 to 10 mg of an antibody with the same purity can be obtained from 20 ml of ascitic fluid.

The chimeric antibody obtained in the above manner is homogeneous as a protein and, upon treatment with a proteinase (e.g. pepsin, papain, bromelain, ficin), can give $F(ab')_2$, Fab and other fragments which retain the ability to bind to human fibrin and which can be used for the same purposes as the chimeric antibodies of the present invention.

The chimeric antibodies of the present invention can be used both in vivo and in vitro. In particular, they can present their characteristic features more markedly when used in vivo. Thus, since they have very low immunogenicity as compared with mouse antibodies, they can be administered to humans for diagnostic and therapeutic purposes. Furthermore, they are more stable and show a longer half-life in the blood as compared with the original mouse antibodies, so that they can advantageously be used for the therapeutic purpose of the present invention. In thrombolytic therapy, in particular, where the continuous intravenous administration technique has been employed for TPA or UK, which has a short half-life in the blood, the combination with such chimeric antibodies is expected to prolong the half-life in blood markedly and enable bolus administration. In the case of an anti-human fibrin antibody, for instance, the thrombus formed in the blood vessel can be imaged by administering it in a form bound to an appropriate radioactive species (e.g. $^{111}In$, $^{123}I$, $^{99m}Tc$) to the living body. Furthermore, the anti-human fibrin chimeric antibodies of the present invention can be bound to various thrombolytic substances (e.g. SK, urokinase, prourokinase, TPA, trypsin, plasmin, protein C, protein S), preferably UKs, for use in the prevention and treatment of thrombotic diseases such as myocardial infarction, peripheral arteriovenous obstruction and cerebral infarction.

They can also be chemically bound to the UK-recognizing chimeric antibodies of the present invention to give anti-human fibrin, anti-human UK bispecific chimeric antibodies, which can be used for the treatment of the above-mentioned thrombotic diseases such as myocardial infarction and cerebral infarction by administering them in the form of immunocomplexes with a UK, such as urokinase or prourokinase. For this purpose, the anti-human fibrin, anti-UK bispecific chimeric monoclonal antibodies of the present invention are suited as well; they can be used in the treatment of thromotic diseases in the form of immunocomplexes with a UK, such as urokinase or prourokinase, in the same manner as the chemically synthesized bispecific antibodies mentioned above.

In thrombolytic therapy using selective thrombolytic protein immunocomplexes prepared from these bispecific chimeric antibodies and a UK, several methods are used. For instance, (1) a bispecific chimeric antibody of the present invention is preliminarily administered to a patient with a thrombotic disease and, after the lapse of a sufficient time, a UK is administered for binding to the thrombus formed in the patient's body; (2) said bispecific chimeric antibody and a UK are simultaneously administered to a patient with a thrombotic disease; or (3) said bispecific chimeric antibody is reacted in advance with a thrombolytic agent and, after separation of unreacted UK, the resulting selective thrombolytic protein conjugate is administered to a patient with a thrombotic disease.

The thrombolytic agent or thrombolytic protein conjugate of the present invention, if necessary after bacterial filtration using a membrane filter, for instance, can be used in the treatment of patients with a thrombotic or obstructive disease, such as myocardial infarction, peripheral arteriovenous obstruction, retinal arteriovenous obstruction, cerebral infarction or pulmonary embolism, by administering the same either as such or in the form of a pharmaceutical preparation, for example an injection, as prepared by admixing with an appropriate, pharmacologically acceptable carrier, excipient or diluent.

The dose of the thrombolytic agent of the present invention may vary depending on the disease to be treated, symptom, route of administration and other factors. In the case of intravenous administration to adult patients with myocardial infarction, the general daily dose is 0.03 to 1.5 mg/kg, preferably about 0.06 to 0.6 mg/kg, as the bispecific chimeric antibody, or about 0.01 to 0.5 mg/kg, preferably about 0.02 to 0.2 mg/kg, as the thrombolytic agent urokinase, or about 0.01 to 1 mg/kg, preferably about 0.02 to 0.5 mg/kg, as the thrombolytic agent prourokinase.

By using the bispecific chimeric antibody of the present invention which can specifically bind to target sites of thrombosis and is substantially unreactive with fibrinogen in the blood in combination with a thrombolytic agent, the thrombin can be dissolved or removed selectively and efficiently while markedly reducing the immune response in patients administered with the same and alleviating side effects.

EXAMPLES

The following Reference Examples and Examples are further illustrative of the present invention but are of course by no means limitative of the scope of the invention.

In practicing the invention, recombinant DNA construction and recombinant introduction into animal cells or microorganisms were performed in accordance with the following monographs unless otherwise specified:

(1) T. Maniatis, E. F. Fritsch and J. Sambrook (authors): Molecular Cloning, published by Cold Spring Harbor Laboratory (U.S.A.).

(2) Yasutaka Takagi (ed.): Idenshi Sosa Jikken Ho (Gene Manipulation Experiments), published by Kodansha, Tokyo.

The animal cells used in the examples have been deposited as shown below.

| Animal cells | (IFO) IFO No. | (FRI) FERM No. |
|---|---|---|
| Mouse hybridoma FIB1-11 | 50174 (Sept. 21, 1988) | BP-2081 (Oct. 4, 1988) |

| | | |
|---|---|---|
| Mouse hybridoma UK1-3 | 50176 (Sept. 21, 1988) | BP-2083 (Oct. 4, 1988) |
| Mouse myeloma FIB1-HO1/X63 | 50257 (Oct. 17, 1990) | BP-3141 (Oct. 25, 1990) |
| Mouse hybrid hybridoma FU1-74 | 50185 (Mar. 13, 1989) | BP-2334 (Mar. 14, 1989) |
| Mouse hybridoma SS/S-3 | 50351 (Oct. 23, 1991) | BP-3635 (Nov. 29, 1991) |
| Mouse hybridoma SU/S-9.21 | 50352 (Oct. 23, 1991) | BP-3636 (Nov. 29, 1991) |
| Mouse hybridoma SUSF/S-8.4 | 50353 (Oct. 23, 1991) | BP-3637 (Nov. 29, 1991) |

IFO: Institute for Fermentation Osaka
FRI: Fermentation Research Institute, Ministry of International Trade and Industry

Reference Example 1

EIA for anti-fibrin antibody assay

A human fibrin monomer solution (1 mg/ml) in phosphate-buffered saline (pH 7.3; hereinafter also abbreviated as PBS) containing 3.3 M urea and 0.01% ethylenediaminetetraacetate (hereinafter also abbreviated as EDTA) was distributed in 50 µl portions into the wells of a 96-well microplate and allowed to stand overnight at 4°C. Then, 150 µl of PBS containing 2% casein and 0.01% thimerosal was added to each well to prepare a sensitized plate.

Then, 50 µl of an antibody-producing cell culture supernatant was added to each well of the above fibrin-sensitized plate, followed by 2 hours of incubation at room temperature. The plate was washed well with PBS containing 0.05% Tween 20 (hereinafter also abbreviated as PBS-TW), a horseradish peroxidase (hereinafter also abbreviated as HRP)-labeled rabbit anti-mouse IgG antibody or goat anti-human IgG antibody (each obtained from Funakoshi) was added, and further incubation was carried out at room temperature for 2 hours.

After washing, 0.1 M citrate buffer containing orthophenylenediamine and $H_2O_2$ as an enzyme substrate was added to each well, and the enzymatic reaction was conducted at room temperature. The reaction was terminated with 1 N sulfuric acid, and the resultant colored material was measured at the wavelength of 492 nm using Multiskan (Flow Laboratories)

Reference Example 2

Production of a mouse monoclonal anti-human fibrin antibody-producing hybridoma

(1) Immunogen preparation

The human fibrin β chain N terminal peptide (1-11)-Cys (3.3 mg) prepared by a known solid-phase synthesis method using peptide synthesizer (Applied Systems model 430A) was added to an aqueous solution (12 mg/2 ml) of bovine serum albumin (hereinafter also abbreviated as BSA) preliminary maleimidated with N-(γ-maleimidobutyryloxysuccinimide) (hereinafter also abbreviated as CMBS) (13 moles of maleimido residue are introduced per mole of BSA), and the mixture was incubated at 30°C for 1 hour to give a human fibrin β chain N terminal peptide (1-11)-BSA conjugate. This conjugate was dialyzed three times with physiological saline (3 liters x 3) and stored under freezing conditions and then used as an immunogen.

(2) Immunization

Equal amount of Freund's complete adjuvant was added to a 1 mg/ml solution of the peptide-BSA conjugate in physiological saline, followed by subcutaneous immunization of mice (female, n = 10; 0.1 mg/0.2 ml/mouse) at the back and abdomen. Additional immunization was conducted by inoculating a mixture of equal volumes of the immunogen and Freund's incomplete adjuvant 5 times at intervals of 2 to 3 weeks.

(3) Cell fusion

Three days after the final immunization, the spleen was excised and a splenocyte suspension was prepared in the conventional manner (about $10^8$ cells). Then, 2 x $10^7$ mouse myeloma (P3U1) cells were added to the suspension and the mixture was subjected to cell fusion according to the method of Köhler and Milstein [Nature, 256, 495 (1975)] using polyethylene glycol (hereinafter also abbreviated as PEG) 6000.

After completion of the fusion procedure, the cell mixture was suspended in the HAT medium which contains hypoxanthine, aminopterin and thymidine, and cultured for 10 days. Thereafter, subculturing was repeated, the medium being replaced by HT medium (HAT medium minus aminopterin) upon completion of parent cell selection.

(4) Hybridoma selection and cloning

The hybridoma culture supernatant was assayed for antibody titer in the presence of human fibrinogen (5 mg/ml), by the EIA as described in Reference Example 1, which uses a microplate with human fibrin monomer adsorbed on the solid phase. Ten to twenty days after fusion, hybridoma appearance was observed and an antibody specifically binding to human fibrin was also detected. Hybridomas showing particularly strong binding activity were submitted for cloning by the limiting dilution method.

The culture supernatants obtained with the cloned hybridomas were subjected to screening by EIA in the same manner as mentioned above for selecting a clone showing high fibrin binding activity.

As a result, the mouse hybridoma FIB1-11 producing a MoAb specifically binding to human fibrin in the presence of a high concentration of human fibrinogen was obtained. The immunoglobulin class and sublcass of the antibody produced by the hybridoma thus obtained were $IgG_1$ (κ chain).

Reference Example 3

Cloning of the human genomic Cκ gene

Macromolecular DNA was prepared from human plasmablast ARH77 cells (hereinafter also abbreviated as ARH77) and digested with EcoRI, and a genomic DNA library was produced by inserting the digest into the λgtWESλB phage vector [Y. Nishimura et al.: Cancer Research, 47, 999 (1987)]. Then, a clone containing the human genomic Cκ gene was obtained by plaque hybridization using the mouse Cκ gene as a cross hybridization probe.

Reference Example 4

Cloning of the human genomic Cγ1 gene

The EcoRI digest of the ARH77 cell-derived macromolecular DNA as described above in Reference Example 3 was inserted into the λ[Charon 4A phage vector to give a genomic DNA library [A. Kudo et al.: Gene, 33, 181 (1985)]. Then, about $10^6$ phages were screened by plaque hybridization using the 3.5 kb human $J_H$ gene fragment as a probe. As a result, a clone containing the human genomic Cγ1 gene was obtained.

Reference Example 5

Cloning of the NL-1-derived genomic $V_H$ gene

DNA was extracted from the mouse hybridome NL-1 (hereinafter also abbreviated as NL-1) line [A. Kudo et al.: Journal of Immunology, 135, 642 (1985)] producing the antibody $IgG_{2a}$ ($\varkappa$ chain) capable of recognizing the common acute lymphocytic leukemia antigen (hereinafter also abbreviated as CALLA) and cleaved with EcoRI, and the cleavage mixture was inserted into the Charon 4A vector to give a genomic DNA library. This library was screened by plaque hybridization using the 1.5 kb mouse $J_{H4}$ EcoRI-HindIII fragment as a probe. And, as a result, phage clones having a 8.1 kb and a 7.9 kb insert fragment, respectively, were obtained.

Northern blot analysis using the whole RNA of NL-1 cells and nucleic acid sequence determination by dideoxy chain termination revealed that the above-mentioned 7.9 kb insert fragment contained a recombinant type heavy chain gene ($V_H$ gene) for the NL-1 antibody molecule.

Reference Example 6

EIA for anti-urokinase antibody assay

A 5 µl/ml solution of a commercial grade of urokinase (produced and distributed by Nihon Seiyaku) was distributed in 100 µl portions into wells of a 96-well microtiter plate. The plate was allowed to stand overnight at 4°C. Then 150 µl of PBS containing 2% casein and 0.01% thimerosal was added to each well for sensitized plate preparation. After removing of the liquid mentioned above and washing the plate with PBS-Tw, 100 µl of the culture supernatant to be tested was added, followed by reaction at room temperature for 2 hours. Thereafter, the enzymatic reaction was performed as described in Reference Exampel 1 for antibody titer determination.

Reference Example 7

EIA for anti-fibrin anti-urokinase bispecific antibody assay

The bispecific antibody-containing solution to be tested was added to a urokinase-sensitized plate prepared in Reference Example 6 and followed by reaction at room temperature for 2 hours. The plate was washed with PBS-Tw, the human fibrin $\beta$ chain N-terminal peptide (1-11)-BSA conjugate prepared in Reference Example 2-(1) and labeled with biotin was added, and the reaction was allowed to proceed at room temperature for 2 hours. Then, an avidin-HRP conjugate was added and, after 1 hour of reaction at room temperature, the activity of HRR bound to the solid phase was measured by the method described in Reference Example 1.

Reference Example 8

Fibrinolysis neutralization test

The hybridoma culture supernatant dilution to be tested was added to a urokinase solution (final concentration 25 ng/ml) and, after 1 hour of incubation at 37°C, the reaction mixture was poured, in 5-µl portions, into wells of a fibrin-agarose plate. After 2 to 6 hours of incubation at 37°C, the diameter of each spot resulting from fibrinolysis was measured and the neutralizing capacity of the MoAb contained in the hybridoma culture supernatant against the enzymatic activity of urokinase was determined.

Reference Example 9

Production of a mouse monoclonal anti-urokinase antibody producing hybridoma

(1) Immunization

To a 200 µg/ml solution of urokinase (produced and distributed by Nihon Seiyaku) in physiological saline was added an equal volume of Freund's complete adjuvant. After thorough emulsification, the emulsion was administered to BALB/c mice (female, 20 µg/0.2 ml/mouse) subcutaneously at the peritonal cavity and the back, followed by booster doses at 2- to 3-week intervals. Individuals showing a maximum serum antibody titer in 10 days after 3 booster injections were given a urokinase antigen solution (50 µg/0.1 ml physiological saline/mouse) by intravenous administration.

(2) Cell fusion

Cell fusion was carried out by the method described in Reference Example 2-(3).

(3) Hybridoma selection and cloning

Hybridomas were screened by EIA using a urokinase-bound microtiter plate as described in Reference Example 6, and anti-urokinase MoAb-producing hybridomas were obtained in the same manner as in Reference Example 2-(4). Among these, a mouse hybridoma designated as UK1-3 was obtained as a hybridoma producing an anti-urokinase MoAb capable of specific binding without loss of fibrinolytic activity in the test described in Reference Example 8. The antibody UK1-3 produced by the hybridoma obtained was found to belong to the immunoglobulin class and subclass $IgG_1$ when tested by the Ouchterlony method.

Reference Example 10

Production of a monoclonal hybrid antibody having anti-urokinase, anti-human fibrin bispecificity

(1) Cell fusion

The anti-human fibrin antibody-producing hybridoma FIB1-11 obtained in Reference Example 2 and the anti-urokinase antibody-producing hybridoma UK1-3 obtained in Reference Example 9 were respectively incubated at 37°C for 30 minutes for fluorescent staining in Iskov-Ham F-12 mixed medium (hereinafter also abbreviated as IH) containing 0.5 μg/ml of fluorescein isothiocyanate (FITC) and 1.5 μg/ml of tetramethylrhodamine isothiocyanate (TRITC). Then, LSM solution (distributed by Wako Pure Chemical Industries) was added and dead cells were removed. Both hybridomas were mixed in a 1:1 ratio and cell fusion was done by the method described in Reference Example 2-(3) using PEG 6000. After 2 hours of incubation at 37°C, the mixture was submitted to a fluorescein-activated cell soter (FACS), followed by separation of 25,000 cells double stained with fluorescein and rhodamine. The above-mentioned double stained cells were inoculated, in an amount of 10 cells per well, into wells of a 96-well microtiter plate inoculated with 5 x $10^5$ mouse thymocytes per well as feeder cells and cultured [cf. European Unexamined Patent Publication No. 363712].

(2) Hybrid hybridoma selection and cloning

The culture supernatant in each of the wells where cell proliferation was observed in 1 to 2 weeks after fusion was subjected to the EIAs described in Reference Examples 1, 6 and 7 to determine antibody activity.

For the well showing the highest hybrid antibody activity, cloning was performed by the limiting dilution method and the desired bispecific antibody-producing mouse hybrid hybridoma FU1-74 was obtained.

(3) Hybrid antibody purification

The ascitic fluid was collected by a per se known method, then subjected to salting out with ammoniumn sulfate and immunoaffinity chromatography using a fibrin-bound column and a urokinase-bound column. About 20 ml of ascitic fluid gave about 28 mg of FU1-74 an anti-urokinase - anti-human fibrin bispecific antibody of the present invention.

Reference Example 11

EIA for human IgG antibody assay

A 5 μg/ml solution of a commercial grade of goat anti-human IgG (Fc fragment) antibody (Cappel) was distributed in 50-μl portions into wells of a 96-well microtiter plate. After overnight standing at 4°C, 150 μl of PBS containing 2% casein and 0.01% thimerosal was added to each well. The thus-prepared antibody-sensitized plate was washed with PBS-Tw, then 50 μl of the culture supernatant to be tested was added, and the reaction was allowed to proceed a room temperature for 2 hours. The enzymatic reaction was conducted as described in Reference Example 1 for antibody titer assaying. A commercially available, chromatography-purified grade of human IgG (Cappel) was used as a standard human IgG.

Example 1 Construction of the vector $pSV_2$-$hF_K$ for mouse-human chimeric light chain expression

(1) Cloning of genomic DNA

Macromolecular genomic DNA was prepared from the mouse anti-human fibrin specific antibody producer hybridoma FIB1-11 obtained in Reference Example 2 and cleaved with the restriction enzyme EcoRI, and the cleavage product was subjected to centrifugation on a 10-40% sucrose density gradient. DNA fragments of 12-20 kb in size were collected and inserted into the EMBL4 phage vector (Strata-Gene) to give a genomic DNA library [Y. Nishimura et al.: Cancer Research, 47, 999 (1987)]. Screening using a $^{32}$P-labeled mouse $J_{K4\text{-}5}$ gene fragment (0.7 kb AvaI-PstI fragment) as a hybridization probe gave three positive recombinant phage clones. Using the insert DNA fragments of these clones as probes, Northern blot analysis of the whole RNA of FIB1-11 cells was performed, whereby a phage clone designated as KE14 was identified as a clone containing a 15 kb EcoRI fragment coding for the $V_K$ gene expressed in FIB1-11 cells (hereinafter also abbreviated as $V_{FK}$ gene).

(2) Nucleic acid sequence determination

The phage clone KE14 was subcloned in the plasmid vector pUC119 and the nucleic acid sequence was determined by the known dideoxy chain termination method. It was found that the DNA fragment contained a transcription promoter (octamer sequence), a leader peptide gene and the $V_K$ structural gene. $V_{FK}$ was a functional $V_K$ gene formed by recombination between the $V_K$ gene belonging to Subgroup II ($V_K$II) and the $J_{K2}$ gene.

The results were as shown in Fig. 1 [Seq. ID No. 11]

(3) Expression vector construction

A 2.6 kb EcoRI-PstI fragment containing the genomic $V_{FK}$ gene was isolated from the 15 kb insertion DNA fragment contained in the phage clone KE14 mentioned in above (1), and inserted into the EcoRI-PstI site of pUC119. The thus-obtained plasmid was cleaved with EcoRI and changed the termini to blunt end by reacting $T_4$ polymerase (hereinafter also abbreviated as $T_4$ pol), followed by joining of a BamHI linker and cleavage with BamHI and HindIII to give a 2.6 kb fragment.

Separately, a 2.5 kb EcoRI fragment isolated from the human plasmablast cell line ARH77 and containing the human $\varkappa$ chain constant region genomic gene (hereinafter also abbreviated as $hC_K$) was inserted into the plasmid vector pBR322 at the EcoRI site thereof. The thus-obtained plasmid was partially cleaved with EcoRI and a 6.9 kb fragment was isolated. The fragment was further cleaved with BamHI and a 2.9 kb fragment was isolated. This DNA fragment was inserted into the pSV2-neo vector at the EcoRI-BamHI site thereof. The plasmid thus obtained was further cleaved with HindIII and BamHI and two fragments, 5.3 kb and 2.4 kb in size, were isolated.

These two DNA fragments were ligated with the above $V_{FK}$ gene-containing 2.6 kb BamHI-HindIII fragment to give a plasmid containing the $V_{FK}$ gene and $hC_K$ gene in that order in the same direction.

Separately, a human IgH enhancer-containing 0.9 kb MluI-HpaI fragment isolated from ARH77 was changed the termini to blunt end by reacting $T_4$ pol therewith and inserted into pUC119 at the EcoRI site thereof using an EcoRI linker. The plasmid obtained was cleaved with EcoRI, an enhancer-containing 0.9 kb fragment was isolated and rendered blunt end using $T_4$ pol, and a BamHI linker was joined thereto. This enhancer fragment was inserted into the above-mentioned plasmid (the pSV2-neo vector containing the $hC_K$ gene and $V_{FK}$ gene) at the BamHI site thereof. A $\varkappa$ chain expression vector, pSV2-$hF_K$ was thus constructed.

The construction scheme was as shown in Fig. 2.

Example 2 Construction of a mouse-human chimeric heavy chain expression vector, pSV2-hFH

(1) Cloning of cDNA

RNA was prepared from the FIB1-11 cells described in Reference Example 2 and further purified using oligotex-dT30 (Takara Shuzo) to give poly(A)$^+$-RNA. A reaction mixture (50 $\mu$l) containing 10 $\mu$g of this

poly(A)$^+$-RNA, 20 pmol 3'mV$_H$ primer, 2 $\mu$M each dNTP, 10 mM dithiothreitol (hereinafter also abbreviated as DTT), 100 mM Tris-HCl (pH 8.3), 10 mM $MgCl_2$ and 140 mM KCl was treated at 70 °C for 10 minutes and then returned to room temperature. Reverse transcriptase (46 units) was added and incubation was performed at 42 °C for 1 hour. To 50 $\mu$l of a reaction mixture containing 5 $\mu$l of above reaction mixture together with 25 pmol 5'mV$_H$ primer, 25 pmol 3'mV$_H$ primer, 250 $\mu$M each dNTP, 67 mM Tris-HCl (pH 8.8), 10 mM $MgCl_2$, 17 mM $(NH_4)_2SO_4$ and 200 $\mu$g/ml gelatin was added 2 units of Taq polymerase. After topping with liquid paraffin, the polymerase chain reaction (hereinafter also abbreviated as PCR) was carried out. The temperature cycle was as follows: 95 °C, 1 minute; 52 °C, 2 minutes; and 72 °C, 2 minutes. After 30 reaction cycles, the sample was subjected to 5% polyacrylamide electrophoresis (hereinafter also abbreviated as PAGE) and a DNA fragment of about 330 bp as amplified was isolated. This had the nucleic acid sequence of the PCR primer used and contained a PvuII site and a BstEII site as cloning sites. The results obtained were as shown in Fig. 3.

5'mV$_H$: 5'-AGGTGCAGCTG(G/T)(G/T)G(G/C)AGTC(G/T)GG-3'
22-mer [Seq. ID No. 9]
3'mV$_H$: 5'-TGAGGAGACGGTGACCAGGGTCCCTTGGCCCCAG-3'
34-mer [Seq. ID No. 10]

(2) Nucleic acid sequence determination

V$_H$ gene-containing DNA fragment (about 330 bp) obtained by the PCR method described in 1) was isolated and subcloned in pUC119, and the nucleic acid sequence was examined. Said fragment was found to be a functional V$_H$ structural gene (hereinafter also abbreviated as V$_{FH}$) comprising a V$_H$ gene belonging to the subgroup III (V$_H$III) and the D$_{SP2}$ and J$_{H4}$ genes. Northern blot analysis using the whole RNA of FIB1-11 cells confirmed that V$_{FH}$ was the gene expressed in FIB1-11 cells.

The results were as shown in Fig. 4 [Seq. ID No. 12].

(3) Construction of an expression vector

To expression chimeric H chain using the V$_{FH}$-cDNA obtained by the PCR method described in (1), a promoter, a leader and a splicing signal sequence which were necessary for the expression in eukaryotic cells, were provided in addition to the human $\gamma$ chain constant region gene hC$_{\gamma1}$. Thus, utilizing the genomic V$_H$ gene derived from mouse hybridoma NL-1 as described in Reference Example 5, a chimeric genomic V$_{FH}$ gene having the promoter, leader and splicing sequences derived from the genom for the antibody H chain produced by NL-1 cells as well as the V$_H$ exon derived from FIB1-11 cells was constructed. A 2.8 kb PvuII fragment of pUC119 and a 2.1 kb EcoRV-BglII genomic DNA fragment containing the V$_H$ gene derived from NL-1 cell (hereinafter also abbreviated as V$_{HNL1}$) were ligated together via an EcoRI linker. Then, the obtained plasmid was deprived of the PvuII-BstPI fragment of the V$_{HNL1}$ exon portion, which fragment was replaced with a 0.34 kb PvuII-BstPI fragment of the above-mentioned V$_{FH}$-CDNA. From the resultant plasmid, a 2.1 kb EcoRI fragment containing the chimeric genomic V$_{FH}$ gene was prepared.

Separately, an EcoRI fragment obtained from human plasmablast ARH77 cells and containing the human C$_{\gamma1}$ gene and human heavy chain enhancer was cleaved with MluI and then rendered blunt-ended using T$_4$ polymerase. An EcoRI linker was joined to this fragment, the resultant fragment was cleaved with EcoRI and BamHI. A 13 kb fragment was isolated and inserted into the pSV2-gpt vector at the EcoRI-BamHI site thereof. The plasmid obtained was cleaved with EcoRI and the above-mentioned chimeric genomic V$_{FH}$ gene-derived EcoRI fragment was inserted thereinto. A plasmid in which V$_{FH}$ and hC$_{\gamma1}$ were bound in the same direction was selected. The chimeric heavy chain expression vector pSV$_2$-hFH was thus constructed.

The construction scheme was as shown in Fig. 5.

Example 3 Production of a chimeric antibody

(1) Gene introduction and transformant selection

The vectors pSV$_2$-hF$_K$ and pSV$_2$-hF$_H$ constructed in Example 1 and Example 2, respectively, were simultaneously introduced into the mouse myeloma cell line X63.Ag8.653 using the technique of electroporation. Thus, cells were washed once with PBS and suspended in PBS to a concentration of 1 x $10^7$ cells/ml. To 0.5 ml of this suspension were added 20 $\mu$g each of the plasmid DNAs ($pSV_2$-$hF_K$ and $pSV_2$-$hF_H$). Electric pulses were applied to the suspension using GenePulserTM (Bio-Rad). The suspension was allowed to stand for 10 minutes with ice cooling, then a medium containing 10% FCS was added thereto and cultivation was performed. Transformant selection was performed using a medium containing 6.5 $\mu$g/ml of mycophenolic acid and 1.0 mg/ml of G418.

(2) Cloning of chimeric antibody-producing cells

An antibody having the human immunoglobulin constant region was detected in the culture supernatant in one of f13 wells showing cell proliferation in the above-mentioned selection medium. This culture supernatant was also subjected to EIA using the HRP-labeled anti-human IgG antibody described in Reference Example 1, whereby it showed anti-human fibrin antibody activity as well. The cells were thus found to be the desired cells producing a chimeric antibody having anti-fibrin activity.

Therefore, cloning was conducted by the limiting dilution method and cloned cell culture supernatants were submitted for screening by EIA which was carried out in the same manner. Thus, a cloned mouse-human chimeric antibody producing cell line, FIB1-HO1/X63 was obtained, which produces an antibody with strong human fibrin-binding ability.

(3) Characteristics of the chimeric antibody-producing cells

FIB1-HO1/X63 cells were seeded at a concentration of 2 x $10^4$ cells/ml, and cultured with determing the number of cells grown and the antibody titer in the culture supernatant at timed intervals. The antibody titer was determined by EIA using a goat anti-human IgG antibody-sensitized microplate described in Reference Example 11.

The results obtained were as shown in Fig. 6. The doubling time was about 1 day (24 hours) and the rate of the antibody production at that time was about 2 $\mu$g/ml.

(4) Characteristics of the chimeric antibody

The chimeric antibody produced by FIB1-HO1/X63 cells was compared with the mouse anti-human fibrin antibody produced by the mouse hybridoma FIB1-11 prepared in Reference Example 2. Thus, both antibodies (final concentration 50 ng/ml) were reacted with various concentrations of the peptide-BSA conjugate described in Reference Example 2-(1) at room temperature for 1 hour. The reaction mixtures were then submitted for EIA, which was carried out in the same manner as in Reference Example 1.

The results obtained were as shown in Fig. 7. The peptide-BSA conjugate concentration required for 50% inhibition of the binding of the anti-fibrin antibody to the fibrin monomer-sensitized plate was 3.2 ng/ml for the chimeric antibody FIB1-HO1/X63 and 4.6 ng/ml for the mouse antibody FIB1-11. These results revealed that the chimeric antibody was substantially comparable in affinity for fibrin to the original mouse antibody.

(5) Purification of the chimeric antibody

Chimeric antibody-producing FIB1-HO1/X63 cells were intraperitoneally inoculated, at a dose of $10^7$ cells/mouse, into hybrid nude mice (Jcl:AF-nu) preliminarily given 0.5 ml of mineral oil by intraperitoneal injection. About 10 to 20 days after inoculation, a 20 ml of the accumulated ascitic fluid was collected and subjected to salting out with 50% saturated ammonium sulfate to give an IgG fraction. This fraction was dialyzed against 20 mM PBS (pH 7.5) and then applied to a fibrin-bound Cellulofine column. Elution was carried out with 0.2 M glycine hydrochloride buffer (pH 2.9). The acidic eluate fraction was dialyzed against PBS to give the mouse-human chimeric anti-fibrin specific antibody.

About 12 mg of the mouse-human chimeric anti-fibrin specific antibody was obtained from each 10 ml of the ascitic fluid.

Example 4 Construction of a mouse-human chimeric anti-human fibrin antibody cDNA expression vector (pTB1387)

(1) Preparation of a mouse-human chimeric antibody light chain cDNA

Poly(A)$^+$-RNA was prepared from the anti-fibrin chimeric antibody-producing transformant FIB1-HO1/X63 obtained in Example 3 using a FirstTruck™ mRNA isolation kit (In Vitrogen). With this poly(A)$^+$-RNA as a template, human Cκ cDNA cloning was performed using the oligo-dT (Pharmacia) primer as a primer for first strand cDNA synthesis and the 3'Eκ and 5'Cκ primers as primers for PCR. Thus, 11 μl of a reaction mixture containing 1 μg of poly(A)$^+$-RNA and 10 pM oligo-dT primer was treated at 70°C for 10 minutes and then returned to room temperature. To this reaction mixture were added Tris-HCl (pH 8.3), $MgCl_2$, KCl, DTT and four dNTPs to the final concentrations of 100 mM, 10 mM, 140 mM, 10 mM and 0.5 mM, respectively, and the final volume was made 20 μl. After further addition of 1 μl of reverse transcriptase [MMTV-RT (Moloney murine T cell leukemia virus RNase reverse transcriptase), 200 units/μl, BRL], incubation was performed at 45°C for 1 hour. To 50 μl of a reaction mixture containing 4 μl of this reaction mixture, 25 picomoles of 5'Cκ primer, 25 picomoles of 3'Eκ primer, 200 μM each dNTP, 10 mM Tris-HCl (pH 8.3), 1.5 mM $MgCl_2$, 50 mM KCl and 0.01% gelatin was further added 2 units of Taq DNA polymerase (Cetus kit) and, after top layering of liquid paraffin, the PCR was performed. The temperature cycle was as follows: 95°C, 1 minute; 52°C, 2 minutes; and 72°C, 3 minutes. After 30 cycles of reaction, the sample was subjected to 5%-PAGE. An amplified DNA fragment of about 0.33 kb was isolated and joined to the SmaI-cleaved plasmid pUC119 (Takara Shuzo) to give a Cκ cDNA-containing vector, pTB1394. Using the same technique as mentioned above, with the 3'Eκ primer as a primer for first strand synthesis and the 5'Lκ and 3'Cκ primers as primers for PCR, an anti-fibrin Vκ cDNA having a BclI site at the 3' terminal (hereinafter also abbreviated as Vκv) was amplified. Furthermore, using the 3'Eκ primer as a primer for first strand synthesis and the 5'mVκ and 3'mVκ primers as primers for PCR, an anti-fibrin Vκ cDNA (with a BglII site at the 3' terminal; hereinafter also abbreviated as Vκ-FIB) was amplified. In addition, a leader sequence cDNA (hereinafter also abbreviated as Lκ) was amplified using the 3'Cκ primer as a primer for first strand synthesis and the 5'Sκ and 3'Lκ primers as primers for PCR. The amplified gene fragments (Lκ: 0.07 kb; Vκv: 0.35 kb; Vκ-FIB: 0.35 kb) were respectively isolated and joined to SmaI-cleaved pUC119 to give the corresponding Lκ-, Vκv- and Vκ-FIB-containing plasmids, pTB1391, pTB1392 and pTB1393.

After confirmation of the nucleic acid sequences of the cDNA fragments obtained, Lκ, Vκ and Cκ were joined together in the right direction to give a plasmid, pTB1427, containing the whole length of the chimeric κ chain cDNA (Fig. 8).

Thus, pTB1391 was digested with the restriction enzymes PstI and MluI and a 0.1 kb DNA fragment containing the Lκ cDNA was isolated. Similarly, pTB1392 was digested with SplI and MluI and a 0.35 kb DNA fragment containing the Vκv cDNA was isolated. Separately, pTB1394 containing the Cκ cDNA was cleaved with SplI and PstI. To this digest were added the 0.07 kb PstI-MluI fragment and 0.35-kb SplI-MluI fragment prepared in advance, and simultaneous ligation was carried out to give a plasmid, pTB1427, containing the whole length of the chimeric antibody κ chain open reading frame (Lκ, Vκ and Cκ each having the right direction; hereinafter also abbreviated as Igkv).

The Vκv cDNA used in the above process contained a BclI site as a restriction site for cloning the Vκ cDNA amplified by PCR. However, introduction of this site led to amino acid substitution at one position (Glu 130 - Val 130). Therefore, for correcting this amino acid substitution, the following measure was taken.

Thus, pEcoRI linker (GGAATTCC, Takara Shuzo) was joined to PvuII-cleaved pUC119 and, after further cleavage with EcoRI, a 2.8 kb fragment was isolated. A plasmid, pTB1405, was constructed by ligating this DNA fragment to a 0.73kb EcoRI fragment isolated from pTB1427 and containing the Igkv cDNA. The plasmid pTB1405 was cleaved with Bc1I and PvuII, followed by ligation to a 0.33 kb PvuII-Bg1II fragment isolated from pTB1393 and containing the Vκ-FIB cDNA. Thus, a plasmid, pTB1410, containing the chimeric κ chain cDNA gene (Igk-FIB) restored to the original form (Val 130 → Glu 130) was obtained.

The whole nucleic acid sequence of the chimeric κ chain c DNA contained in pTB1427 is shown in Fig. 9.

The nucleic acid sequences of the primers used in this experiment are shown below.

5'SK 5'-AGAATTCCGCC ATG ATG AGT CCT GCC CAG TTC CTG-3' [Seq. ID No. 28]

3'LK 5'-C ACG CGT TTC CCG AAT CCA GAG CAC TAA-3' [Seq. ID No. 27]

5'LK 5'-A ACG CGT GGT GAT ATT CAG CTG GCC CAG ACT CCA CTFC ACT-3' [Seq. ID No. 26]

3'CK 5'-C CGT ACG TTT GAT CAC CAG CTT GGT CCC CCC TCC GAA-3' [Seq. ID No. 25]

5'CK 5'-A CGT ACG GTG GCT GCA CCA TCT GTC T-3' [Seq. ID No. 24]

3'EK 5'-AGAATT CTA ACA CTC TCC GCG GTT GAA GCT CTT TGT GAC-3' [Seq. ID No. 23]

3'mVK. 5'-G TTA GAT CTC CAG CTT GGT CCC-3' [Seq. ID No. 40]

5'mVk. 5'-GAC ATT CAG CTG ACM CAG WCT CCA-3' [Seq. ID No. 39]

(2) Preparation of a mouse-human chimeric antibody heavy chain cDNA

Using essentially the same technique as mentioned above (1), a plasmid, pTB1373, containing the whole length of a mouse-human chimeric anti-human fibrin heavy chain cDNA open reading frame was prepared. Thus, using the poly(A)$^+$-RNA prepared from FIB1-HO1/X63 cells as above (1) as a template, an oligo-dT primer as a primer for first strand cDNA synthesis and the primers 5'C2H and 3'EH as primers for PCR, a human $\gamma_1$ chain CH2-CH3 domain-encoding cDNA (hereinafter also abbreviated as CH2CH3) was amplified. Similarly, a human $\gamma_1$ chain CH1 domain-encoding cDNA (hereinafter also abbreviated as CH1), an anti-fibrin antibody $V_H$ cDNA (hereinafter also abbreviated as $V_H$-FIB) and a leader peptide cDNA (hereinafter also abbreviated as $L_H$) were amplified using the primer 3'EH, 3'C2H and 3'C1H, respectively, as a primer for first strand cDNA synthesis and the primer combination of 5'C1H and 3'C2H, of 5'LH and 3'C1H and of 5'SH and 3'LH, respectively, as primers for PCR. The amplified gene fragments ($L_H$, 0.08 kb; $V_H$-FIB, 0.35 kb; CH1, 0.33 kb; CH2CH3, 0.67 kb) were respectively isolated and ligated to SmaI-cleaved pUC119 to give plasmids, pTB1386, pTB1389, pTB1388 and pTB1390, containing $L_H$, $V_H$-FIB, CH1 and CH2CH3, respectively.

After confirmation of the nucleic acid sequence of each cDNA fragment obtained, $L_H$, $V_H$, CH1 and CH2CH3 were joined together in the appropriate direction [Fig. 10].

Thus, pTB1388 was digested with the restriction enzymes HindIII and XhoI, a CH1-containing 0.33 kb DNA fragment was isolated. Separately, the $V_H$-FIB1-containing plasmid pTB1389 was digested with HindIII and XhoI, followed by ligation to the previously prepared 0.33 kb CH1 fragment, to give a plasmid, pTB1371, containing the $V_H$-FIB1 and CH1 cDNAs each in the appropriate direction. Similarly, after digestion with the restriction enzymes EcoRI and SpeI, this pTB1371 was ligated to a 0.08 kb EcoRI-SpeI fragment isolated from the plasmid pTB1386 and coding for the $L_H$ cDNA to give a plasmid pTB1372 containing the $L_H$, $V_H$-FIB1 and CH1 cDNAs each in the appropriate direction.

Further, a 0.74 kb EcoRI-PmaCI fragment containing the $L_H$, $V_H$ and CH1 cDNAs was isolated from said plasmid pTB1372, and a 0.67 kb EcoRI-PmaCI fragment containing the CH2CH3 cDNA from the plasmid pTB1390. These two fragments were subjected to simultaneous ligation to EcoRI-digested pUC119 to give pTB1373 containing the whole-length chimeric H chain ($L_H$, $V_H$, CH1, CH2CH3; hereinafter also abbreviated as IgH-FIB). The whole nucleic acid sequence of the chimeric H chain cDNA contained in pTB1373 is shown in Fig. 11. The nucleic acid sequences of the primers used are shown below.

5'SH 5'-TGAATTCCACC ATG GAC TCC AGG CTC AAT-3' [Seq. ID No. 36]

3'LH 5'-CAC TAG TTG CAC CTC ACA GTC GAC ACC TTT TAA AAT AAG-3' [Seq. ID No. 35]

5'LH 5'-CAA CTA GTG GAG TCG GGG GGA GGC TTA GTG-3' [Seq. ID No. 34]

3'C1H 5'-ACT CGA GAC GGT GAC CAG GGT CCC TT-3' [Seq. ID No. 33]

5'C1H 5'-TC TCG AGT GCT AGC ACC AAG GGC CCA TCG GTC TTC-3' [Seq. ID No. 32]

3'C2H 5'-GCA CGT GTG AGT TTT GTC ACA AGA T-3' [Seq. ID No. 31]

5'C2H 5'-AC ACG TGT CCA CCG TGC CCG GCG CCT GAA CTC CTG GGG-3' [Seq. ID No. 29]

3'EH 5'-CGAATTCA TTT ACC CGG GGA CAG GGA GAG GCT-3' [Seq. ID No. 30]

(3) Construction of a vector, pTB1387, for mouse-human chimeric anti-human fibrin antibody cDNA expression

pTB1373 and pTB1410 constructed as described in above (1) and (2) were respectively cleaved with EcoRI, and an IgH-FIB-containing 1.4k b EcoRI fragment and an IgK-FIB-containing 0.73 kb EcoRI fragment were isolated. Then each fragment was joined to EcoRI-cleaved pCDL-SRα296 (expression vector for use in animal cells) to give a chimeric antibody H chain expression vector, pTB1374, and a chimeric antibody κ chain expression vector, pTB1411, respectively (Fig. 12).

pCDL-SRα296 used in this experiment is a vector produced for the expression of cDNAs in animal cells. It has the SRα promoter [composed of SV40 early promoter, SV40 replication origin, part of HTLV(I) LTR (part of R and U5) and SV40 late region intron] and the SV40 late poly A addition signal (poly A). Insertion of a cDNA into the EcoRI site occurring between said SRα promoter and poly A can result in efficient expression of the cDNA [Takabe et al.: Molecular and Cellular Biology, 8, 466-472 (1988)].

Then, a vector, pTB1387, which would enable simultaneous expression of the mouse-human chimeric H and κ chains and an animal cell selective marker gene (Eco-gpt) was constructed (Fig. 13). Thus, pHSG396 (Takara Shuzo) was cleaved with the restriction enzymes HindIII and SacI, fragments were changed their termini to blunt-ended by using T4 polymerase, and a multicloning site-containing 0.06kb DNA fragment was isolated. pCU118 (Takara Shuzo) was cleaved with PvuII and a 2.8kb fragment was isolated. This fragment was ligated to the above-mentioned multicloning site-conaining 0.06-kb fragment to give pTB1379. Separately, pMAM (Clonetech) was cleaved with BamHI, then changed its termini to blunt-ended by using T4 polymerase. After joining thereto of a pClaI linker (CATCGATG; Takara Shuzo), further cleaved with ClaI, and an E. coli gpt-containing 2.25 kb fragment was isolated. Ligation of this 2.25 kb E. coli gpt fragment to the ClaI-cleaved pTB1379 gave an E. coli gpt-containing plasmid, pTB1375.

Then, pTB1411 was cleaved with SalI and ScaI, fragments were changed their termini to blunt-ended by using T4 polymerase, and an SRα promoter/IgK-FIB/poly A site-containing 2.4 kb fragment was isolated. This DNA fragment was ligated to an XhoI-cleaved pTB1375 fragment and changed their termini to rendered blunt-ended by using T4 polymerase, whereby pTB1425 was constructed.

Further, pTB1374 was first cleaved with ScaI, then partially digested with SalI and changed the termini to blunt-ended by using T4 polymerase, and an SRα promoter/IgH-FIB/poly A site-containing 3.01 kb DNA fragment was isolated. To this DNA fragment was ligated a fragment prepared from the above-mentioned pTB1425 by cleavage with XbaI and changed the termini to blunt-ended by using T4 polymerase, whereby a vector, pTB1387, capable of expressing IgK-FIB, IgH-FIB and E. coli gpt simultaneously was obtained.

(4) Production of a mouse-human chimeric anti-human fibrin antibody

pTB1387 constructed as described in above (3) was introduced into mouse myeloma SP2/0 cells by electroporation. Transformants were selected using a medium containing 6.5 µg/ml of mycophenolic acid. The culture supernatants of 31 wells which showed cell proliferation were subjected to EIA for anti-fibrin chimeric antibody assay as described in Reference Example 1. Anti-fibrin activity was detected for 7 wells. A chimeric anti-fibrin antibody-producing transformant cell line (SS/S-3) obtained in this manner produced 150 ng/ml of antibody.

Example 5 Construction of a mouse-human chimeric anti-urokinase antibody expression vector

(1) Construction of cloning vectors

Cloning vectors, pTB1420 and pTB1423, were constructed for cloning the antibody $V_H$ gene and $V_K$ gene amplified by PCR [Fig. 14].

First, pTB1374 constructed in Example 4 was cleaved with SalI and XhoI, fragments were changed their termini to blunt-ended by using T4 polymerase, and a transcription terminator and poly A site-containing 0.48-kb DNA fragment was isolated. This DNA fragment was ligated to a 2.9-kb fragment prepared from pTB1379, which constructed in Example 4 by digestion with XbaI and BamHI followed by changing the termini to blunt-end using T4 polymerase, whereby pTB1415 was constructed. Then, pTB1374 was cleaved with ClaI and XhoI, fragments were changed their termini to blunt-end by using T4 polymerase, and a 0.64 kb DNA fragment and a 0.17 kb DNA fragment were isolated. Separately, pTB1415 was cleaved with XhoI, changing the termini to blunt-end using T4 polymerase, and subjected to simultaneous ligation to the previously prepared 0.64 kb and 0.17 kb DNA fragments, whereby a plasmid, pTB1417, containing the 0.64 kb and 0.17 kb fragments in the appropriate direction relative to the poly A site was obtained. In pTB1417, the XhoI sites originally occurring within the SRα promoter and upstream from the poly A site are deleted (hereinafter referred to as SRα'promoter).

Then, pTB1373 and pTB1427 (each constructed in Example 4) were respectively cleaved with EcoRI and an IgH-FIB-containing 1.4-kb EcoRI fragment and an Igkv-containing EcoRI fragment were isolated. pTB1417 was cleaved with SalI and, after cleavage with T4 polymerase, a pEcoRI linker (GGAATTCC; Takara Shuzo) was joined thereto. The fragment obtained by further cleavage with EcoRI was ligated to the previously prepared 1.4 kb IgH-FIB or 0.7 kb Igkv EcoRI fragment so that the latter fragment had the, appropriate direction relative to the promoter. Thus were constructed a $V_H$ gene cloning vector, pTB1420 and a $V_K$ gene cloning vector, pTB1421. As a further $V_K$ gene cloning vector, pTB1423 was prepared. Thus, pTB1421 was cleaved with HindIII and ClaI, fragments were changed the termini to blunt-end using T4 polymerase, and a pXbaI linker (CTCTAGAG; Takara Shuzo) was joined thereto. After further cleavage with XbaI, an SRα' promoter/Igkv/poly A site-containing 2 kb fragment was isolated. This 2 kb fragment was inserted into pTB1375 at the XbaI site to give pTB1423.

(2) Preparation of an anti-urokinase antibody light chain variable region cDNA

From mouse anti-urokinase antibody-producing hybridoma UK1-3 cells obtained in Reference Example 9, poly(A)$^+$-RNA was prepared using a First Truck™ mRNA isolation kit (In Vitrogen). Using this poly(A)$^+$-RNA as a template, an anti-urokinase antibody $V_K$ cDNA (hereinafter also abbreviated as $V_K$urokinase) was amplified using the same manner as mentioned in Example 4 with the mC$\varkappa$ primer as a primer for first strand cDNA synthesis and the 3'mV$\varkappa$ and 5'mV$\varkappa$ primers described in Example 4-(1) as primers for PCR. This amplified fragment was cleaved with the restriction enzymes PvuII and Bg1II, and ligated to a 6.6 kb PvuII-BclI fragment of the pTB1423 vector constructed in above (1) to give an anti-urokinase antibody $V_K$ cDNA-containing plasmid pTB1456. The nucleic acid sequence of the cDNA containing in pTB1456 was determined and it was confirmed that said cDNA was a functional $V_K$ gene. Said nucleic acid sequence is shown in Fig. 15 [Seq. ID No.22]. The nucleic acid sequence of the primer used herein is shown below.

$_mC_{\gamma 1}$5'-CAGGGGCCACTGGATAGAC-3' [Seq. ID No. 43]

(3) Preparation of an anti-urokinase antibody variable region heavy chain region cDNA

Using the UK1-3 cell poly(A)$^+$-RNA prepared in above (2) as a template, an anti-urokinase antibody $V_H$ cDNA (hereinafter also abbreviated as $V_H$urokinase) was amplified by the same manner as described in Example 4-(1) using the mC$\gamma_1$ primer as a primer for first strand cDNA synthesis and the 3'mVH2 and 5'mVHi primers as primers for PCR. An amplified $V_H$urokinase fragment of about 0.36 kb was isolated. With this DNA fragment as a template, the PCR was carried out using the $V_H$01 and $J_H$01 primers, and an amplified $V_H$urokinase fragment of about 0.37 kb was isolated. This DNA fragment was cleaved with SalI and NheI, and then joined to a pTB1420-derived 6.6 kb SalI-NheI fragment to give a $V_H$urokinase-containing plasmid, pTB1455. The nucleic acid sequence of the cDNA contained in pTB1455 was determined, whereupon it was confirmed that said cDNA was a functional $V_H$ gene [Fig. 16], [Seq. ID No.21].

The plasmids pTB1456 and pTB1455 obtained as in above (2) were simultaneously introduced into COS cells by the DEAE-dextran method [Nigel Whittle et al.: Protein Engineering, Vol. 1, No. 6, 499-505 (1987)]. Thus, COS cells were suspended in Dulbecco's modified Eagle medium (hereinafter also abbreviated as DMEM) containing 5% FCS and inoculated about 6 x $10^5$ cells into a dish for tissue culture (6 cm in diameter). After incubation overnight at 37° C, medium exchange was made. After further 2 hours of incubation, 100 $\mu$l of a 10 mg/ml DEAE-dextran (Pharmacia) solution containing 1 $\mu$g each of the plasmid DNAs (pTB1456 and pTB1455) and 2 $\mu$l of 100 mM chloroquine solution were added dropwise to the dish. Incubation at 37° C was continued further for 2 to 4 hours. The medium was removed, 2 ml of PBS containing 10% dimethyl sulfoxide (hereinafter also abbreviated as DMSO) was added and, after 2 minutes of standing at room temperature, cells were washed with PBS, 2 ml of DMEM containing 5% FCS was added, and the cells were cultured at 37° C. After 3 days, the culture supernatant was subjected to EIA for chimeric anti-urokinase antibody assay using HRP-labeled anti-human IgG antibody as described in Reference Example 6, whereupon anti-urokinase antibody activity was detected. It was thus found that pTB1455 and pTB1456 contained the $V_H$ gene and $V_K$ gene, respectively, affording the desired anti-urokinase activity. The nucleic acid sequences of the primers used are shown below.

mCK-1 5'-CATTTTGTCGTTCACTGCCATC-3'
[Seq. ID No. 44]

5'mVHi 5'-AT GTG CAA CTA GTG GAG TCS GG-3'
[Seq. ID No. 37]

3'mVH2 5'-ATTAACT CGA GAC GGT GAC CGT GGT CCC TTG GCC CCA-3'
[Seq. ID No. 38]

VHO1. 5'-TC GTC GAC TGT GAG GTG CAA CTA GTG GAG-3'
[Seq. ID No. 41]

JHO1. 5'-TC GCT AGC ACT CGA GAC GGT GAC CG-3'
[Seq. ID No. 42]

(4) Construction of a mouse-human chimeric anti-urokinase antibody expression vector (pTB1458)

The plasmid pMAM-neo (Clonetech) was cleaved with BamHI and then was changed the termini to rendered blunt-end by using T4 polymerase, and a neomycin resistance gene-containing 2.7 kb DNA fragment was isolated. Ligation of this 2.7 kb fragment to a DNA fragment prepared from pTB1455 obtained in above (3) by cleavage with HindIII and the subsequent changing the termini to blunt-end led to construction of pTB1457.

Then, pTB1456 obtained in above (2) was cleaved with XbaI and changed the termini to blunt-end by using T4 polymerase, and a chimeric $\kappa$ chain cDNA-containing 2.0 kb fragment was isolated. Ligation of this 2.0 kb fragment to a DNA fragment prepared from pTB1457 by cleavage with ClaI and the subsequent changing the termini to end to gave pTB1458. pTB1458 is a vector capable of simultaneous expression of the chimeric anti-human urokinase heavy chain, chimeric anti-human urokinase light chain and neomycin resistance genes.

The above process is as shown in Fig. 17.

(5) Production of a mouse-human chimeric anti-human urokinase antibody

pTB1458 constructed in above (4) was introduced into mouse myeloma SP2/0 cells by electroporation. Transformants were selected on a medium containing 1 mg/ml of G418. The culture supernatants from 72 wells showing cell proliferation were subjected to EIA for chimeric anti-urokinase antibody assay using HRP-labeled anti-human IgG antibody as described in Reference Example 6, whereupon anti-urokinase antibody activity was detected for all wells. It was thus found that the transformant cells were the desired chimeric anti-urokinase antibody-producing cells.

Therefore, cloning was carried out by the limiting dilution method, and the cloned cell culture supernatants were subjected to the same EIA as mentioned above. In this way, a mouse-human chimeric antibody-producing cell clone, SU/S-9.21, capable of stably producing an antibody having highly binding affinity to human urokinase was obtained.

(6) Features of chimeric antibody-producing cells

SU/S-9.21 cells were seeded at a concentration of 1 x $10^5$ cells/ml and the antibody titer in each culture supernatant was determined at timed intervals. The antibody titer was determined by EIA using a goat anti-human IgG antibody-sensitized microtiter plate. The results obtained are shown in Fig. 18. The antibody production was about 18 $\mu$g/ml.

Example 6 Production of a mouse-human chimeric anti-human fibrin-anti-human urokinase bispecific antibody (1)

(1) Gene introduction and transformant cell line selection

The vector pTB1458 constructed in Example 5 was introduced into the mouse-human chimeric anti-fibrin antibody-producing transformant cell lines SS/S-3 (obtained in Example 4) by electroporation. Novel transformant cell line selection was performed using a medium containing 6.5 $\mu$g/ml of mycophenolic acid and 1.0 mg/ml of G418.

The culture supernatant of each of 48 wells in which cell proliferation was observed in the above medium was subjected to EIA for anti-fibrin-anti-urokinase bispecific antibody assay as described in Reference Example 7. Bispecific antibody activity was detected for 17 wells.

Therefore, the wells showed high bispecific antibody acivity, were carried out by the limiting dilution method and the cloned cell culture supernatants were subjected to screening by the same EIA as mentioned above. In this way, a mouse-human chimeric bispecific antibody-producing cell clone, SUSF/S-8.4 (which produces the desired bispecific antibody stable) was obtained.

(2) Features of bispecific chimeric antibody-producing cells

SUSF/S-8.4 cells were seeded at a concentration of 1 x $10^6$ cells/ml and inculated 24 hours, the culture supernatant was tested for bispecific antibody activity by the same EIA procedure as described in Reference Example 7. The results are shown in Fig. 19.

Example 7 Production of a mouse-human chimeric anti-human fibrin-anti-urokinase bispecific antibody (2)

(1) Maleimidation of a chimeric anti-fibrin antibody

10 mg of the chimeric anti-fibrin antibody FIB1-HO1/X63 obtained in Example 3-(5) was dissolved in 2 ml of 5 mM acetate buffer (pH 5.0), a dimethylformamide solution (50 $\mu$l) containing twice as much molar N-($\epsilon$-maleimidocaproyloxy)succinimide ester was added, and the reaction was carried out at 30 ° C for 20 minutes. The reaction mixture was applied to a Sephadex G-25 column which is equilibrated with 0.1 M phosphate buffer (pH 6.5) to remove the coupling agent.

(2) Chimeric anti-urokinase antibody sulfhydrylation

Chimeric anti-urokinase antibody producing SU/S-9.21 cells obtained in Example 5-(5) were grown in ascites by the method described in Example 3-(5). 10 mg of the thus-obtained purified antibody was dissolved in 2 ml of 0.05 M PBS (pH 7.3), and a methanol solution (50 µl) containing twice as much molar SPDP was added. The reaction was conducted at 30° C for 30 minutes, then 50 µl of 0.1 M aqueous DTT solution was added to effect reduction. The reaction mixture was applied to Sephadex G-25 column prepared the same manner as described in above (1) to remove the excess of reagent.

(3) Production of a bispecific chimeric antibody

To 8 mg of the maleimidated anti-fibrin antibody obtained in (1) was added 8 mg of the sulfhydrylated anti-urokinase antibody prepared in (2) gradually with ice cooling and stirring. Then the reaction was allowed to proceed overnight. The reaction mixture was applied to a Sephacryl S-200 column for separating the unreacted antibodies from the chemically bound bispecific antibody . As a result, the desired chimeric anti-human fibrin-anti-urokinase bispecific antibody was obtained in a yield of about 7 mg.

(4) Binding ability of the bispecific antibody

The bispecific antibody produced in (3) was subjected to the same EIA as described in Reference Example 3, whereupon it showed strong binding ability to both human fibrin and prourokinase, like the bispecific chimeric antibody described in Example 6-(2).

Example 8 Enhancement of the fibrinolytic activity of prourokinase (1)

Plasma clot lysis assay was performed by a per se known method [D. Collen et al.: Thrombosis and Haemostasis, 45, 225 (1981)]. Thus, the bispecific chimeric antibody prepared by culturing bispecific chimeric antibody-producing cell line SUSF/S-8.4 which obtained in Example 6-(1) in ascitic fluid by the method described in Example 3-(5) was added in various concentrations to a predetermined amount of prourokinase (final concentration 250 ng/ml), and the reaction was carried out at room temperature for 20 minutes. Human plasma was added to each prourokinase-antibody mixture and then human thrombin was added to a final concentration of 10 U/ml to thereby cause plasma coagulation.

The turbidity of plasma was measured at timed intervals using a euglobulin lysis analyzer "ELT-6" Mebanix Co. and the time required for lysis was determined.

The results obtained are shown in Fig. 20. The addition of the bispecific chimeric antibody SUSF/S-8.4 to prourokinase (●) resulted in enhanced lysis of plasma, with a substantial plateau being attained in a molar ratio of 1:1. This enhancing effect on the lytic activity of prourokinase was almost equally potent as compared with the original mouse bispecific antibody FU1-74 (o).

Example 9 Enhancement of the fibrinolytic activity of prourokinase (2)

(1) Preparation of $^{125}$I-labeled plasma clots

A commercial grade of $^{125}$I-labeled human fibrinogen (10 µg/10 µl; distributed by Muromachi Kagaku Kogyo) was added to 600 µl of human plasma, then bovine thrombin (1 U/100 µl) was added and quickly followed by stirring. The mixture was sucked into a catheter treated in advance with 10% Tween 80, allowed to stand at room temperature for 1 minute and incubated at 37° C for 30 minutes. The coagulated plasma was extruded into a dish containing physiological saline and cut to a length of 1 cm with a knife, and each section was tested for radioactivity using a gamma counter.

(2) Experiment with a hamster pulmonary embolism model

Pentobarbital (6 mg/0.3 ml) was intraperitoneally administered to hamsters (weight 80-100 g). For each animal, a blood-collecting catheter was inserted into the femoral vein. The $^{125}$I-labeled plasma clot prepared in (1) was sucked into a catheter and injected into the jugular vein, followed by insertion of a catheter for sample administration. Through the jugular vein, NaI (0.2 mg/0.1 ml) and heparin (100 units/0.1 ml) were administered, followed by further administration of 350 µl of prourokinase or an immune complex prepared by adding the bispecific chimeric antibody to prourokinase in a 2:1 molar ratio. After 90 minutes of standing at room temperature, a blood sample (1 ml) was collected. Further, following thoracotomy, the right and left lungs and the heart were excised and the were respectively measured for radioactivity using a gamma counter. The percent plasma clot lysis was calculated based on the ratio between the total radioactivity administered and the radioactivity remaining in the three organs. The results are shown in Fig. 21. In Fig. 2, (●) shows the conjugate between the bispecific chimeric antibody SUSF/S-8.4 and prourokinase and (o) shows prourokinase alone.

Sequence listing

SEQ ID NO : 1
SEQUENCE LENGTH : 16
SEQUENCE TYPE : amino acid
TOPOLOGY : linear
MOLECULE TYPE : peptide
ORIGINAL SOURCE
  ORGANISM : BALB/c mouse
  TISSUE TYPE : spleen
  CELL TYPE : B cell hybridoma
  CELL LINE : FIB1-11

Thr Ser Ser Gln Ser Leu Leu Asp Ser Asp Gly Lys Thr Tyr Leu Asn
 1 5 10 15

SEQ ID NO : 2
SEQUENCE LENGTH : 7
SEQUENCE TYPE : amino acid
TOPOLOGY : linear
MOLECULE TYPE : peptide
ORIGINAL SOURCE
  ORGANISM : BALB/c mouse
  TISSUE TYPE : spleen
  CELL TYPE : B cell hybridoma
  CELL LINE : FIB1-11

Leu Val Ser Lys Leu Tyr Ser
 1 5

SEQ ID NO : 3
SEQUENCE LENGTH : 8
SEQUENCE TYPE : amino acid
TOPOLOGY : linear
MOLECULE TYPE : peptide
ORIGINAL SOURCE

ORGANISM : BALB/c mouse
TISSUE TYPE : spleen
CELL TYPE : B cell hybridoma
CELL LINE : FIB1-11

Trp Gln Gly Ile His Phe Pro Tyr
1 5

SEQ ID NO : 4
SEQUENCE LENGTH : 5
SEQUENCE TYPE : amino acid
TOPOLOGY : linear
MOLECULE TYPE : peptide
ORIGINAL SOURCE
ORGANISM : BALB/c mouse
TISSUE TYPE : spleen
CELL TYPE : B cell hybridoma
CELL LINE : FIB1-11

Asn Tyr Asp Met Ser
1 5

SEQ ID NO : 5
SEQUENCE LENGTH : 16
SEQUENCE TYPE : amino acid
TOPOLOGY : linear
MOLECULE TYPE : peptide
ORIGINAL SOURCE
ORGANISM : BALB/c mouse
TISSUE TYPE : spleen
CELL TYPE : B cell hybridoma
CELL LINE : FIB1-11

Ser Ile Ser Val Gly Gly Thr Thr Tyr Tyr Pro Asp Ser Met Lys Gly
1 5 10 15

SEQ ID NO : 6
SEQUENCE LENGTH : 9
SEQUENCE TYPE : amino acid
TOPOLOGY : linear
MOLECULE TYPE : peptide
ORIGINAL SOURCE
  ORGANISM : BALB/c mouse
  TISSUE TYPE : spleen
  CELL TYPE : B cell hybridoma
  CELL LINE : FIB1-11

Gly Asn Phe Ala Asp Ala Met Asp Tyr
1 5

SEQ ID NO : 7
SEQUENCE LENGTH : 112
SEQUENCE TYPE : amino acid
TOPOLOGY : linear
MOLECULE TYPE : protein
ORIGINAL SOURCE
  ORGANISM : BALB/c mouse
  TISSUE TYPE : spleen
  CELL TYPE : B cell hybridoma
  CELL LINE : FIB1-11

Asp Val Val Met Ala Gln Thr Pro Leu Thr Leu Ser Val Thr Ile Gly
1 5 10 15
Gln Pro Ala Phe Ile Ser Cys Thr Ser Ser Gln Ser Leu Leu Asp Ser
20 25 30
Asp Gly Lys Thr Tyr Leu Asn Trp Leu Leu Gln Arg Pro Gly Gln Ser
35 40 45
Pro Lys Arg Leu Ile Tyr Leu Val Ser Lys Leu Tyr Ser Gly Val Pro
50 55 60
Asp Arg Phe Thr Gly Ser Gly Ser Gly Thr Ala Phe Thr Leu Lys Ile
65 70 75 80

Asn Arg Val Glu Ala Glu Asp Leu Gly Val Tyr Tyr Cys Trp Gln Gly
85 90 95
Ile His Phe Pro Tyr Thr Phe Gly Gly Gly Thr Lys Leu Glu Ile Lys
100 105 110

SEQ ID NO : 8
SEQUENCE LENGTH : 115
SEQUENCE TYPE : amino acid
TOPOLOGY : linear
MOLECULE TYPE : protein
ORIGINAL SOURCE
ORGANISM : BALB/c mouse
TISSUE TYPE : spleen
CELL TYPE : B cell hybridoma
CELL LINE : FIB1-11

Asp Val Gln Leu Trp Glu Ser Gly Gly Gly Leu Val Lys Pro Gly Gly
1 5 10 15
Ser Leu Lys Leu Ser Cys Ala Ala Ser Gly Phe Thr Phe Ser Asn Tyr
20 25 30
Asp Met Ser Trp Val Arg Gln Thr Pro Glu Arg Arg Leu Glu Trp Val
35 40 45
Ala Ser Ile Ser Val Gly Gly Thr Thr Tyr Tyr Pro Asp Ser Met Lys
50 55 60
Gly Arg Phe Thr Ile Ser Arg Asp Asn Ala Arg Asn Ile Leu Tyr Leu
65 70 75 80
Gln Leu Ser Ser Leu Arg Ser Glu Asp Thr Ala Met Tyr Tyr Cys Gly
85 90 95
Asn Phe Ala Asp Ala Met Asp Tyr Trp Gly Gln Gly Thr Leu Val Thr
100 105 110
Val Ser Ser
115

SEQ ID NO : 9
SEQUENCE LENGTH : 22

SEQUENCE TYPE : nucleic acid
STRANDEDNESS : single
TOPOLOGY : linear
MOLECULE TYPE : other nucleic acid (synthetic DNA)

AGGTGCAGCT GKKGSAGTCK GG 22

SEQ ID NO : 10
SEQUENCE LENGTH : 34
SEQUENCE TYPE : nucleic acid
STRANDEDNESS : single
TOPOLOGY : linear
MOLECULE TYPE : other nucleic acid (synthetic DNA)

TGAGGAGACG GTGACCAGGG TCCCTTGGCC CCAG 34

SEQ ID NO : 11
SEQUENCE LENGTH : 973
SEQUENCE TYPE : nucleic acid
STRANDEDNESS : double
TOPOLOGY : linear
MOLECULE TYPE : Genomic DNA
ORIGINAL SOURCE
ORGANISM : BALB/c mouse
TISSUE TYPE : spleen
CELL TYPE : B cell hybridoma
CELL LINE : FIB1-11
FEATURE:
from 1 to 152 bp intron 1 S
from 202 to 602 bp intron 2 S
from 951 to 973 bp intron 3 S
from 153 to 613 bp sig peptide S
from 614 to 949 bp mat peptide S

GCCCACATAA CTGCCCCTTC TTTGTATACT GTTATACTGT CCAGAACATT TGCATATTGT 60
TCCCTGGGAA ATCTTTGCCC TGTTGGCCTG AGATAAAACC TCAAGTGTCC TCTTGCCTCC 120

```
ACTGATCACT CTCCTATGTT CATTTCCTCA AA ATG ATG AGT CCT GCC CAG TTC CTG 176
                                    Met Met Ser Pro Ala Gln Phe Leu
                                    -20                 -15
TTT CTG TTA GTG CTC TGG ATT CGG GGTAAGGAGT TCTGGAATGG GAGGGATGAG    230
Phe Leu Leu Val Leu Trp Ile Arg
        -10                 -5
AATGGGGATG GAGGGTGATC TCTGGATGCC TATGTGTGCT GTTTATTTGT GGTGGGGCAG   290
GTCATATCTT CTAGGATGTG AGGTTTTGTT ACATCCTAAT GAGATATTCC AGATGGAACA   350
GTAGGTGTAC TGAGATCAAT ATTCTGACAT AGATTGGATG GAGTGGTGTA GACTCTGATG   410
ATTAGAGCCT TCAACATTTG TTTCATGACA AAATATTTGA TATATAATAT TTTTAAATCT   470
GAAAAACTGG TAGGATCTTA CTTGAAGGAA TACCATTTTC GAGTAAGATT TCAAGAAGAT   530
TTTCAAGTAG ATTTCACAAA GGTTACTCAG GACCTTTGCA CATGATTTTC CACTATTCTA   590
TTGTCATTTC AG AA ACC AAC GGT GAT GTT GTG ATG GCC CAG ACT CCA CTC    640
             Glu Thr Asn Gly Asp Val Val Met Ala Gln Thr Pro Leu
                             1               5
ACT TTG TCG GTT ACC ATT GGA CAA CCA GCC TTC ATC TCT TGC ACG TCA     688
Thr Leu Ser Val Thr Ile Gly Gln Pro Ala Phe Ile Ser Cys Thr Ser
 10                  15                  20                  25
AGT CAG AGC CTC TTA GAT AGT GAT GGA AAG ACA TAT TTG AAT TGG TTG     736
Ser Gln Ser Leu Leu Asp Ser Asp Gly Lys Thr Tyr Leu Asn Trp Leu
                30                  35                  40
TTA CAG AGG CCA GGC CAG TCT CCA AAG CGC CTA ATC TAT CTG GTG TCT     784
Leu Gln Arg Pro Gly Gln Ser Pro Lys Arg Leu Ile Tyr Leu Val Ser
            45                  50                  55
AAA CTG TAC TCT GGA GTC CCT GAC AGG TTC ACT GGC AGT GGA TCA GGG     832
Lys Leu Tyr Ser Gly Val Pro Asp Arg Phe Thr Gly Ser Gly Ser Gly
        60                  65                  70
ACA GCT TTC ACA CTG AAA ATC AAC AGA GTG GAG GCT GAG GAT TTG GGA     880
Thr Ala Phe Thr Leu Lys Ile Asn Arg Val Glu Ala Glu Asp Leu Gly
    75                  80                  85
GTT TAT TAT TGC TGG CAA GGT ATA CAT TTT CCG TAC ACG TTC GGA GGG     928
Val Tyr Tyr Cys Trp Gln Gly Ile His Phe Pro Tyr Thr Phe Gly Gly
 90                  95                  100                 105
GGG ACC AAG CTG GAA ATA AAA CGTAAGTAGT CTTCTCAACT CTTG              973
Gly Thr Lys Leu Glu Ile Lys
                110
```

SEQ ID NO : 12
SEQUENCE LENGTH : 684
SEQUENCE TYPE : nucleic acid
STRANDEDNESS : double
TOPOLOGY : linear
MOLECULE TYPE : Other nucleic acid
(cDNA to mRNA + Genomic DNA)
ORIGINAL SOURCE
ORGANISM : BALB/c mouse
TISSUE TYPE : spleen
CELL TYPE : B cell hybridoma
CELL LINE : FIB1-11
FEATURE:
from 1 to 150 bp intron 1 S
from 197 to 313 bp intron 2 S
from 670 to 684 bp intron 3 S
from 151 to 324 bp sig peptide S
from 325 to 669 bp mat peptide S

```
GCATGCTATA GAGGAAGATA TGCAAATAAT TCTTCTCTGA GTTCATATAA ACCAGCCCTG    60
CCCCGAGTCT GTAGCTCTGA CAGAGGAGCC AAGCCCTGGA TTCCCAGGTC CTCACATTCA   120
GTGATCAGCA CTGAACACAG ACCACTCACC ATG GAC TCC AGG CTC AAT TTA GTT    174
                                 Met Asp Ser Arg Leu Asn Leu Val
                                                 -15
TTC CTT GTC CTT ATT TTA AAA GGTAATTTGT AGAGATGAGT TTCTGCCTGT        225
Phe Leu Val Leu Ile Leu Lys
    -10                 -5
TGTGTGCCCA AGGGAAATAG AAACATTGTT TGTTTCATTA TTTTATTTTG TTAGTAACAG   285
TTTTCTGACC AGCATTCTCT GTTTGCAG GT GTC CAG TGT GAT GTG CAG CTG TGG   339
                               Gly Val Gln Cys Asp Val Gln Leu Trp
                                               1               5
GAG TCG GGG GGA GGC TTA GTG AAG CCT GGA GGG TCC CTG AAA CTC TCC     387
Glu Ser Gly Gly Gly Leu Val Lys Pro Gly Gly Ser Leu Lys Leu Ser
                10                  15                  20
TGT GCA GCC TCT GGA TTC ACT TTC AGT AAC TAT GAC ATG TCT TGG GTT     435
```

```
Cys Ala Ala Ser Gly Phe Thr Phe Ser Asn Tyr Asp Met Ser Trp Val
                25                  30                  35
CGC CAG ACT CCA GAG AGG AGG CTG GAG TGG GTC GCA TCC ATT AGT GTT      483
Arg Gln Thr Pro Glu Arg Arg Leu Glu Trp Val Ala Ser Ile Ser Val
            40                  45                  50
GGT GGT ACC ACC TAC TAT CCA GAC AGT ATG AAG GGC CGA TTC ACC ATC      531
Gly Gly Thr Thr Tyr Tyr Pro Asp Ser Met Lys Gly Arg Phe Thr Ile
        55                  60                  65
TCC AGA GAT AAT GCC AGG AAC ATC CTG TAT CTG CAA TTG AGC AGT CTG      579
Ser Arg Asp Asn Ala Arg Asn Ile Leu Tyr Leu Gln Leu Ser Ser Leu
 70                  75                  80                  85
AGG TCT GAA GAC ACG GCC ATG TAT TAC TGT GGT AAC TTC GCG GAT GCT      627
Arg Ser Glu Asp Thr Ala Met Tyr Tyr Cys Gly Asn Phe Ala Asp Ala
                     90                  95                 100
ATG GAC TAC TGG GGC CAA GGG ACC CTG GTC ACC GTC TCC TCA GGTAAGCTGG  679
Met Asp Tyr Trp Gly Gln Gly Thr Leu Val Thr Val Ser Ser
            105                 110                 115
CTTTT                                                                684
```

SEQ ID NO : 13
SEQUENCE LENGTH : 10
SEQUENCE TYPE : amino acid
TOPOLOGY : linear
MOLECULE TYPE : peptide
ORIGINAL SOURCE
  ORGANISM : BALB/c mouse
  TISSUE TYPE : spleen
  CELL TYPE : B cell hybridoma
  CELL LINE : UK1-3

```
Ser Ala Ser Ser Ser Val Gly Tyr Met Tyr
 1               5                   10
```

SEQ ID NO : 14
SEQUENCE LENGTH : 7
SEQUENCE TYPE : amino acid

TOPOLOGY : linear
MOLECULE TYPE : peptide
ORIGINAL SOURCE
  ORGANISM : BALB/c mouse
  TISSUE TYPE : spleen
  CELL TYPE : B cell hybridoma
  CELL LINE : UK1-3

Leu Thr Ser Asn Leu Ala Ser
 1               5

SEQ ID NO : 15
SEQUENCE LENGTH : 9
SEQUENCE TYPE : amino acid
TOPOLOGY : linear
MOLECULE TYPE : peptide
ORIGINAL SOURCE
  ORGANISM : BALB/c mouse
  TISSUE TYPE : spleen
  CELL TYPE : B cell hybridoma
  CELL LINE : UK1-3

Gln Gln Trp Ser Ser Asp Pro Pro Thr
 1               5

SEQ ID NO : 16
SEQUENCE LENGTH : 6
SEQUENCE TYPE : amino acid
TOPOLOGY : linear
MOLECULE TYPE : peptide
ORIGINAL SOURCE
  ORGANISM : BALB/c mouse
  TISSUE TYPE : spleen
  CELL TYPE : B cell hybridoma
  CELL LINE : UK1-3

Ser Asp Tyr Ala Trp Asn
1 5

SEQ ID NO : 17
SEQUENCE LENGTH : 16
SEQUENCE TYPE : amino acid
TOPOLOGY : linear
MOLECULE TYPE : peptide
ORIGINAL SOURCE
ORGANISM : BALB/c mouse
TISSUE TYPE : spleen
CELL TYPE : B cell hybridoma
CELL LINE : UK1-3

Tyr Ile Asn Tyr Ser Gly Thr Thr Ser Tyr Asn Pro Ser Leu Lys Ser
1 5 10 15

SEQ ID NO : 18
SEQUENCE LENGTH : 12
SEQUENCE TYPE : amino acid
TOPOLOGY : linear
MOLECULE TYPE : peptide
ORIGINAL SOURCE
ORGANISM : BALB/c mouse
TISSUE TYPE : spleen
CELL TYPE : B cell hybridoma
CELL LINE : UK1-3

Leu Gly Asp Phe Asp Ala Gly Asp Tyr Phe Asp Tyr
1 5 10

SEQ ID NO : 19
SEQUENCE LENGTH : 107
SEQUENCE TYPE : amino acid
TOPOLOGY : linear
MOLECULE TYPE : protein

ORIGINAL SOURCE
ORGANISM : BALB/c mouse
TISSUE TYPE : spleen
CELL TYPE : B cell hybridoma
CELL LINE : UK1-3

Asp Ile Gln Leu Thr Gln Ser Pro Ala Leu Met Ser Ala Val Pro Gly
1 5 10 15
Glu Lys Val Thr Met Thr Cys Ser Ala Ser Ser Ser Val Gly Tyr Met
20 25 30
Tyr Trp Tyr Gln Gln Lys Pro Arg Ser Ser Pro Lys Pro Trp Ile Ser
35 40 45
Leu Thr Ser Asn Leu Ala Ser Gly Val Pro Ala Arg Phe Ser Gly Ser
50 55 60
Gly Ser Gly Thr Ser Tyr Ser Leu Thr Ile Ser Ser Met Glu Ala Glu
65 70 75 80
Asp Ala Ala Thr Tyr Tyr Cys Gln Gln Trp Ser Ser Asp Pro Pro Thr
85 90 95
Phe Gly Gly Gly Thr Lys Leu Glu Ile Lys Arg
100 105

SEQ ID NO : 20
SEQUENCE LENGTH : 123
SEQUENCE TYPE : amino acid
TOPOLOGY : linear
MOLECULE TYPE : protein
ORIGINAL SOURCE
ORGANISM : BALB/c mouse
TISSUE TYPE : spleen
CELL TYPE : B cell hybridoma
CELL LINE : UK1-3

Glu Val Gln Leu Val Glu Ser Gly Pro Gly Leu Val Lys Pro Ser Gln
1 5 10 15
Ser Leu Ser Leu Thr Cys Thr Val Thr Gly Tyr Ser Ile Thr Ser Asp
20 25 30

Tyr Ala Trp Asn Trp Ile Arg Gln Phe Pro Gly Asn Lys Leu Glu Trp
35 40 45

Met Gly Tyr Ile Asn Tyr Ser Gly Thr Thr Ser Tyr Asn Pro Ser Leu
50 55 60

Lys Ser Arg Ile Ser Ile Thr Arg Asp Thr Ser Asn Asn Gln Phe Phe
65 70 75 80

Leu Gln Leu Asn Ser Val Thr Ser Glu Asp Thr Ala Thr Tyr Tyr Cys
85 90 95

Ala Arg Leu Gly Asp Phe Asp Ala Gly Asp Tyr Phe Asp Tyr Trp Gly
100 105 110

Gln Gly Thr Thr Val Thr Val Ser Ser Ala Ser
115 120

SEQ ID NO : 21

SEQUENCE LENGTH : 378

SEQUENCE TYPE : nucleic acid

STRANDEDNESS : double

TOPOLOGY : linear

MOLECULE TYPE : cDNA

ORIGINAL SOURCE

ORGANISM : BALB/c mouse

TISSUE TYPE : spleen

CELL TYPE : B cell hybridoma

CELL LINE : UK1-3

FEATURE:

1 to 9 S sig peptide

10 to 378 S mat peptide

GTC GAC TGT GAG GTG CAA CTA GTG GAG TCG GGA CCT GGC CTG GTG AAA 48
Val Asp Cys Glu Val Gln Leu Val Glu Ser Gly Pro Gly Leu Val Lys
1 5 10 15

CCT TCT CAG TCT CTG TCC CTC ACC TGC ACT GTC ACT GGC TAC TCA ATC 96
Pro Ser Gln Ser Leu Ser Leu Thr Cys Thr Val Thr Gly Tyr Ser Ile
20 25 30

ACC AGT GAT TAT GCC TGG AAC TGG ATC CGG CAG TTT CCA GGA AAC AAA 144

```
Thr Ser Asp Tyr Ala Trp Asn Trp Ile Arg Gln Phe Pro Gly Asn Lys
         35                  40                  45
CTG GAG TGG ATG GGC TAC ATA AAC TAC AGT GGT ACC ACT AGT TAC AAC  192
Leu Glu Trp Met Gly Tyr Ile Asn Tyr Ser Gly Thr Thr Ser Tyr Asn
     50                  55                  60
CCA TCT CTC AAA AGT CGA ATC TCT ATC ACT CGA GAC ACA TCC AAT AAC  240
Pro Ser Leu Lys Ser Arg Ile Ser Ile Thr Arg Asp Thr Ser Asn Asn
 65                  70                  75                  80
CAG TTC TTC CTG CAG TTG AAT TCT GTG ACT TCT GAG GAC ACT GCC ACA  288
Gln Phe Phe Leu Gln Leu Asn Ser Val Thr Ser Glu Asp Thr Ala Thr
                 85                  90                  95
TAT TAC TGT GCA AGA TTG GGT GAT TTC GAC GCG GGT GAC TAC TTT GAC  336
Tyr Tyr Cys Ala Arg Leu Gly Asp Phe Asp Ala Gly Asp Tyr Phe Asp
            100                 105                 110
TAC TGG GGC CAA GGG ACC ACG GTC ACC GTC TCG AGT GCT AGC          378
Tyr Trp Gly Gln Gly Thr Thr Val Thr Val Ser Ser Ala Ser
        115                 120                 125
```

SEQ ID NO : 22

SEQUENCE LENGTH : 321

SEQUENCE TYPE : nucleic acid

STRANDEDNESS : double

TOPOLOGY : linear

MOLECULE TYPE : cDNA

ORIGINAL SOURCE

ORGANISM : BALB/c mouse

TISSUE TYPE : spleen

CELL TYPE : B cell hybridoma

CELL LINE : UK1-3

FEATURE:

1 to 321 S mat peptide

```
GAT ATT CAG CTG ACA CAG TCT CCA GCA CTC ATG TCT GCA GTT CCA GGG   48
Asp Ile Gln Leu Thr Gln Ser Pro Ala Leu Met Ser Ala Val Pro Gly
 1               5                  10                  15
GAG AAG GTC ACC ATG ACC TGC AGT GCC AGC TCA AGT GTA GGT TAC ATG   96
```

```
Glu Lys Val Thr Met Thr Cys Ser Ala Ser Ser Ser Val Gly Tyr Met
                20                  25                  30
TAT TGG TAT CAG CAG AAG CCA AGA TCC TCC CCC AAG CCC TGG ATT TCT  144
Tyr Trp Tyr Gln Gln Lys Pro Arg Ser Ser Pro Lys Pro Trp Ile Ser
            35                  40                  45
CTC ACA TCC AAC CTG GCT TCT GGA GTC CCT GCT CGC TTC AGT GGC AGT  192
Leu Thr Ser Asn Leu Ala Ser Gly Val Pro Ala Arg Phe Ser Gly Ser
        50                  55                  60
GGG TCT GGG ACC TCT TAC TCT CTC ACC ATC AGC AGC ATG GAG GCT GAA  240
Gly Ser Gly Thr Ser Tyr Ser Leu Thr Ile Ser Ser Met Glu Ala Glu
 65                  70                  75                  80
GAT GCT GCC ACT TAT TAC TGC CAG CAG TGG AGT AGT GAC CCA CCC ACG  288
Asp Ala Ala Thr Tyr Tyr Cys Gln Gln Trp Ser Ser Asp Pro Pro Thr
                 85                  90                  95
TTC GGA GGG GGG ACC AAG CTG GAG ATC AAA CGT                      321
Phe Gly Gly Gly Thr Lys Leu Glu Ile Lys Arg
            100                 105
```

SEQ ID NO : 23

SEQUENCE LENGTH : 39

SEQUENCE TYPE : nucleic acid

STRANDEDNESS : single

TOPOLOGY : linear

MOLECULE TYPE : other nucleic acid (synthetic DNA)

```
AGAATTCTAA CACTCTCCGC GGTTGAAGCT CTTTGTGAC                       39
```

SEQ ID NO : 24

SEQUENCE LENGTH : 26

SEQUENCE TYPE : nucleic acid

STRANDEDNESS : single

TOPOLOGY : linear

MOLECULE TYPE : other nucleic acid (synthetic DNA)

```
ACGTACGGTG GCTGCACCAT CTGTCT                                     26
```

SEQ ID NO : 25
SEQUENCE LENGTH : 37
SEQUENCE TYPE : nucleic acid
STRANDEDNESS : single
TOPOLOGY : linear
MOLECULE TYPE : other nucleic acid (synthetic DNA)

CCGTACGTTT GATCACCAGC TTGGTCCCCC CTCCGAA 37

SEQ ID NO : 26
SEQUENCE LENGTH : 40
SEQUENCE TYPE : nucleic acid
STRANDEDNESS : single
TOPOLOGY : linear
MOLECULE TYPE : other nucleic acid (synthetic DNA)

AACGCGTGGT GATATTCAGC TGGCCCAGAC TCCACTCACT 40

SEQ ID NO : 27
SEQUENCE LENGTH : 28
SEQUENCE TYPE : nucleic acid
STRANDEDNESS : single
TOPOLOGY : linear
MOLECULE TYPE : other nucleic acid (synthetic DNA)

CACGCGTTTC CCGAATCCAG AGCACTAA 28

SEQ ID NO : 28
SEQUENCE LENGTH : 35
SEQUENCE TYPE : nucleic acid
STRANDEDNESS : single
TOPOLOGY : linear
MOLECULE TYPE : other nucleic acid (synthetic DNA)

AGAATTCCGC CATGATGAGT CCTGCCCAGT TCCTG 35

SEQ ID NO : 29
SEQUENCE LENGTH : 38
SEQUENCE TYPE : nucleic acid
STRANDEDNESS : single
TOPOLOGY : linear
MOLECULE TYPE : other nucleic acid (synthetic DNA)

ACACGTGTCC ACCGTGCCCG GCGCCTGAAC TCCTGGGG 38

SEQ ID NO : 30
SEQUENCE LENGTH : 32
SEQUENCE TYPE : nucleic acid
STRANDEDNESS : single
TOPOLOGY : linear
MOLECULE TYPE : other nucleic acid (synthetic DNA)

CGAATTCATT TACCCGGGGA CAGGGAGAGG CT 32

SEQ ID NO : 31
SEQUENCE LENGTH : 35
SEQUENCE TYPE : nucleic acid
STRANDEDNESS : single
TOPOLOGY : linear
MOLECULE TYPE : other nucleic acid (synthetic DNA)

GCACGTGTGA GTTTTGTCAC AAGAT 25

SEQ ID NO : 32
SEQUENCE LENGTH : 35
SEQUENCE TYPE : nucleic acid
STRANDEDNESS : single
TOPOLOGY : linear
MOLECULE TYPE : other nucleic acid (synthetic DNA)

TCTCGAGTGC TAGCACCAAG GGCCCATCGG TCTTC 35

SEQ ID NO : 33
SEQUENCE LENGTH : 26
SEQUENCE TYPE : nucleic acid
STRANDEDNESS : single
TOPOLOGY : linear
MOLECULE TYPE : other nucleic acid (synthetic DNA)

ACTCGAGACG GTGACCAGGG TCCCTT 26

SEQ ID NO : 34
SEQUENCE LENGTH : 30
SEQUENCE TYPE : nucleic acid
STRANDEDNESS : single
TOPOLOGY : linear
MOLECULE TYPE : other nucleic acid (synthetic DNA)

CAACTAGTGG AGTCGGGGGG AGGCTTAGTG 30

SEQ ID NO : 35
SEQUENCE LENGTH : 39
SEQUENCE TYPE : nucleic acid
STRANDEDNESS : single
TOPOLOGY : linear
MOLECULE TYPE : other nucleic acid (synthetic DNA)

CACTAGTTGC ACCTCACAGT CGACACCTTT TAAAATAAG 39

SEQ ID NO : 36
SEQUENCE LENGTH : 29
SEQUENCE TYPE : nucleic acid
STRANDEDNESS : single
TOPOLOGY : linear
MOLECULE TYPE : other nucleic acid (synthetic DNA)

TGAATTCCAC CATGGACTCC AGGCTCAAT 29

SEQ ID NO : 37
SEQUENCE LENGTH : 22
SEQUENCE TYPE : nucleic acid
STRANDEDNESS : single
TOPOLOGY : linear
MOLECULE TYPE : other nucleic acid (synthetic DNA)

ATGTGCAACT AGTGGAGTCS GG 22

SEQ ID NO : 38
SEQUENCE LENGTH : 37
SEQUENCE TYPE : nucleic acid
STRANDEDNESS : single
TOPOLOGY : linear
MOLECULE TYPE : other nucleic acid (synthetic DNA)

ATTAACTCGA GACGGTGACC GTGGTCCCTT GGCCCCA 37

SEQ ID NO : 39
SEQUENCE LENGTH : 24
SEQUENCE TYPE : nucleic acid
STRANDEDNESS : single
TOPOLOGY : linear
MOLECULE TYPE : other nucleic acid (synthetic DNA)

GACATTCAGC TGACMCAGWC TCCA 24

SEQ ID NO : 40
SEQUENCE LENGTH : 22
SEQUENCE TYPE : nucleic acid
STRANDEDNESS : single
TOPOLOGY : linear
MOLECULE TYPE : other nucleic acid (synthetic DNA)

GTTAGATCTC CAGCTTGGTC CC 22

SEQ ID NO : 41
SEQUENCE LENGTH : 29
SEQUENCE TYPE : nucleic acid
STRANDEDNESS : single
TOPOLOGY : linear
MOLECULE TYPE : other nucleic acid (synthetic DNA)

TCGTCGACTG TGAGGTGCAA CTAGTGGAG 29

SEQ ID NO : 42
SEQUENCE LENGTH : 25
SEQUENCE TYPE : nucleic acid
STRANDEDNESS : single
TOPOLOGY : linear
MOLECULE TYPE : other nucleic acid (synthetic DNA)

TCGCTAGCAC TCGAGACGGT GACCG 25

SEQ ID NO : 43
SEQUENCE LENGTH : 19
SEQUENCE TYPE : nucleic acid
STRANDEDNESS : single
TOPOLOGY : linear
MOLECULE TYPE : other nucleic acid (synthetic DNA)

CAGGGGCCAG TGGATAGAC 19

SEQ ID NO : 44
SEQUENCE LENGTH : 22
SEQUENCE TYPE : nucleic acid
STRANDEDNESS : single
TOPOLOGY : linear
MOLECULE TYPE : other nucleic acid (synthetic DNA)

CATTTTGTCG TTCACTGCCA TC 22

## Claims

1. A chimeric monoclonal antibody comprising an anti-human fibrin antibody light chain variable region comprising at least one of the polypeptide chains A, B and C represented by the formulas

A: -Thr Ser Ser Gln Ser Leu Leu Asp Ser Asp Gly Lys Thr Tyr Leu Asn-,
B: -Leu Val Ser Lys Leu Tyr Ser- and
C: -Trp Gln Gly Ile His Phe Pro Tyr-,

respectively, and a human antibody light chain constant region.

2. A chimeric monoclonal antibody comprising an anti-human fibrin antibody heavy chain variable region comprising at least one of the polypeptide chains D, E and F represented by the formulas

D: -Asn Tyr Asp Met Ser-,
E: -Ser Ile Ser Val Gly Gly Thr Thr Tyr Tyr Pro Asp Ser Met Lys Gly and
F: -Gly Asn Phe Ala Asp Ala Met Asp Tyr-,

respectively, and a human antibody heavy chain constant region.

3. The chimeric monoclonal antibody as claimed in Claim 1, wherein said antibody further comprises the anti-human fibrin antibody heavy chain variable region claimed in Claim 2.

4. The chimeric monoclonal antibody as claimed in Claim 1, 2 or 3, wherein said antibody is an anti-human fibrin mouse-human chimeric antibody.

5. A DNA which contains a DNA coding for the anti-human fibrin antibody light chain variable region as claimed in Claim 1.

6. The DNA as claimed in Claim 5, which further comprising a DNA coding for the human antibody light chain constant region.

7. A DNA which contains a DNA coding for the anti-human fibrin antibody heavy chain variable region as claimed in Claim 2.

8. The DNA as claimed in Claim 7, which further comprising a DNA coding for the human antibody heavy chain constant region.

9. The DNA as claimed in Claim 5, wherein said DNA further comprises the DNA as claimed in Claim 7.

10. The DNA as claimed in claim 5, wherein said DNA further comprises the DNA as claimed in claim 8.

11. The DNA as claimed in claim 6, wherein said DNA further comprises the DNA as claimed in claim 7.

12. The DNA as claimed in claim 6, wherein said DNA further comprises the DNA as claimed in claim 8.

13. The DNA as claimed in Claim 5, 6, 7 or 8, wherein the anti-human fibrin antibody variable region-encoding DNA is derived from a mouse hybridoma.

**14.** The DNA as claimed in Claim 13, wherein said mouse hybridoma is the mouse hybridoma FIB1-11.

**15.** A eukaryotic cell capable of expressing an anti-human fibrin chimeric monoclonal antibody, which carries the DNA as claimed in Claim 5 or 6.

**16.** A eukaryotic cell capable of expressing an anti-human fibrin chimeric monoclonal antibody, which carries the DNA claimed in Claim 7 or 8.

**17.** The eukaryotic cell as claimed in Claim 15, wherein said cell carries the DNA claimed in Claim 11.

**18.** The eukaryotic cell as claimed in Claim 17, wherein said cell is a mouse myeloma cell line FIB1-HO1/X63, which produces an anti-human fibrin mouse-human chimeric antibody.

**19.** The eukaryotic cell as claimed in claim 17, wherein said cell is a mouse hybridoma cell line SS/S-3, which produces an anti-human fibrin mouse-human chimeric antibody.

**20.** A chimeric monoclonal antibody which comprising an anti-urokinase antibody light chain variable region comprising at least one of the polypeptide chains G, H and I represented by the formulas

G : -Ser Ala Ser Ser Ser Val Gly Tyr Met Tyr-,

H : Leu Thr Ser Asn Leu Ala Ser- and
I : -Gln Gln Trp Ser Ser Asp Pro Pro Thr-,

respectively, and a human antibody light chain constant region.

**21.** A chimeric monoclonal antibody comprising an anti-urokinase antibody heavy chain variable region comprising at least one of the polypeptide chains J, K and L represented by the formulas

J : -Ser Asp Tyr Ala Trp Asn-,
K : -Tyr Ile Asn Tyr Ser Gly Thr Thr Ser Tyr Asn Pro Ser Leu Lys Ser- and
L : -Leu Gly Asp Phe Asp Ala Gly Asp Tyr Phe Asp Tyr-,

respectively and a human antibody heavy chain constant region.

**22.** The chimeric monoclonal antibody as claimed in Claim 20, wherein said antibody further comprising the anti-urokinase antibody heavy chain variable region as claimed in Claim 21.

**23.** The chimeric monoclonal antibody as claimed in Claim 20, 21 or 22, wherein said antibody is an anti-urokinase mouse-human chimeric antibody.

**24.** A DNA which comprises a DNA coding for the anti-urokinase antibody light chain variable region as claimed in Claim 20.

**25.** The DNA as claimed in Claim 24 which further comprises a DNA coding for the human antibody light chain constant region.

**26.** A DNA which comprises a DNA coding for the anti-urokinase antibody heavy chain variable region as claimed in Claim 21.

**27.** The DNA as claimed in Claim 26 which further comprises a DNA coding for the human antibody heavy chain constant region.

**28.** The DNA as claimed in Claim 24, wherein said DNA further comprises the DNA as claimed in Claim 26.

**29.** The DNA as claimed in Claim 24, wherein said DNA further comprising the DNA as claimed in Claim 27.

**30.** The DNA as claimed in Claim 25, wherein said DNA further comprises the DNA as claimed in Claim 26.

**31.** The DNA as claimed in Claim 25, wherein said DNA further comprises the DNA as claimed in Claim 27.

**32.** The DNA as claimed in Claim 24, 25, 26 or 27, wherein the anti-urokinase antibody variable region-encoding DNA is a DNA derived from a mouse hybridoma.

**33.** The DNA as claimed in Claim 32, wherein said mouse hybridoma is the mouse hybridoma UK1-3.

**34.** A eukaryotic cell capable of expressing an anti-urokinase chimeric monoclonal antibody, which carries the DNA as claimed in Claim 24 or 25.

**35.** A eukaryotic cell capable of expressing an anti-urokinase chimeric monoclonal antibody, which carries the DNA in claimed in Claim 26 or 27.

**36.** The eukaryotic cell as claimed in Claim 34, wherein said cell carries the DNA claimed in Claim 30, 31, 32 or 33.

**37.** The eukaryotic cell as claimed in Claim 36, wherein said cell is a mouse hybridoma cell line SU/S-9.21 which produces an anti-urokinase specific mouse-human chimeric antibody.

**38.** A bispecific chimeric monoclonal antibody, which comprises an anti-human fibrin antibody variable region, an anti-urokinase antibody variable region and a human antibody constant region.

**39.** The bispecific chimeric monoclonal antibody as claimed in Claim 38, wherein a light chain portion of said anti-human fibrin antibody variable region comprises at least one of peptide chains A, B and C of Claim 1.

**40.** The bispecific chimeric monoclonal antibody as claimed in Claim 39, wherein a heavy chain portion of said anti-human fibrin antibody variable region comprises at least one of peptide chains D, E and F of Claim 2.

**41.** The bispecific chimeric monoclonal antibodies as claimed in Claim 38, wherein a light chain portion of said anti-urokinase antibody variable region comprises at least one of peptide chains G, H and I of Claim 20.

**42.** The bispecific chimeric monoclonal antibody as claimed in Claim 41, wherein said a heavy chain portion of said anti-urokinase antibody variable region comprises at least one of peptide chains J, K and L of Claim 21.

**43.** A eukaryotic cell capable of expressing a bispecific chimeric monoclonal antibody, which carries both of the DNAs claimed in Claim 11 and 30.

**44.** The eukaryotic cell as claimed in Claim 43, wherein said eukaryotic cell is mouse hybridoma cell line SUSF/S-8.4 which produces an anti-human fibrin-anti-urokinase bispecific mouse-human chimeric antibody.

**45.** A thrombolytic protein immunocomplex comprising (1) an anti-human fibrin chimeric antibody fragment and (2) an anti-urokinase chimeric antibody fragment, wherein a urokinase is immunologically coupled to said fragment (2).

**46.** The immunocomplex as claimed in Claim 45, which comprises a protein complex comprising an anti-human fibrin chimeric antibody and an anti-urokinase chimeric antibody, wherein said antiboies are bound covalently.

**47.** The immunocomplex as claimed in Claim 46, wherein said protein complex comprises a chimeric antibody of Claim 1 and a chimeric antibody of Claim 20, wherein said antibodies are bound covalently.

**48.** The immunocomplex as claimed in Claim 46, wherein said protein complex comprises a chimeric antibody of Claim 1 and a chimeric antibody of Claim 21, wherein said antibodies are bound covalently.

**49.** The immunocomplex as claimed in Claim 46, wherein said protein complex comprises a chimeric antibody of Claim 2 and a chimeric antibody of Claim 20, wherein said antibodies are bound covalently.

**50.** The immunocomplex as claimed in Claim 46, wherein said protein complex comprises a chimeric antibody of Claim 2 and a chimeric antibody of Claim 21, wherein said antibodies are bound covalently.

**51.** The immunocomplex as claimed in Claim 46, wherein said protein complex comprises a chimeric antibody of Claim 3 and a chimeric antibody of Claim 22, wherein said antibodies are bound covalently.

**52.** The immunocomplex as claimed in Claim 45, which comprises a bispecific chimeric monoclonal antibody comprising an anti-human fibrin antibody variable region, an anti-urokinase antibody variable region and a human antibody constant region, wherein a urokinase is immunologically coupled to said bispecific antibody.

Fig. 1-(1)

```
GCCCACATAACTGCCCCTTCTTTGTATACTGTTATACTGTCCAGAACATTTGCATATTGTTCCCTGGGAAATCTTTGCCC

                                                                      -20
                                                                      Met Met
TGTTGGCCTGAGATAAAACCTCAAGTGTCCTCTTGCCTCCACTGATCACTCTCCTATGTTCATTTCCTCAAA ATG ATG
---------------------------------------- Leader --------------//

Ser Pro Ala Gln Phe Leu Phe Leu Leu Val Leu Trp Ile Arg G
AGT CCT GCC CAG TTC CTG TTT CTG TTA GTG CTC TGG ATT CGG G/GT AAGGAGTTCTGGAATGGGA

GGGATGAGAATGGGGATGGAGGGTGATCTCTGGATGCCTATGTGTGCTGTTTATTTGTGGTGGGGCAGGTCATATCTTC

TAGGATGTGAGGTTTTGTTACATCCTAATGAGATATTCCAGATGGAACAGTAGGTGTACTGAGATCAATATTCTGACAT

AGATTGGATGGAGTGGTGTAGACTCTGATGATTAGAGCCTTCAACATTTGTTTCATGACAAAATATTTGATATATAATA

TTTTTAAATCTGAAAAACTGGTAGGATCTTACTTGAAGGAATACCATTTTCGAGTAAGATTTCAAGAAGATTTTCAAGT
```

(Cont.)

Fig. 1-(2)

```
                                                                  lu Thr Asn Gly    -1
AGATTTCACAAAGGTTACTCAGGACCTTTGCACATGATTTTCCACTATTCTATTGTCATTTCA GAA ACC AAC GGT    -1
                            FR-1
1
Asp Val Val Met Ala Gln Thr Pro Leu Thr Leu Ser Val Thr Ile Gly Gln Pro Ala Phe    20
GAT GTT GTG ATG GCC CAG ACT CCA CTC ACT TTG TCG GTT ACC ATT GGA CAA CCA GCC TTC    60
                            CDR-1
Ile Ser Cys Thr Ser Ser Gln Ser Leu Leu Asp Ser Asp Gly Lys Thr Tyr Leu Asn Trp    40
ATC TCT TGC ACG TCA AGT CAG AGC CTC TTA GAT AGT GAT GGA AAG ACA TAT TTG AAT TGG   120
       FR-2                                                     CDR-2
Leu Leu Gln Arg Pro Gly Gln Ser Pro Lys Arg Leu Ile Tyr Leu Val Ser Lys Leu Tyr    60
TTG TTA CAG AGG CCA GGC CAG TCT CCA AAG CGC CTA ATC TAT CTG GTG TCT AAA CTG TAC   180
                            FR-3
Ser Gly Val Pro Asp Arg Phe Thr Gly Ser Gly Ser Gly Thr Ala Phe Thr Leu Lys Ile    80
TCT GGA GTC CCT GAC AGG TTC ACT GGC AGT GGA TCA GGG ACA GCT TTC ACA CTG AAA ATC   240
                                                                CDR-3
Asn Arg Val Glu Ala Glu Asp Leu Gly Val Tyr Tyr Cys Trp Gln Gly Ile His Phe Pro   100
AAC AGA GTG GAG GCT GAG GAT TTG GGA GTT TAT TAT TGC TGG CAA GGT ATA CAT TTT CCG   300
                     FR-4                                         VkII
Tyr Thr Phe Gly Gly Gly Thr Lys Leu Glu Ile Lys                                   112
TAC ACG TTC GGA GGG GGG ACC AAG CTG GAA ATA AAA CGT AAGTAGTCTTCTCAACTCTTG         360
Jk2
```

Fig. 2-(1)

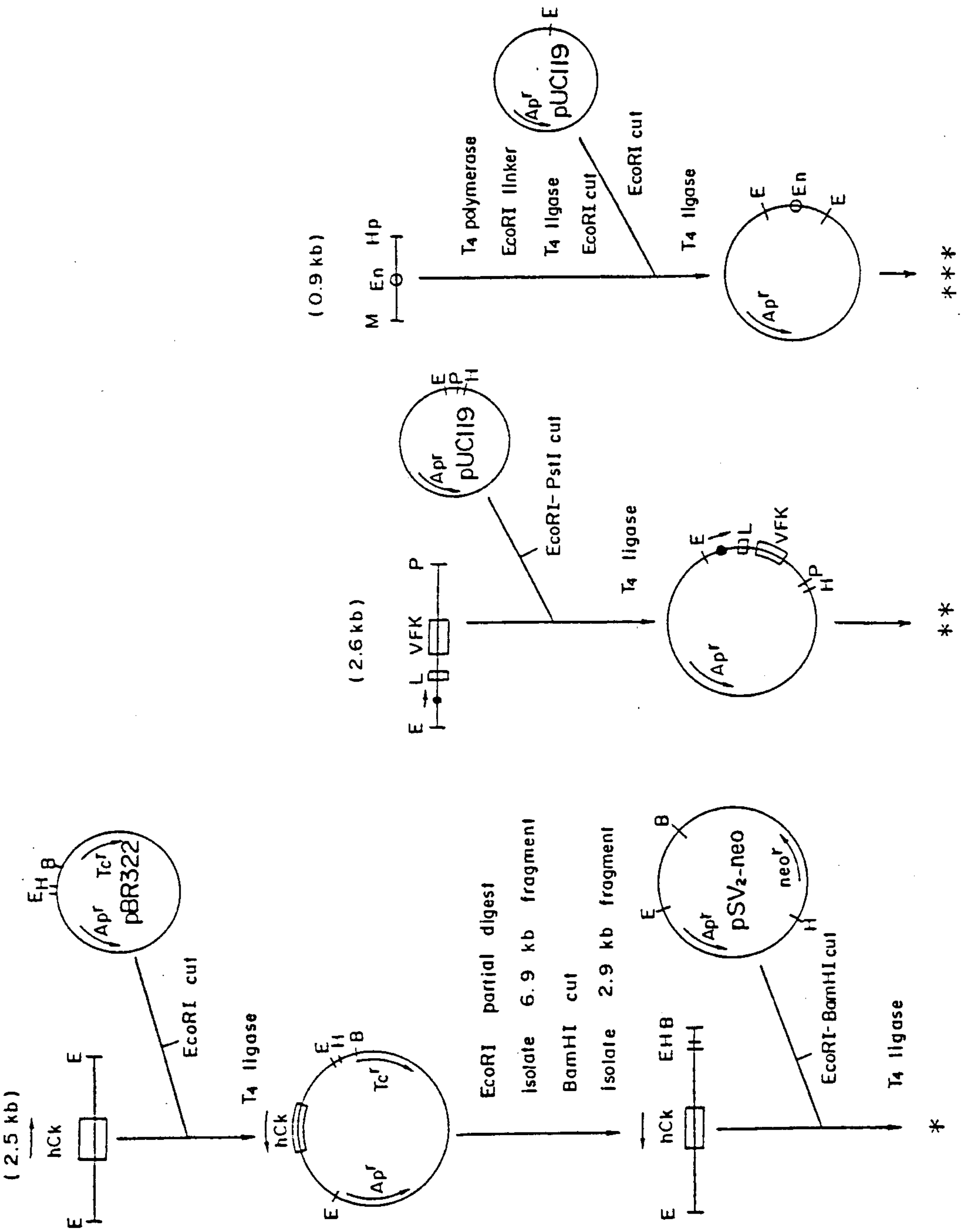
(2.5 kb)
hCk
E
E
Ap^r
Tc^r
pBR322
E H B
EcoRI cut
T4 ligase
hCk
Ap^r
Tc^r
E
E H B
EcoRI partial digest
Isolate 6.9 kb fragment
BamHI cut
Isolate 2.9 kb fragment
hCk
E
E H B
Ap^r
pSV2-neo
neo^r
E
B
H
EcoRI-BamHI cut
T4 ligase
*
(2.6 kb)
E L VFK P
Ap^r
pUC119
E P H
EcoRI-PstI cut
T4 ligase
Ap^r
E L VFK
H P
**
(0.9 kb)
M En Hp
T4 polymerase
EcoRI linker
T4 ligase
EcoRI cut
Ap^r
pUC119
E
EcoRI cut
T4 ligase
Ap^r
E En E
***

(Cont.)

Fig. 2-(2)

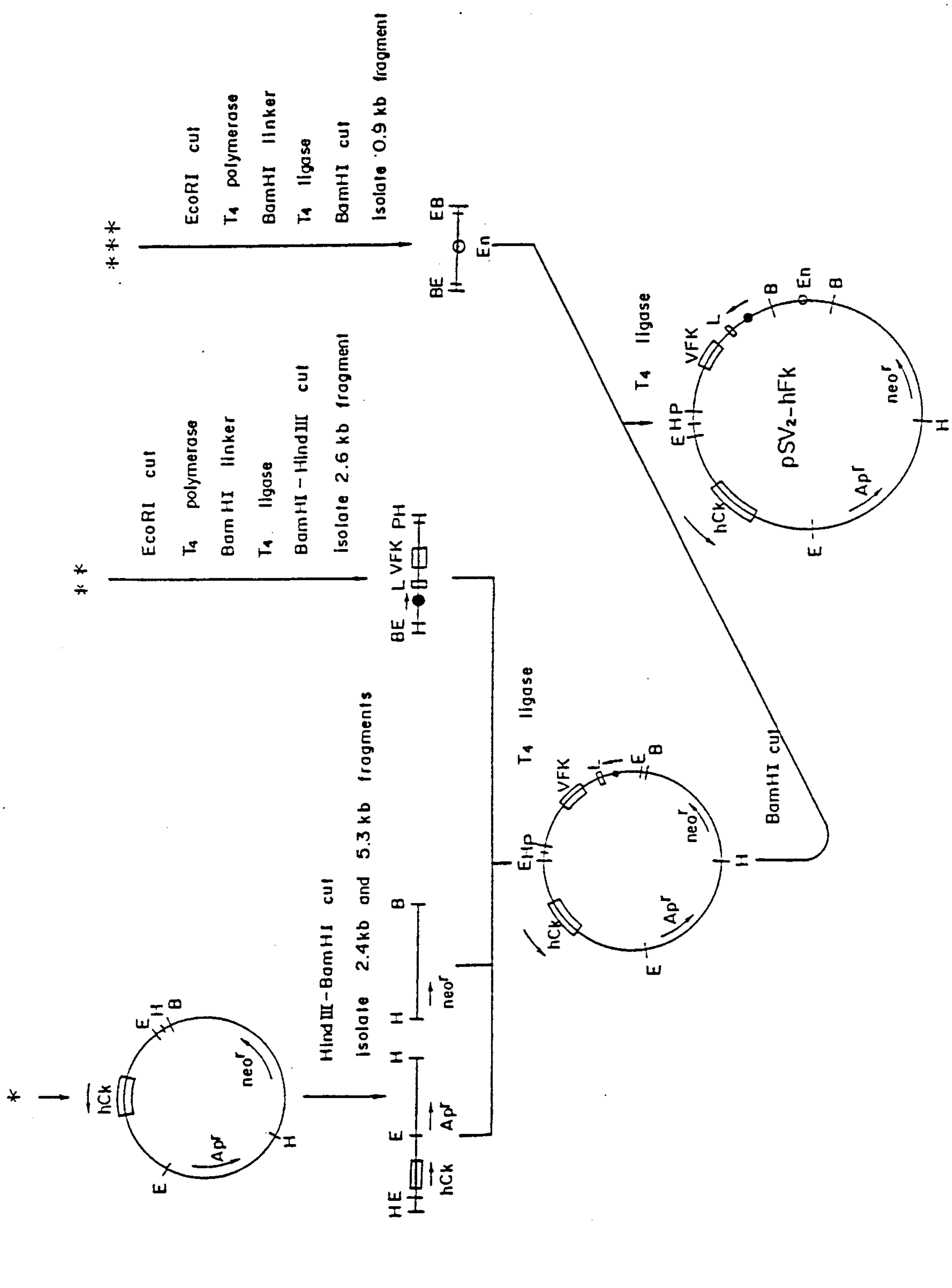
*
**
***
HindIII-BamHI cut
Isolate 2.4kb and 5.3 kb fragments
EcoRI cut
$T_4$ polymerase
BamHI linker
$T_4$ ligase
BamHI-HindIII cut
Isolate 2.6 kb fragment
EcoRI cut
$T_4$ polymerase
BamHI linker
$T_4$ ligase
BamHI cut
Isolate 0.9 kb fragment
$T_4$ ligase
$T_4$ ligase
BamHI cut
hCk
$neo^r$
$Ap^r$
VFK
L
En
$pSV_2$-hFk

Fig. 3

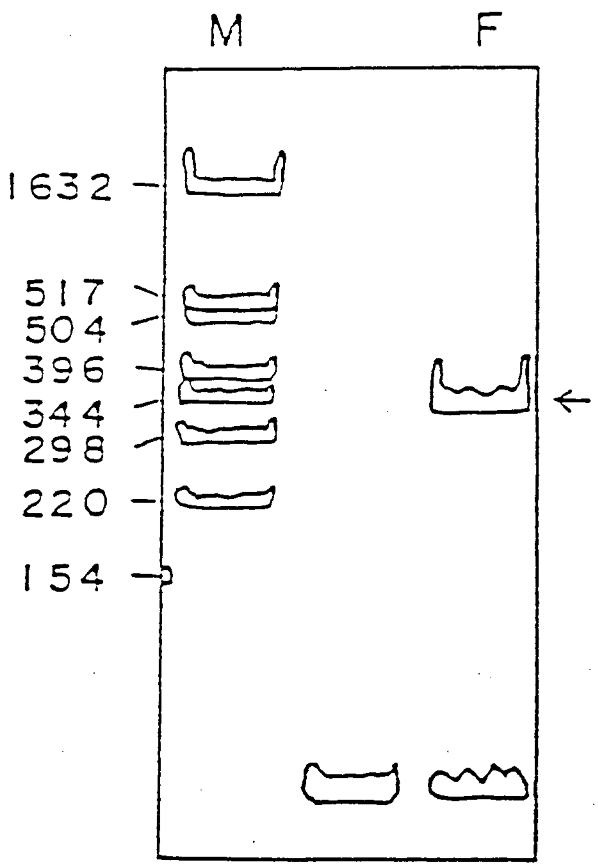

Fig. 4-(1)

```
                                                 GCATGCTATAGAGGAAGATATGCAAATA

ATTCTTCTCTGAGTTCATATAAACCAGCCCTGCCCCGAGTCTGTAGCTCTGACAGAGGAGCCAAGCCCTGGATTCCCAG
                                           ---------- Leader ----------
                                           -19
                                           Met Asp Ser Arg Leu Asn Leu Val Phe
GTCCTCACATTCAGTGATCAGCACTGAACACAGACCACTCACC ATG GAC TCC AGG CTC AAT TTA GTT TTC
-------------------------//

Leu Val Leu Ile Leu Lys G
CTT GTC CTT ATT TTA AAA G/GT AATTTGTAGAGATGAGTTTCTGCCTGTTGTGCCCAAGGGAAATAGAAAC
                                                              //--------------
                                                              ly Val Gln Cys   -1
ATTGTTTGTTTCATTATTTTATTTTGTTAGTAACAGTTTTCTGACCAGCATTCTCTGTTTGCA G/GT GTC CAG TGT   -1
```

(Cont.)

Fig. 4-(2)

```
                                    FR-1
1     VHIIIA<->VFII
Asp Val Gln|Leu Trp Glu Ser Gly Gly Gly Leu Val Lys Pro Gly Gly Ser Leu Lys Leu    20
GAT GTG CAG|CTG TGG GAG TCG GGG GGA GGC TTA GTG AAG CCT GGA GGG TCC CTG AAA CTC    60
        PvuII
                                            CDR-1
Ser Cys Ala Ala Ser Gly Phe Thr Phe Ser|Asn Tyr Asp Met Ser|Trp Val Arg Gln Thr    40
TCC TGT GCA GCC TCT GGA TTC ACT TTC AGT AAC TAT GAC ATG TCT TGG GTT CGC CAG ACT   120
         FR-2                                                  CDR-2
Pro Glu Arg Arg Leu Glu Trp Val Ala|Ser Ile Ser Val Gly Gly Thr Thr Tyr Tyr Pro    60
CCA GAG AGG AGG CTG GAG TGG GTC GCA TCC ATT AGT GTT GGT GGT ACC ACC TAC TAT CCA   180
                                                               FR-3
Asp Ser Met Lys Gly|Arg Phe Thr Ile Ser Arg Asp Asn Ala Arg Asn Ile Leu Tyr Leu    80
GAC AGT ATG AAG GGC CGA TTC ACC ATC TCC AGA GAT AAT GCC AGG AAC ATC CTG TAT CTG   240
                                                                   CDR-3
Gln Leu Ser Ser Leu Arg Ser Glu Asp Thr Ala Met Tyr Tyr Cys|Gly Asn Phe Ala Asp   100
CAA TTG AGC AGT CTG AGG TCT GAA GAC ACG GCC ATG TAT TAC TGT|GGT AAC TTC GCG GAT   300
                           FR-4                     VHIII<->   Dsp2
                         VFII <--|--> VHNLI
Ala Met Asp Tyr|Trp Gly Gln Gly Thr Leu Val Thr Val Ser Ser|                        115
GCT ATG GAC TAC TGG GGC CAA GGG ACC CTG GTC ACC GTC TCC TCA G/GT AAGCTGGCTTTT      360
JH4                                  BstPI
```

Fig. 5-(1)

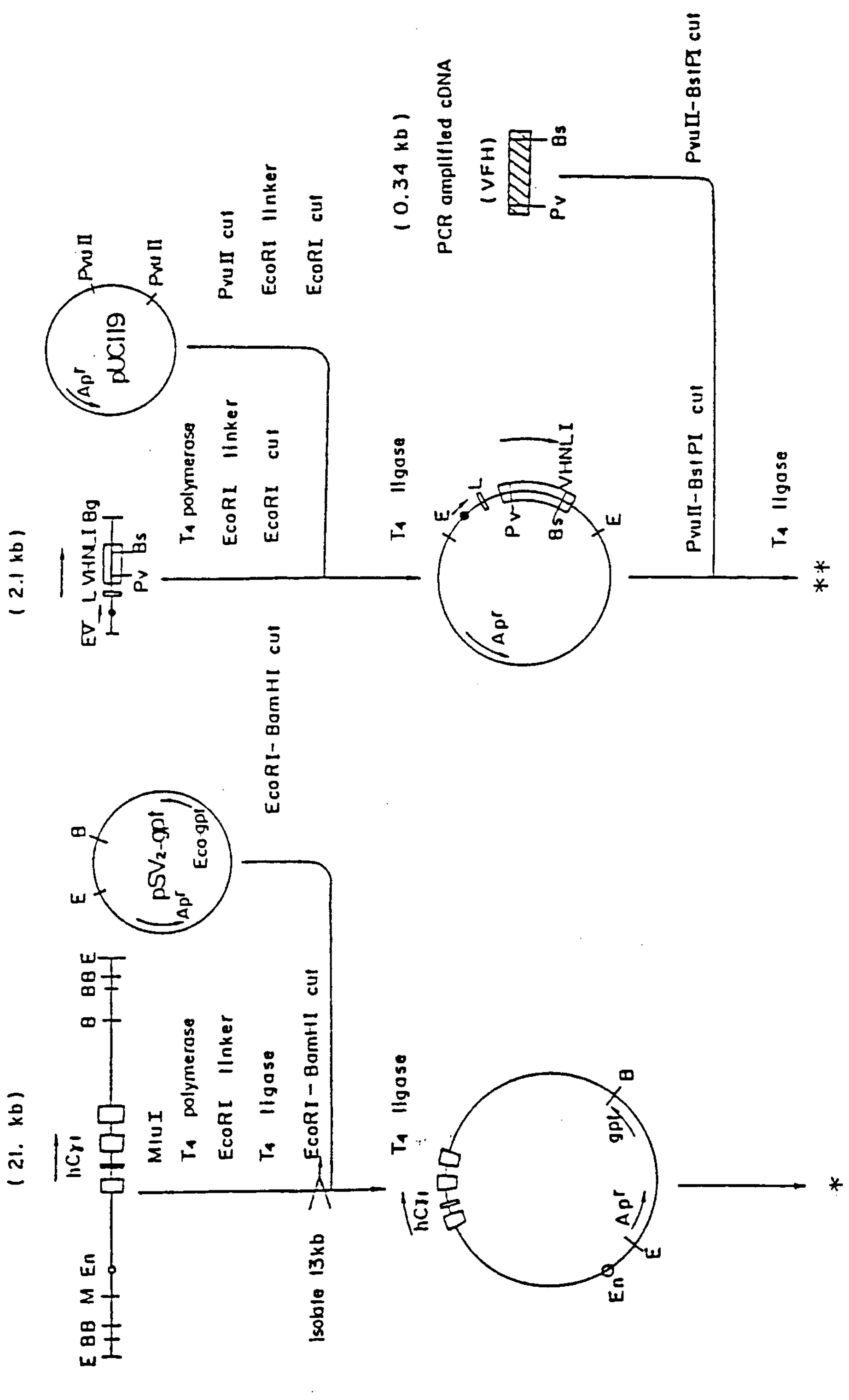
( 21. kb)
hCγ1
E BB M En
B BB E
pSV2-gpt
Eco-gpt
Apr
E
B
MluI
T4 polymerase
EcoRI linker
T4 ligase
EcoRI - BamHI cut
Isolate 13kb
T4 ligase
hCγ1
gpt
Apr
En
E
B
*
( 2.1 kb)
EV L VHNL I Bg
Pv Bs
pUC119
PvuII
T4 polymerase
EcoRI linker
EcoRI cut
EcoRI-BamHI cut
PvuII cut
EcoRI linker
EcoRI cut
T4 ligase
VHNL I
( 0.34 kb )
PCR amplified cDNA
( VFH )
Pv Bs
PvuII-BstPI cut
PvuII-BstPI cut
T4 ligase
**

(Cont.)

Fig. 5-(2)

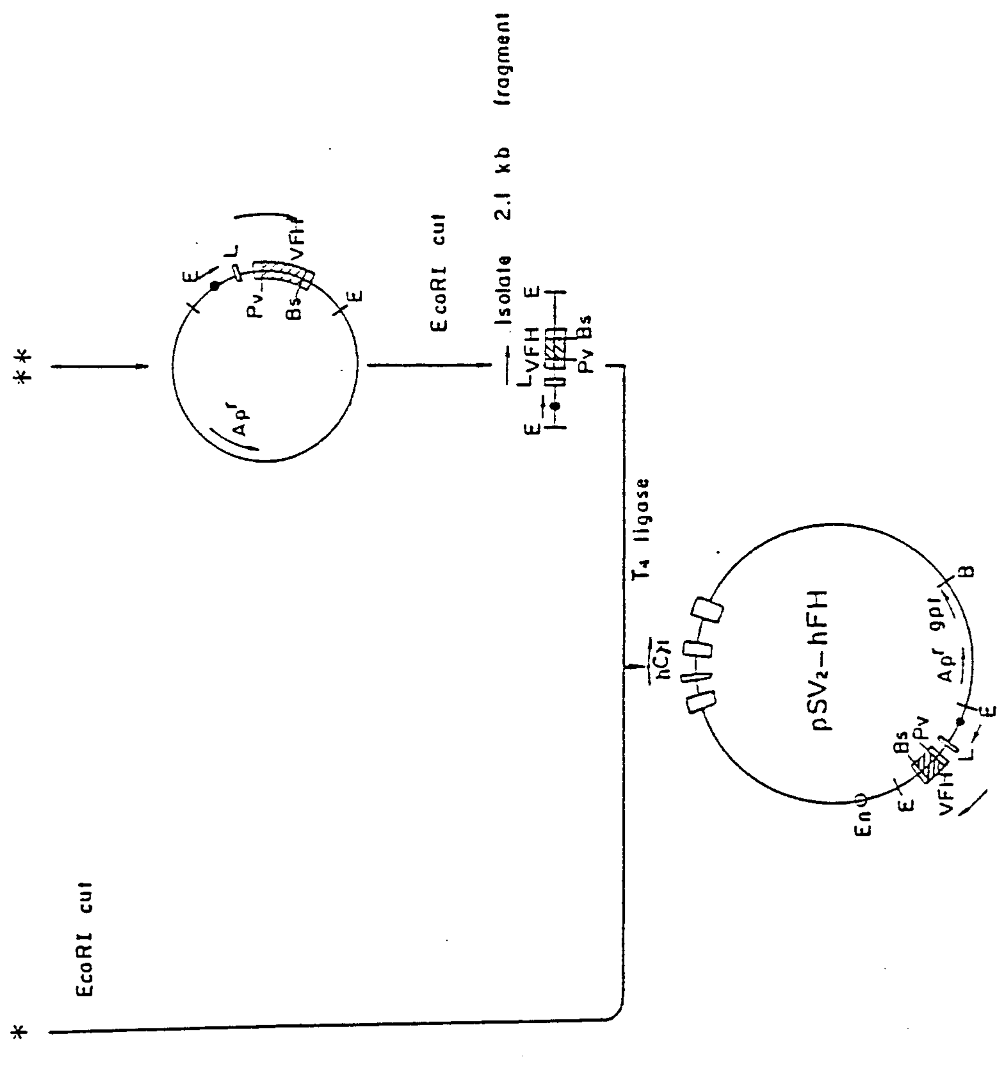
**
*
EcoRI cut
E coRI cut
Isolate 2.1 kb fragment
T4 ligase
pSV2-hFH
Apr
gpt
hCγ1
VFH
Pv
Bs
E
L
B
En

Fig. 6

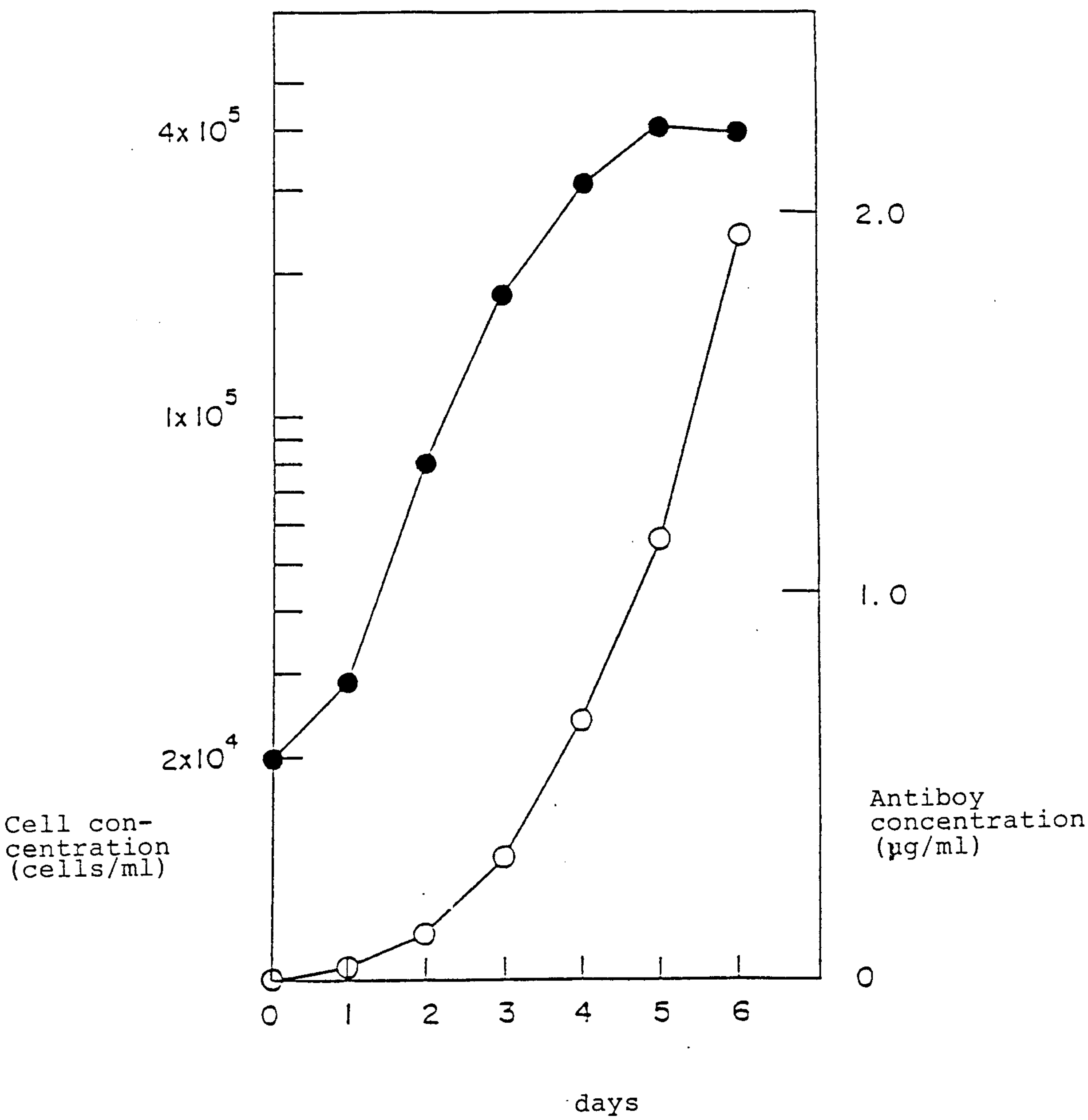
4x10^5
1x10^5
2x10^4
Cell concentration (cells/ml)
2.0
1.0
0
Antiboy concentration (µg/ml)
0
1
2
3
4
5
6
days

Fig. 7

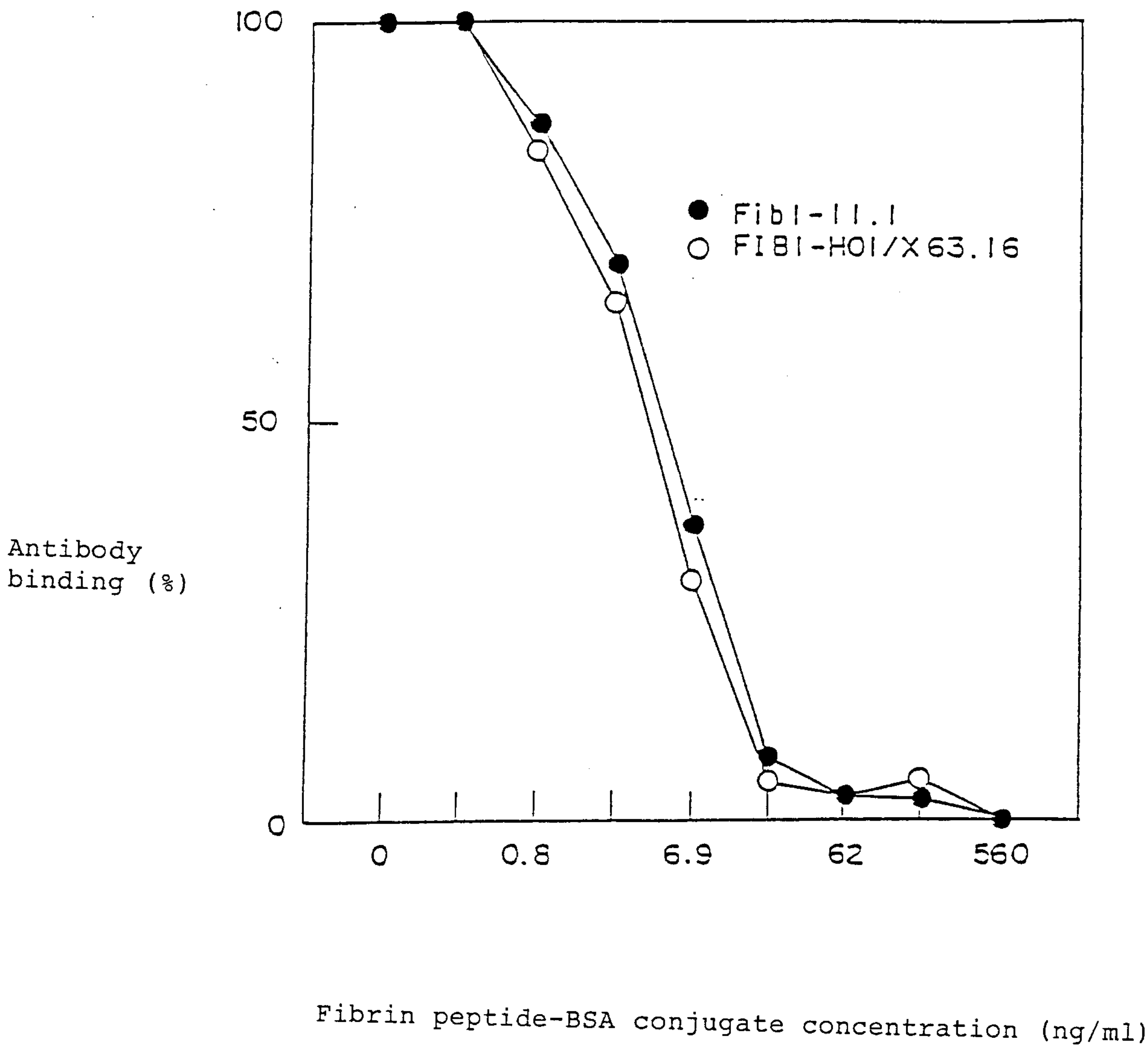
100
50
0
Antibody
binding (%)
0
0.8
6.9
62
560
Fibl-11.1
FIBI-HOI/X63.16
Fibrin peptide-BSA conjugate concentration (ng/ml)

Fig. 8-(1)

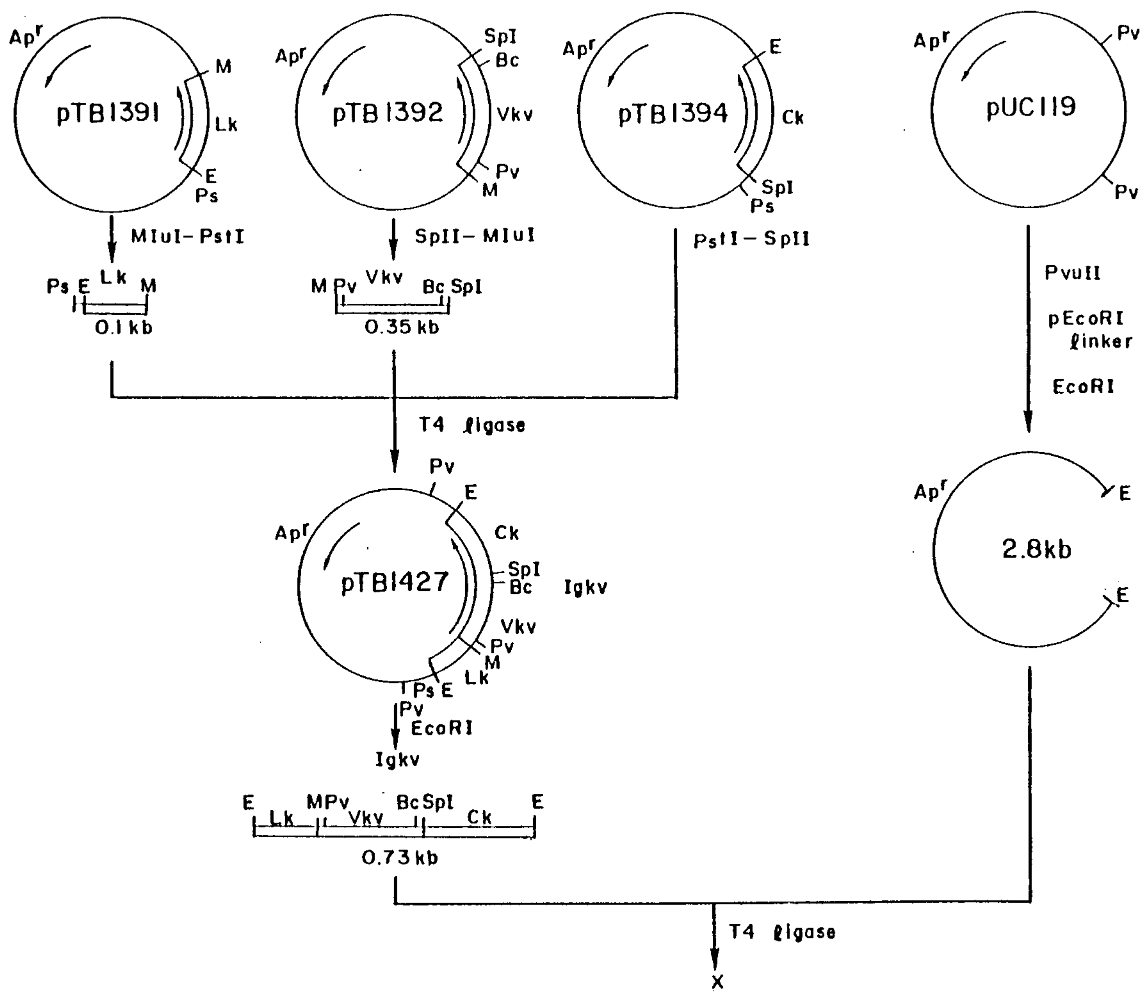
pTB1391
Apr
M
Lk
E
Ps
MluI-PstI
Ps E Lk M
0.1 kb
pTB1392
Apr
SpI
Bc
Vkv
Pv
M
SpII-MluI
M Pv Vkv Bc SpI
0.35 kb
pTB1394
Apr
E
Ck
SpI
Ps
PstI-SpII
pUC119
Apr
Pv
Pv
PvuII
pEcoRI linker
EcoRI
T4 ligase
pTB1427
Apr
Pv
E
Ck
SpI
Bc
Igkv
Vkv
Pv
M
Lk
Ps E
Pv
EcoRI
Igkv
E Lk MPv Vkv BcSpI Ck E
0.73 kb
2.8kb
Apr
E
E
T4 ligase
X

(Cont.)

Fig. 8-(2)

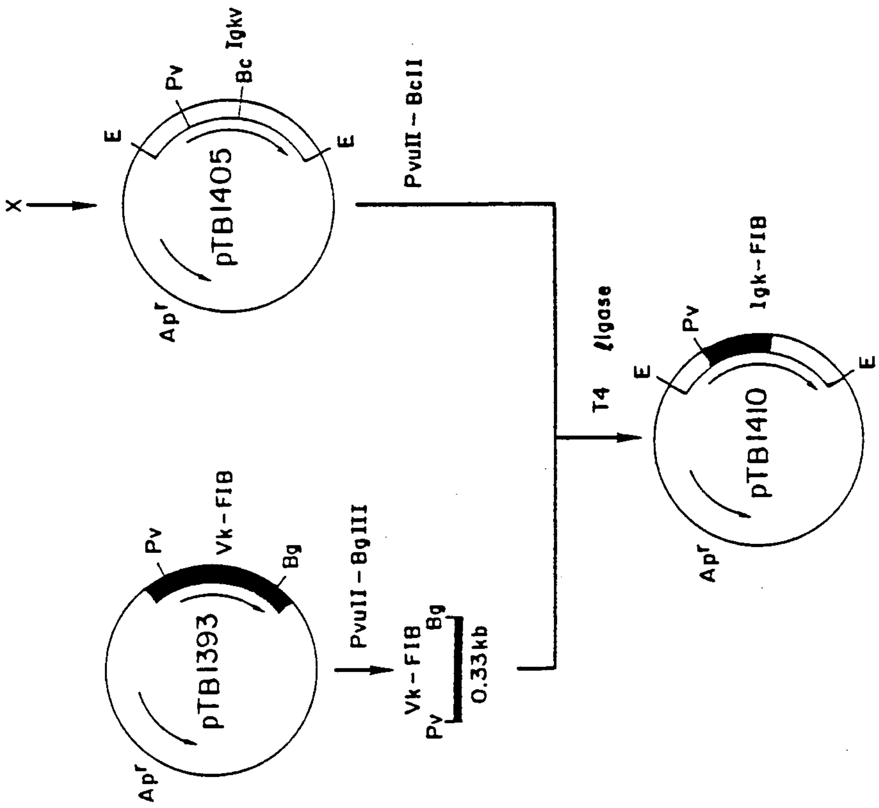

X
pTB1405
Ap^r
E
Pv
Bc
Igkv
E
PvuII - BclI
pTB1393
Ap^r
Pv
Vk-FIB
Bg
PvuII-BglII
Vk-FIB
Pv
Bg
0.33kb
T4 ligase
pTB1410
Ap^r
E
Pv
Igk-FIB
E

Fig. 9-(1)

```
                     Met Met Ser Pro Ala Gln Phe Leu Phe Leu Leu Val      12
 AG AAT TCC GCC ATG ATG AGT CCT GCC CAG TTC CTG TTT CTG TTA GTG      47
   EcoRI                      Leader <---> Vk

Leu Trp Ile Arg Glu Thr Arg Gly|Asp Ile Gln Leu Ala Gln Thr Pro      28
CTC TGG ATT CGG GAA ACG CGT GGT|GAT ATT CAG CTG GCC CAG ACT CCA      95
                     MluI                PvuII

Leu Thr Leu Ser Val Thr Ile Gly Gln Pro Ala Phe Ile Ser Cys Thr      44
CTC ACT TTG TCG GTT ACC ATT GGA CAA CCA GCC TTC ATC TCT TGC ACG     143

Ser Ser Gln Ser Leu Leu Asp Ser Asp Gly Lys Thr Tyr Leu Asn Trp      60
TCA AGT CAG AGC CTC TTA GAT AGT GAT GGA AAG ACA TAT TTG AAT TGG     191

Leu Leu Gln Arg Pro Gly Gln Ser Pro Lys Arg Leu Ile Tyr Leu Val      76
TTG TTA CAG AGG CCA GGC CAG TCT CCA AAG CGC CTA ATC TAT CTG GTG     239

Ser Lys Leu Tyr Ser Gly Val Pro Asp Arg Phe Thr Gly Ser Gly Ser      92
TCT AAA CTG TAC TCT GGA GTC CCT GAC AGG TTC ACT GGC AGT GGA TCA     287

Gly Thr Ala Phe Thr Leu Lys Ile Asn Arg Val Glu Ala Glu Asp Leu     108
GGG ACA GCT TTC ACA CTG AAA ATC AAC AGA GTG GAG GCT GAG GAT TTG     335

Gly Val Tyr Tyr Cys Trp Gln Gly Ile His Phe Pro Tyr Thr Phe Gly     124
GGA GTT TAT TAT TGC TGG CAA GGT ATA CAT TTT CCG TAC ACG TTC GGA     383
```

(Cont.)

Fig. 9-(2)

```
                          (Glu)             Vκ <------> Cκ
Gly Gly Thr Lys Leu Val Ile Lys Arg|Thr Val Ala Ala Pro Ser Val     140
GGG GGG ACC AAG CTG GTG ATC AAA CGT|ACG GTG GCT GCA CCA TCT GTC     431
                        BclI        SplI

Phe Ile Phe Pro Pro Ser Asp Glu Gln Leu Lys Ser Gly Thr Ala Ser     156
TTC ATC TTC CCG CCA TCT GAT GAG CAG TTG AAA TCT GGA ACT GCC TCT     479

Val Val Cys Leu Leu Asn Asn Phe Tyr Pro Arg Glu Ala Lys Val Gln     172
GTT GTG TGC CTG CTG AAT AAC TTC TAT CCC AGA GAG GCC AAA GTA CAG     527

Trp Lys Val Asp Asn Ala Leu Gln Ser Gly Asn Ser Gln Glu Ser Val     188
TGG AAG GTG GAT AAC GCC CTC CAA TCG GGT AAC TCC CAG GAG AGT GTC     575

Thr Glu Gln Asp Ser Lys Asp Ser Thr Tyr Ser Leu Ser Ser Thr Leu     204
ACA GAG CAG GAC AGC AAG GAC AGC ACC TAC AGC CTC AGC AGC ACC CTG     623

Thr Leu Ser Lys Ala Asp Tyr Glu Lys His Lys Val Tyr Ala Cys Glu     220
ACG CTG AGC AAA GCA GAC TAC GAG AAA CAC AAA GTC TAC GCC TGC GAA     671

Val Thr His Gln Gly Leu Ser Ser Pro Val Thr Lys Ser Phe Asn Arg     236
GTC ACC CAT CAG GGC CTG AGC TCG CCC GTC ACA AAG AGC TTC AAC CGC     719

Gly Glu Cys ***                                                     242
GGA GAG TGT TAG AAT TCT                                             737
                 EcoRI
```

Fig. 10-(1)

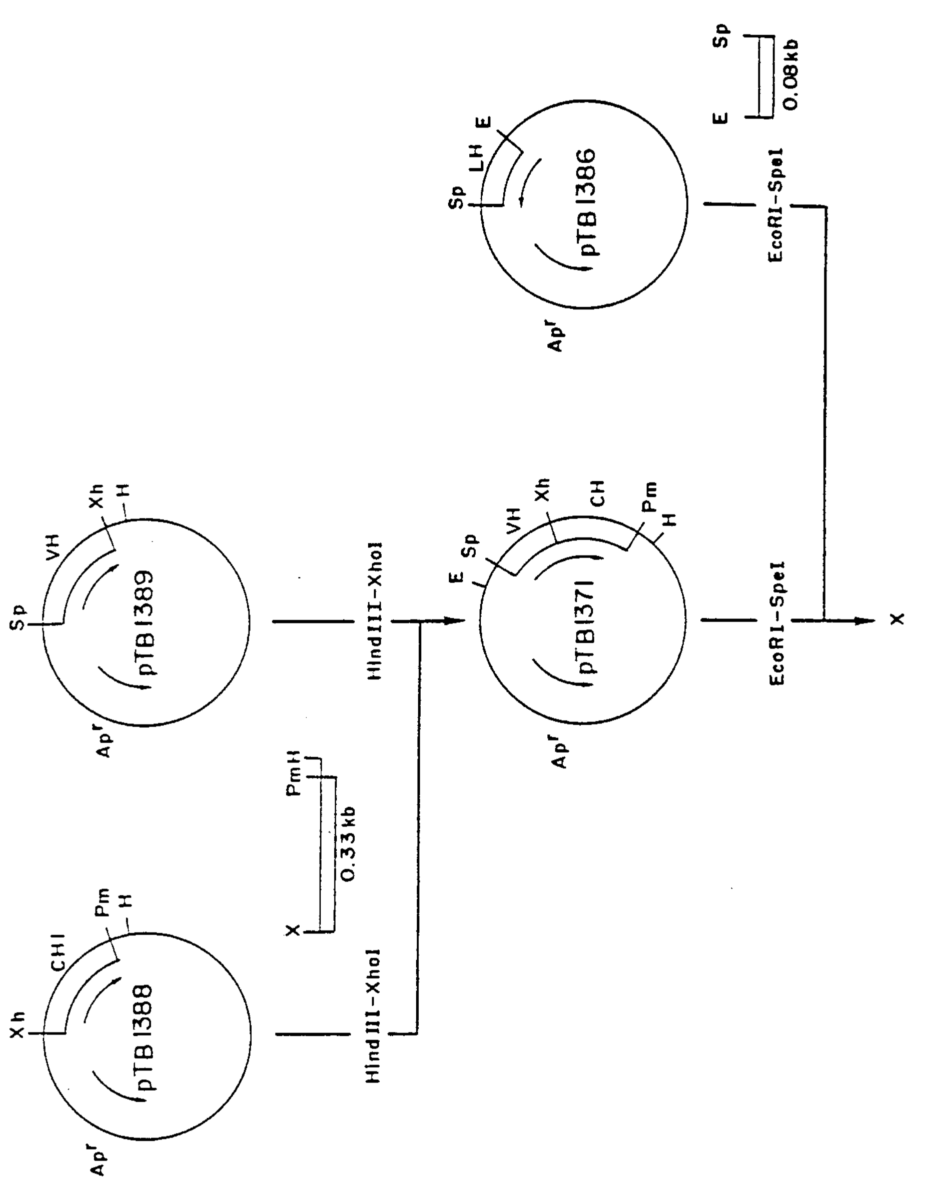
pTB1388
Xh
CHI
Pm
H
Apr
HindIII-XhoI
X
PmH
0.33kb
pTB1389
Sp
VH
Xh
H
Apr
HindIII-XhoI
pTB1371
E
Sp
VH
Xh
CH
Pm
H
Apr
EcoRI-SpeI
X
pTB1386
Sp
LH
E
Apr
EcoRI-SpeI
E
Sp
0.08kb

(Cont.)

Fig. 10-(2)

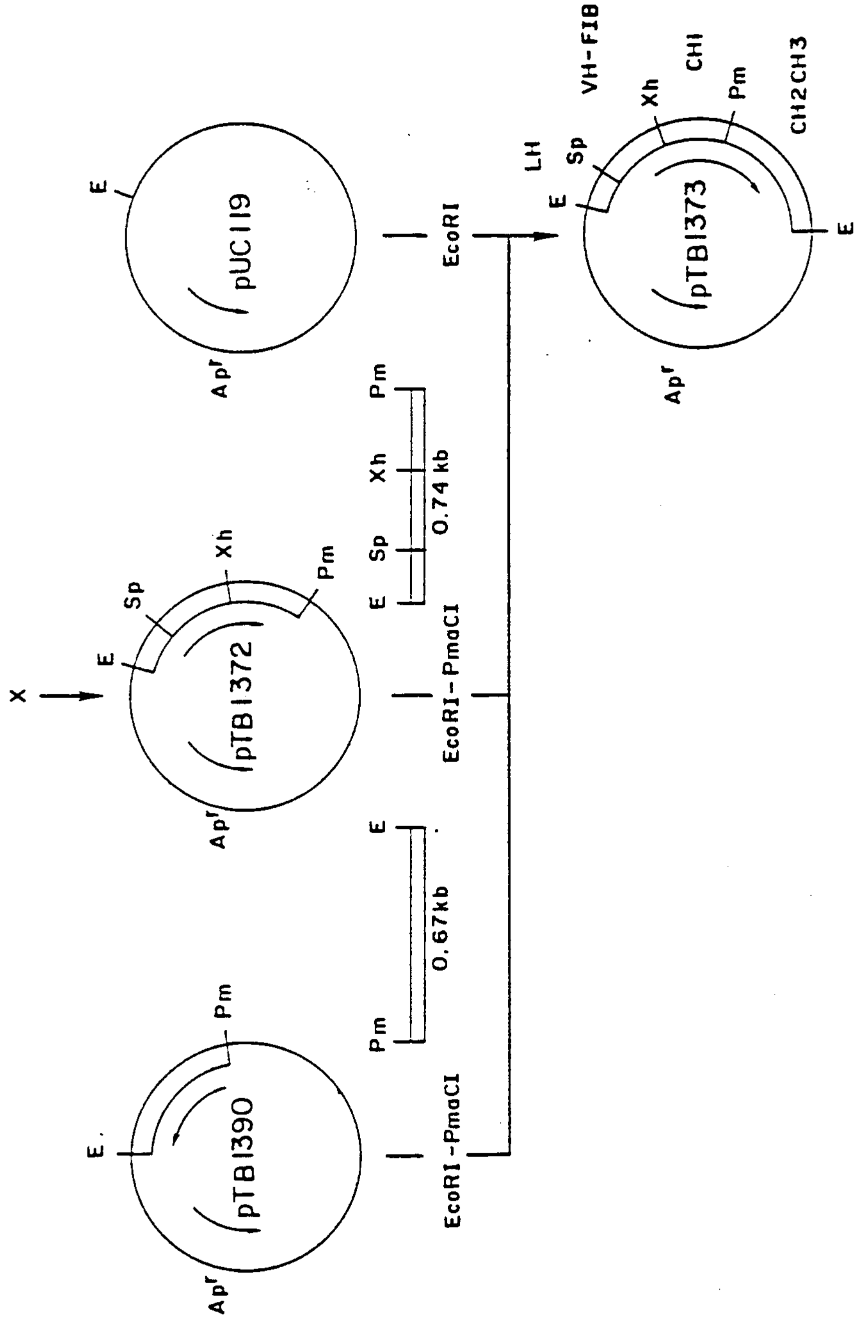
pTB1390
E
Pm
Apr
EcoRI-PmaCI
Pm
0.67kb
E
X
pTB1372
E
Sp
Xh
Pm
Apr
EcoRI-PmaCI
E
Sp
Xh
Pm
0.74 kb
pUC119
E
Apr
EcoRI
pTB1373
LH
VH-FIB
CH1
CH2CH3
E
Sp
Xh
Pm
E
Apr

Fig. 11-(1)

```
                        Met Asp Ser Arg Leu Asn Leu Val Phe Leu Val Leu      12
CTG AAT TCC ACC ATG GAC TCC AGG CTC AAT TTA GTT TTC CTT GTC CTT      48
     EcoRI                Leader ←→ VH
Ile Leu Lys Gly Val Asp Cys|Glu Val Gln Leu Val Glu Ser Gly Gly      28
ATT TTA AAA GGT GTC GAC TGT|GAG GTG CAA CTA GTG GAG TCG GGG GGA      96
                 SalI                   SpeI
Gly Leu Val Lys Pro Gly Gly Ser Leu Lys Leu Ser Cys Ala Ala Ser      44
GGC TTA GTG AAG CCT GGA GGG TCC CTG AAA CTC TCC TGT GCA GCC TCT     144

Gly Phe Thr Phe Ser Asn Tyr Asp Met Ser Trp Val Arg Gln Thr Pro      60
GGA TTC ACT TTC AGT AAC TAT GAC ATG TCT TGG GTT CGC CAG ACT CCA     192

Glu Arg Arg Leu Glu Trp Val Ala Ser Ile Ser Val Gly Gly Thr Thr      76
GAG AGG AGG CTG GAG TGG GTC GCA TCC ATT AGT GTT GGT GGT ACC ACC     240

Tyr Tyr Pro Asp Ser Met Lys Gly Arg Phe Thr Ile Ser Arg Asp Asn      92
TAC TAT CCA GAC AGT ATG AAG GGC CGA TTC ACC ATC TCC AGA GAT AAT     288

Ala Arg Asn Ile Leu Tyr Leu Gln Leu Ser Ser Leu Arg Ser Glu Asp     108
GCC AGG AAC ATC CTG TAT CTG CAA TTG AGC AGT CTG AGG TCT GAA GAC     336

Thr Ala Met Tyr Tyr Cys Gly Asn Phe Ala Asp Ala Met Asp Tyr Trp     124
ACG GCC ATG TAT TAC TGT GGT AAC TTC GCG GAT GCT ATG GAC TAC TGG     384
```

(Cont.)

Fig. 11-(2)

```
                                                VH ←─┬─→ CH1
Gly Gln Gly Thr Leu Val Thr Val Ser Ser|Ala Ser Thr Lys Gly Pro     140
GGC CAA GGG ACC CTG GTC ACC GTC TCG AGT|GCT AGC ACC AAG GGC CCA     432
                                XhoI     NheI
Ser Val Phe Pro Leu Ala Pro Ser Ser Lys Ser Thr Ser Gly Gly Thr     156
TCG GTC TTC CCC CTG GCA CCC TCC TCC AAG AGC ACC TCT GGG GGC ACA     480

Ala Ala Leu Gly Cys Leu Val Lys Asp Tyr Phe Pro Glu Pro Val Thr     172
GCG GCC CTG GGC TGC CTG GTC AAG GAC TAC TTC CCC GAA CCG GTG ACG     528

Val Ser Trp Asn Ser Gly Ala Leu Thr Ser Gly Val His Thr Phe Pro     188
GTG TCG TGG AAC TCA GGC GCC CTG ACC AGC GGC GTG CAC ACC TTC CCG     576

Ala Val Leu Gln Ser Ser Gly Leu Tyr Ser Leu Ser Ser Val Val Thr     204
GCT GTC CTA CAG TCC TCA GGA CTC TAC TCC CTC AGC AGC GTG GTG ACC     624

Val Pro Ser Ser Ser Leu Gly Thr Gln Thr Tyr Ile Cys Asn Val Asn     220
GTG CCC TCC AGC AGC TTG GGC ACC CAG ACC TAC ATC TGC AAC GTG AAT     672
                                                 CH1 ←─┬─→ hinge
His Lys Pro Ser Asn Thr Lys Val Asp Lys Arg Val|Glu Pro Lys Ser     236
CAC AAG CCC AGC AAC ACC AAG GTG GAC AAG AGA GTT|GAG CCC AAA TCT     720
                                     hinge ←─┬─→ CH2
Cys Asp Lys Thr His Thr Cys Pro Pro Cys Pro|Ala Pro Glu Leu Leu     252
TGT GAC AAA ACT CAC ACG TGT CCA CCG TGC CCG|GCG CCT GAA CTC CTG     768
                    PamCI                    BbeI
```

(Cont.)

Fig. 11-(3)

```
Gly Gly Pro Ser Val Phe Leu Phe Pro Pro Lys Pro Lys Asp Thr Leu      268
GGG GGA CCG TCA GTC TTC CTC TTC CCC CCA AAA CCC AAG GAC ACC CTC      816

Met Ile Ser Arg Thr Pro Glu Val Thr Cys Val Val Val Asp Val Ser      284
ATG ATC TCC CGG ACC CCT GAG GTC ACA TGC GTG GTG GTG GAC GTG AGC      864

His Glu Asp Pro Glu Val Lys Phe Asn Trp Tyr Val Asp Gly Val Glu      300
CAC GAA GAC CCT GAG GTC AAG TTC AAC TGG TAC GTG GAC GGC GTG GAG      912

Val His Asn Ala Lys Thr Lys Pro Arg Glu Glu Gln Tyr Asn Ser Thr      316
GTG CAT AAT GCC AAG ACA AAG CCG CGG GAG GAG CAG TAC AAC AGC ACG      960

Tyr Arg Val Val Ser Val Leu Thr Val Leu His Gln Asp Trp Leu Asn      332
TAC CGT GTG GTC AGC GTC CTC ACC GTC CTG CAC CAG GAC TGG CTG AAT     1008

Gly Lys Glu Tyr Lys Cys Lys Val Ser Asn Lys Ala Leu Pro Ala Pro      348
GGC AAG GAG TAC AAG TGC AAG GTC TCC AAC AAA GCC CTC CCA GCC CCC     1056
                                CH2 <--|--> CH3
Ile Glu Lys Thr Ile Ser Lys Ala Lys|Gly Gln Pro Arg Glu Pro Gln      364
ATC GAG AAA ACC ATC TCC AAA GCC AAA|GGG CAG CCC CGA GAA CCA CAG     1104

Val Tyr Thr Leu Pro Pro Ser Arg Glu Glu Met Thr Lys Asn Gln Val      380
GTG TAC ACC CTG CCC CCA TCC CGG GAG GAG ATG ACC AAG AAC CAG GTC     1152
```

(Cont.)

Fig. 11-(4)

```
Ser Leu Thr Cys Leu Val Lys Gly Phe Tyr Pro Ser Asp Ile Ala Val      396
AGC CTG ACC TGC CTG GTC AAA GGT TTC TAT CCC AGC GAC ATC GCC GTG     1200

Glu Trp Glu Ser Asn Gly Gln Pro Glu Asn Asn Tyr Lys Thr Thr Pro      412
GAG TGG GAG AGC AAT GGG CAG CCG GAG AAC AAC TAC AAG ACC ACG CCT     1248

Pro Val Leu Asp Ser Asp Gly Ser Phe Phe Leu Tyr Ser Lys Leu Thr      428
CCC GTG CTG GAC TCC GAC GGC TCC TTC TTC CTC TAT AGC AAG CTC ACC     1296

Val Asp Lys Ser Arg Trp Gln Gln Gly Asn Val Phe Ser Cys Ser Val      444
GTG GAC AAG AGC AGG TGG CAG CAG GGG AAC GTC TTC TCA TGC TCC GTG     1344

Met His Glu Ala Leu His Asn His Tyr Thr Gln Lys Ser Leu Ser Leu      460
ATG CAT GAG GCT CTG CAC AAC CAC TAC ACG CAG AAG AGC CTC TCC CTG     1392

Ser Pro Gly Lys *** Ile                                              466
TCC CCG GGT AAA TGA ATT CG                                          1412
    SmaI            EcoRI
```

Fig. 12

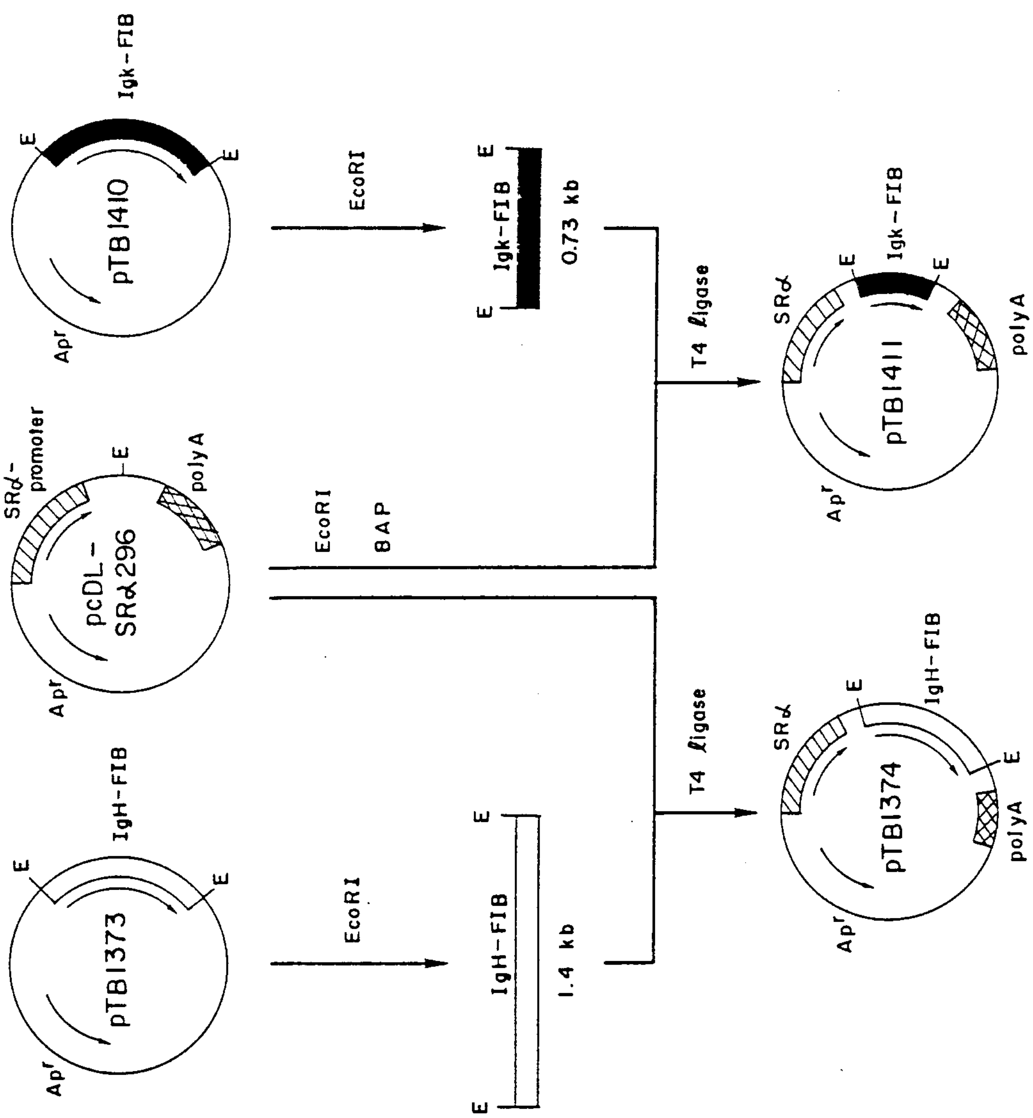
pTB1410
Igk-FIB
E
Apr
EcoRI
Igk-FIB
0.73 kb
pcDL-
SRα296
SRα-
promoter
polyA
EcoRI
BAP
T4 ℓigase
pTB1411
pTB1373
IgH-FIB
1.4 kb
pTB1374
polyA

Fig. 13-(1)

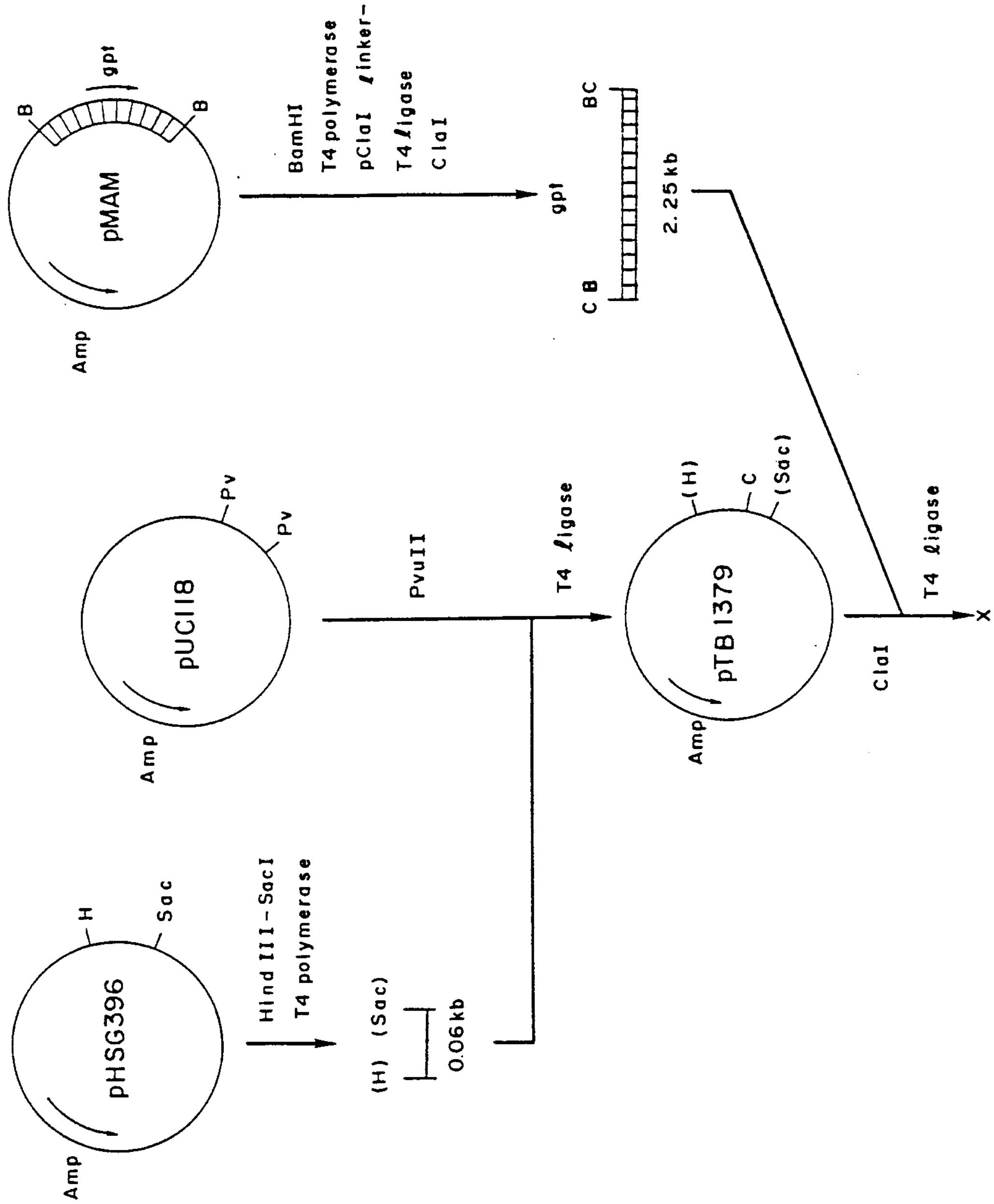
pHSG396
Amp
H
Sac
Hind III - SacI
T4 polymerase
(H) (Sac)
0.06kb
pUC118
Amp
Pv
Pv
PvuII
T4 ℓigase
pTB1379
Amp
(H)
C
(Sac)
ClaI
T4 ℓigase
X
pMAM
Amp
B
gpt
B
BamHI
T4 polymerase
pClaI ℓinker-
T4 ℓigase
ClaI
gpt
C B
BC
2.25kb

(Cont.)

Fig. 13-(2)

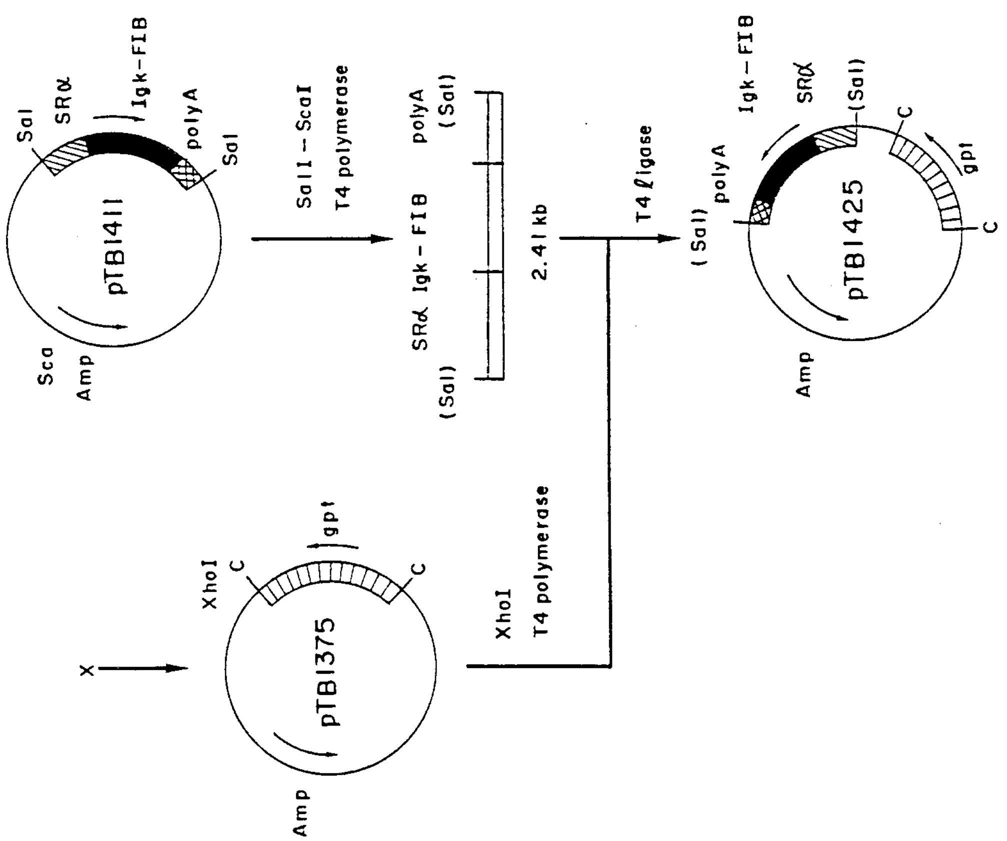
X
pTB1375
Amp
XhoI
C
gpt
C
XhoI
T4 polymerase
pTB1411
Sca
Amp
Sal
SRα
Igk-FIB
polyA
Sal
SalI - ScaI
T4 polymerase
(Sal)
SRα Igk - FIB
polyA
(Sal)
2.41 kb
T4 ℓigase
pTB1425
Amp
(Sal)
polyA
Igk - FIB
SRα
(Sal)
C
gpt
C

(Cont.)

Fig. 13-(3)

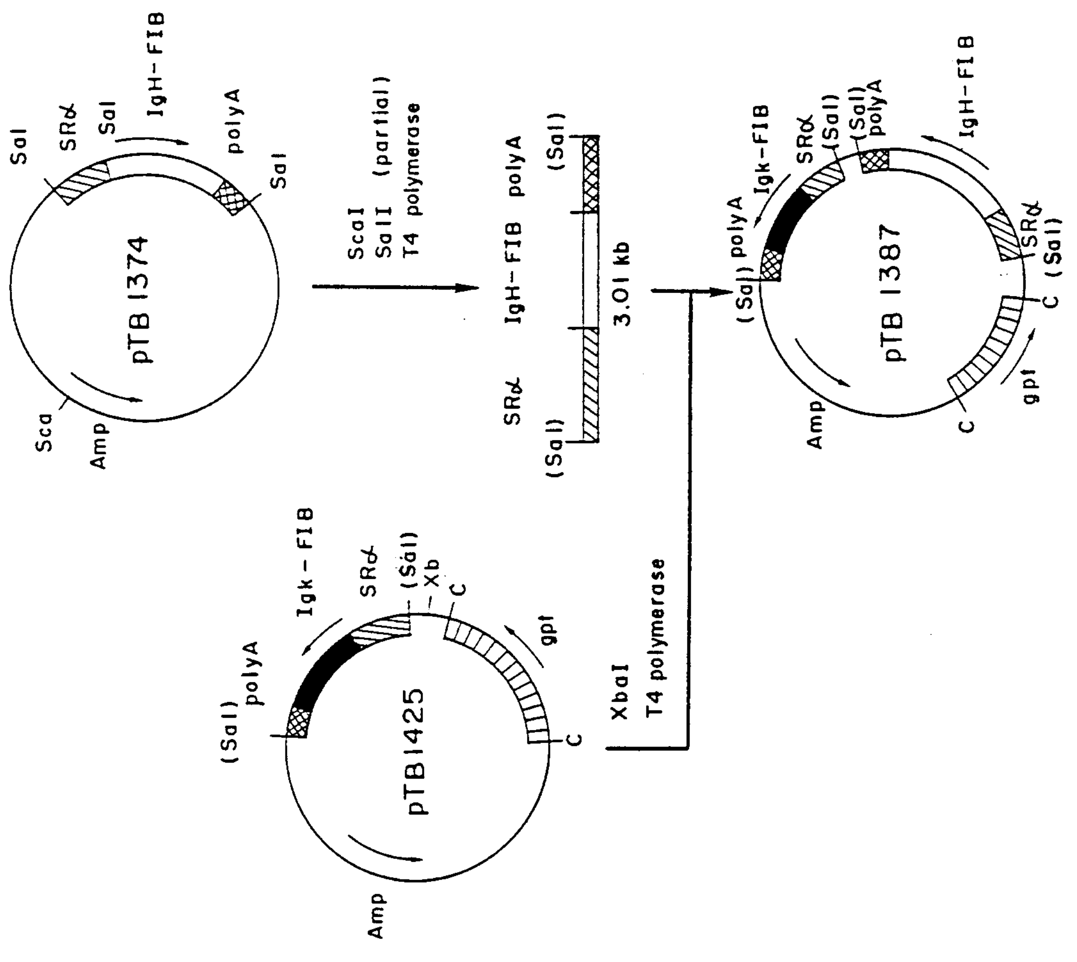
pTB 1374
Sal
SRα
Sal
IgH-FIB
polyA
Sal
Sca
Amp
ScaI
SalI (partial)
T4 polymerase
(Sal)
SRα
IgH-FIB
polyA
(Sal)
3.01 kb
pTB1425
(Sal)
polyA
Igk-FIB
SRα
(Sal)
Xb
C
gpt
C
Amp
XbaI
T4 polymerase
pTB 1387
(Sal) polyA
Igk-FIB
SRα
(Sal)
(Sal)
polyA
IgH-FIB
SRα
(Sal)
C
gpt
C
Amp

Fig. 14-(1)

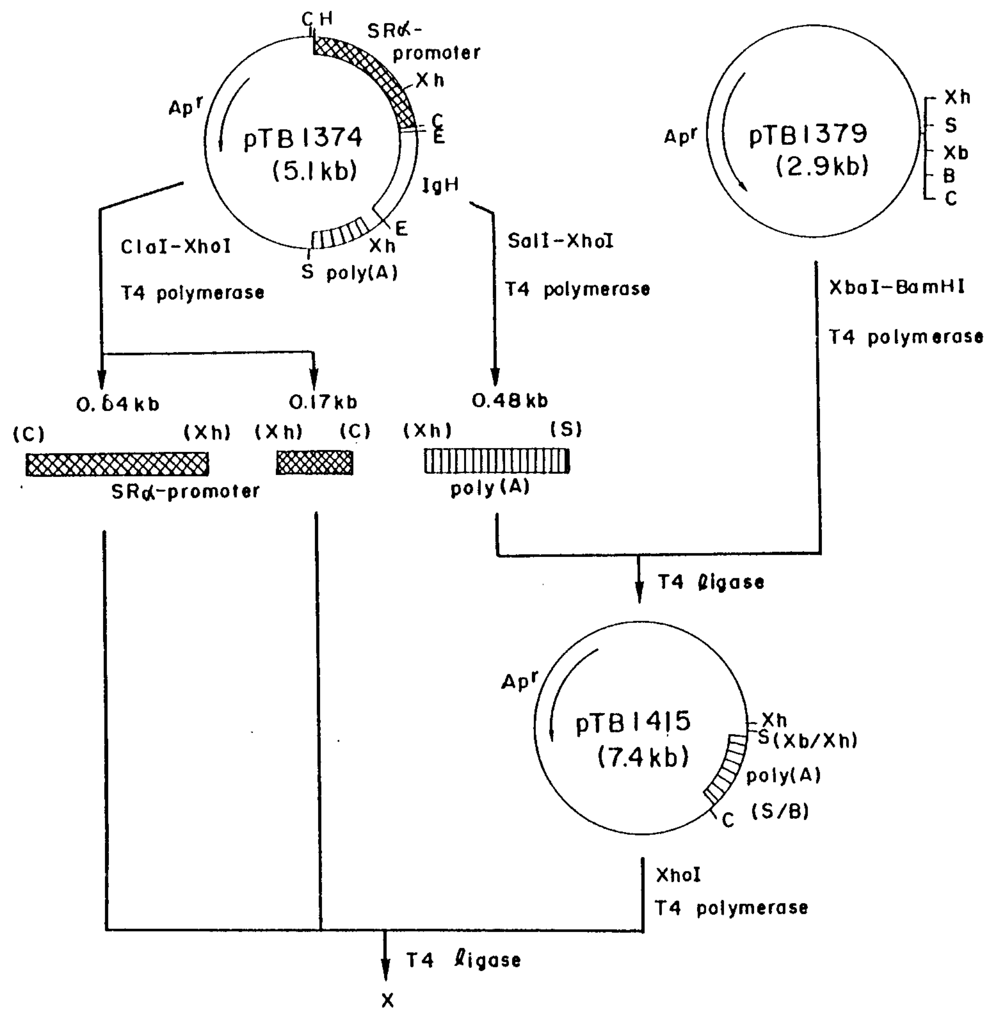

pTB1374
(5.1kb)
Apr
C H
SRα-
promoter
Xh
C
E
IgH
E
Xh
S poly(A)
ClaI-XhoI
T4 polymerase
SalI-XhoI
T4 polymerase
pTB1379
(2.9kb)
Apr
Xh
S
Xb
B
C
XbaI-BamHI
T4 polymerase
0.64kb
(C)
(Xh)
SRα-promoter
0.17kb
(Xh)
(C)
0.48kb
(Xh)
(S)
poly(A)
T4 ligase
pTB1415
(7.4kb)
Apr
Xh
S(Xb/Xh)
poly(A)
(S/B)
C
XhoI
T4 polymerase
T4 ligase
X


(Cont.)

Fig. 14-(2)

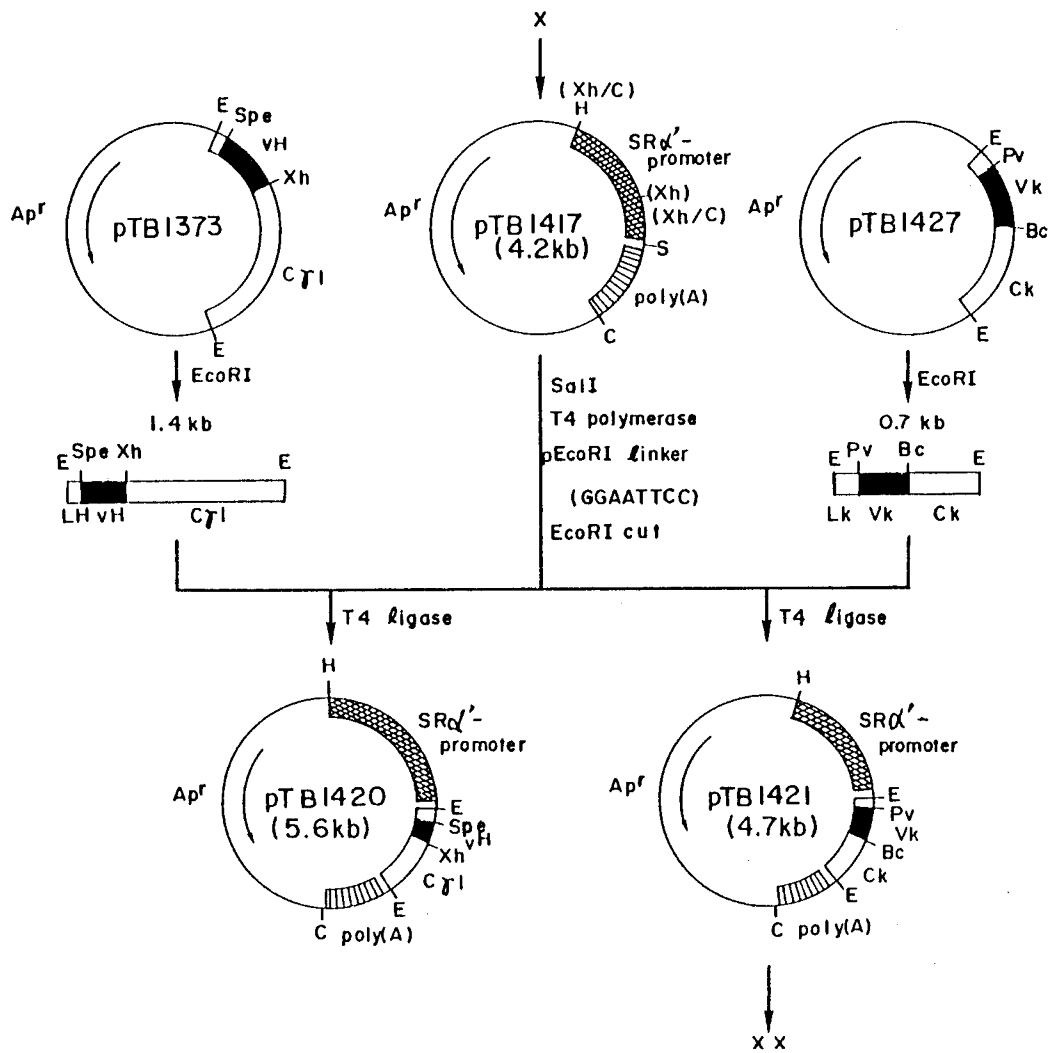
X
(Xh/C)
H
SRα'-promoter
(Xh)
(Xh/C)
S
poly(A)
C
pTB1417
(4.2kb)
Apr
pTB1373
E
Spe
vH
Xh
Cγ1
EcoRI
1.4 kb
LH vH
pTB1427
Pv
Vk
Bc
Ck
0.7 kb
Lk
SalI
T4 polymerase
pEcoRI linker
(GGAATTCC)
EcoRI cut
T4 ligase
pTB1420
(5.6kb)
pTB1421
(4.7kb)
X X

(Cont.)

Fig. 14-(3)

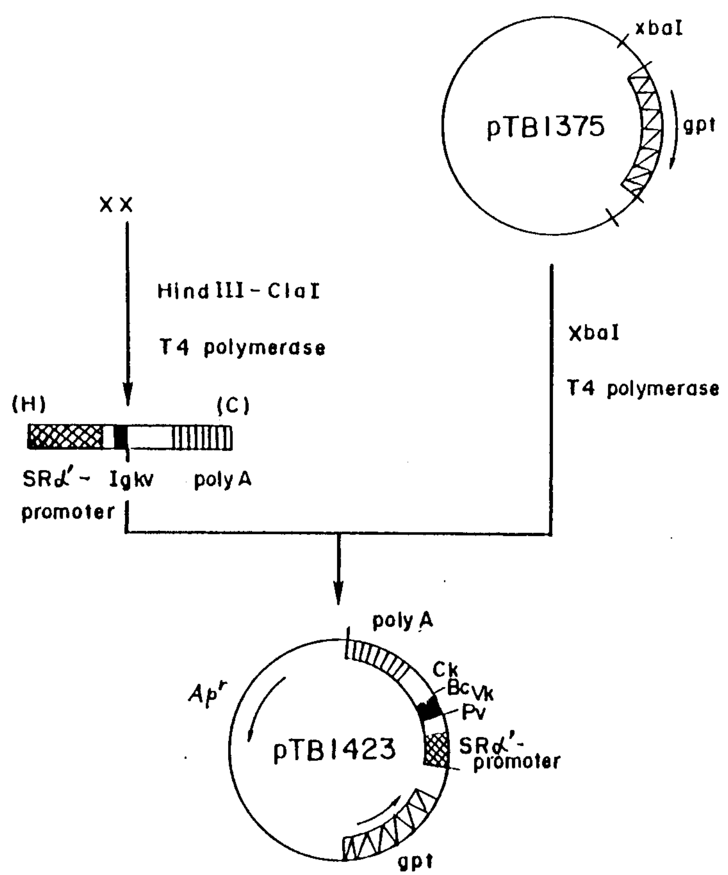
pTB1375
xbaI
gpt
XX
HindIII-ClaI
T4 polymerase
XbaI
T4 polymerase
(H)
(C)
SRα'- Igkv
promoter
poly A
pTB1423
poly A
Ck
Bc
Vk
Pv
SRα'-
promoter
gpt
$Ap^r$

Fig. 15

```
  1  Val Asp Cys Glu Val Gln Leu Val Glu Ser Gly Pro Gly Leu Val Lys   16
  1  GTC GAC TGT GAG GTG CAA CTA GTG GAG TCG GGA CCT GGC CTG GTG AAA   48
      SalI      VH01 primer   SpeI       5'VH1 primer
 17  Pro Ser Gln Ser Leu Ser Leu Thr Cys Thr Val Thr Gly Tyr Ser Ile   32
 49  CCT TCT CAG TCT CTG TCC CTC ACC TGC ACT GTC ACT GGC TAC TCA ATC   96
                  CDR 1
 33  Thr Ser Asp Tyr Ala Trp Asn Trp Ile Arg Gln Phe Pro Gly Asn Lys   48
 97  ACC AGT GAT TAT GCC TGG AAC TGG ATC CGG CAG TTT CCA GGA AAC AAA  144
                                                         CDR 2
 49  Leu Glu Trp Met Gly Tyr Ile Asn Tyr Ser Gly Thr Thr Ser Tyr Asn   64
145  CTG GAG TGG ATG GGC TAC ATA AAC TAC AGT GGT ACC ACT AGT TAC AAC  192
                                                  SpeI
 65  Pro Ser Leu Lys Ser Arg Ile Ser Ile Thr Arg Asp Thr Ser Asn Asn   80
193  CCA TCT CTC AAA AGT CGA ATC TCT ATC ACT CGA GAC ACA TCC AAT AAC  240
                                          XhoI
 81  Gln Phe Phe Leu Gln Leu Asn Ser Val Thr Ser Glu Asp Thr Ala Thr   96
241  CAG TTC TTC CTG CAG TTG AAT TCT GTG ACT TCT GAG GAC ACT GCC ACA  288
                                                  CDR 3
 97  Tyr Tyr Cys Ala Arg Leu Gly Asp Phe Asp Ala Gly Asp Tyr Phe Asp  112
289  TAT TAC TGT GCA AGA TTG GGT GAT TTC GAC GCG GGT GAC TAC TTT GAC  336
                                                 VHI-D       JH2
113  Tyr Trp Gly Gln Gly Thr Thr Val Thr Val Ser Ser Ala Ser          126
337  TAC TGG GGC CAA GGG ACC ACG GTC ACC GTC TCG AGT GCT AGC          378
         3'mVH primer       JH01 primer     XhoI    NheI
```

Fig. 16

```
  1  Asp Ile Gln Leu Thr Gln Ser Pro Ala Leu Met Ser Ala Val Pro Gly   16
  1  GAT ATT CAG CTG ACA CAG TCT CCA GCA CTC ATG TCT GCA GTT CCA GGG   48
             PvuII   5'mVκ primer          CDR 1
 17  Glu Lys Val Thr Met Thr Cys Ser Ala Ser Ser Ser Val Gly Tyr Met   32
 49  GAG AAG GTC ACC ATG ACC TGC AGT GCC AGC TCA AGT GTA GGT TAC ATG   96

 33  Tyr Trp Tyr Gln Gln Lys Pro Arg Ser Ser Pro Lys Pro Trp Ile Ser   48
 97  TAT TGG TAT CAG CAG AAG CCA AGA TCC TCC CCC AAG CCC TGG ATT TCT  144
             CDR 2
 49  Leu Thr Ser Asn Leu Ala Ser Gly Val Pro Ala Arg Phe Ser Gly Ser   64
145  CTC ACA TCC AAC CTG GCT TCT GGA GTC CCT GCT CGC TTC AGT GGC AGT  192

 65  Gly Ser Gly Thr Ser Tyr Ser Leu Thr Ile Ser Ser Met Glu Ala Glu   80
193  GGG TCT GGG ACC TCT TAC TCT CTC ACC ATC AGC AGC ATG GAG GCT GAA  240
                                        CDR 3
 81  Asp Ala Ala Thr Tyr Tyr Cys Gln Gln Trp Ser Ser Asp Pro Pro Thr   96
241  GAT GCT GCC ACT TAT TAC TGC CAG CAG TGG AGT AGT GAC CCA CCC ACG  288

 97  Phe Gly Gly Gly Thr Lys Leu Glu Ile Lys Arg                      107
289  TTC GGA GGG GGG ACC AAG CTG GAG ATC AAA CGT                      321
                 3'mVκ primer     BglII/BclI
```

Fig. 17

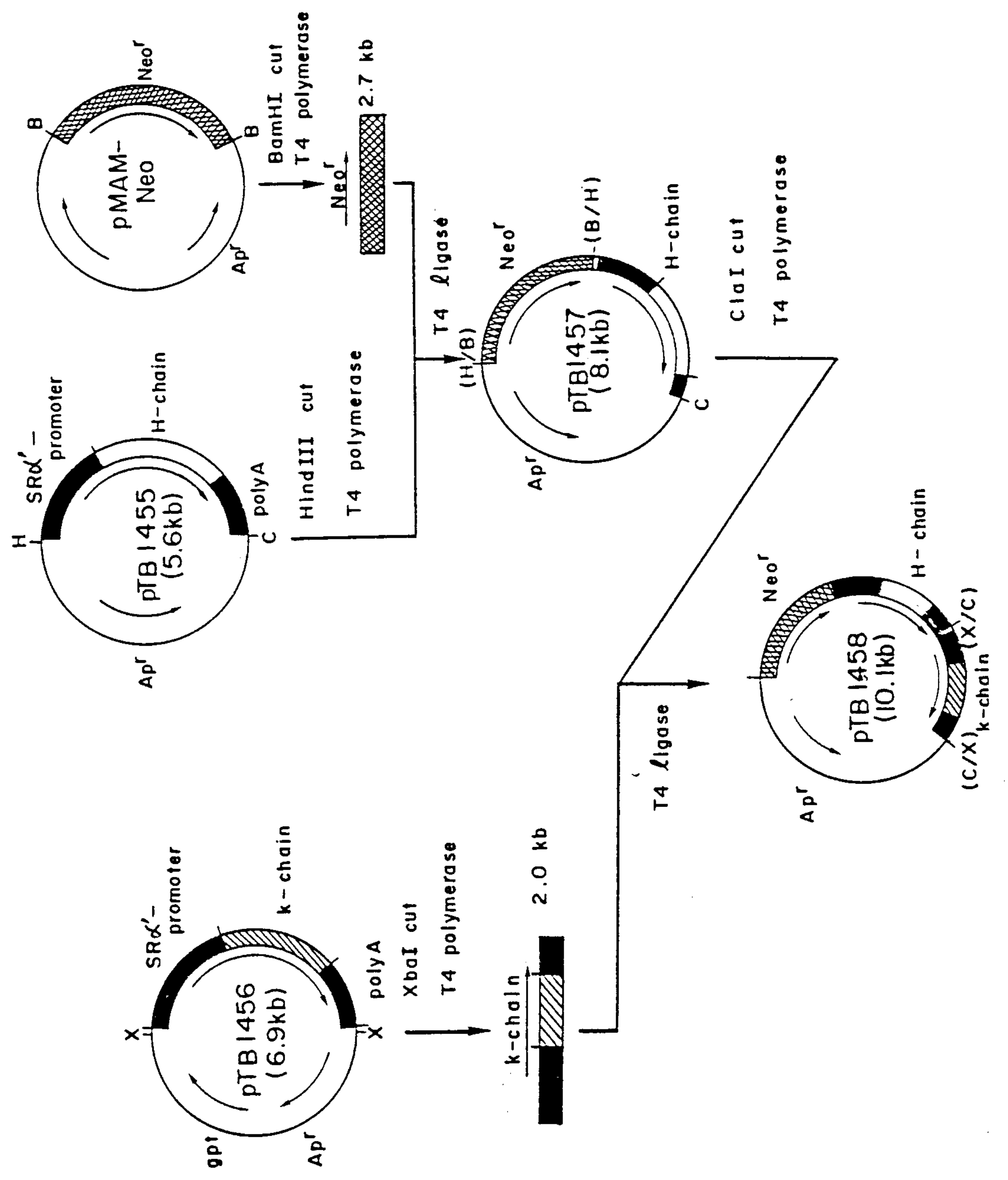
pMAM-Neo
B
B
Neo$^r$
Ap$^r$
BamHI cut
T4 polymerase
Neo$^r$
2.7 kb
pTB1455 (5.6kb)
H
SRα'-promoter
H-chain
polyA
C
Ap$^r$
HindIII cut
T4 polymerase
T4 ℓigase
pTB1457 (8.1kb)
(H/B)
Neo$^r$
(B/H)
H-chain
C
Ap$^r$
ClaI cut
T4 polymerase
pTB1456 (6.9kb)
X
SRα'-promoter
k-chain
polyA
X
gpt
Ap$^r$
XbaI cut
T4 polymerase
k-chain
2.0 kb
T4 ℓigase
pTB1458 (10.1kb)
Neo$^r$
H-chain
(X/C)
k-chain
(C/X)
Ap$^r$

Fig. 18

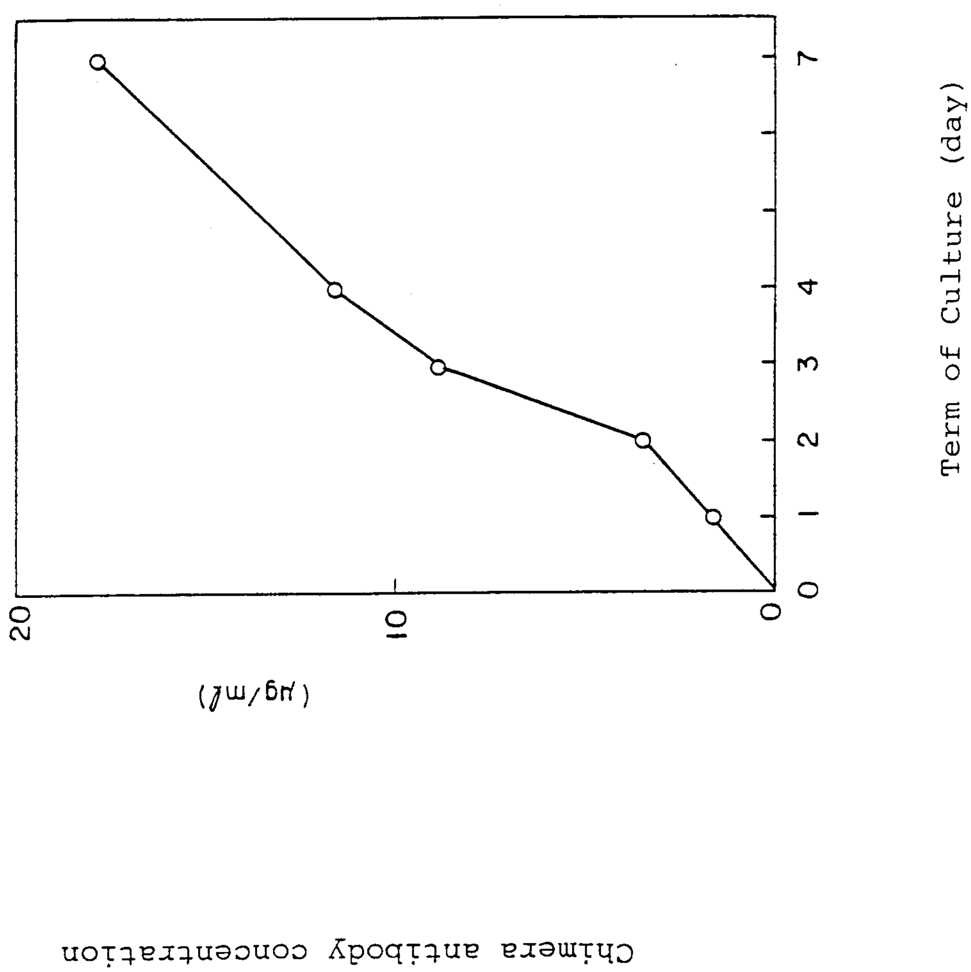
Chimera antibody concentration
(μg/mℓ)
20
10
0
0
1
2
3
4
7
Term of Culture (day)

Fig. 19

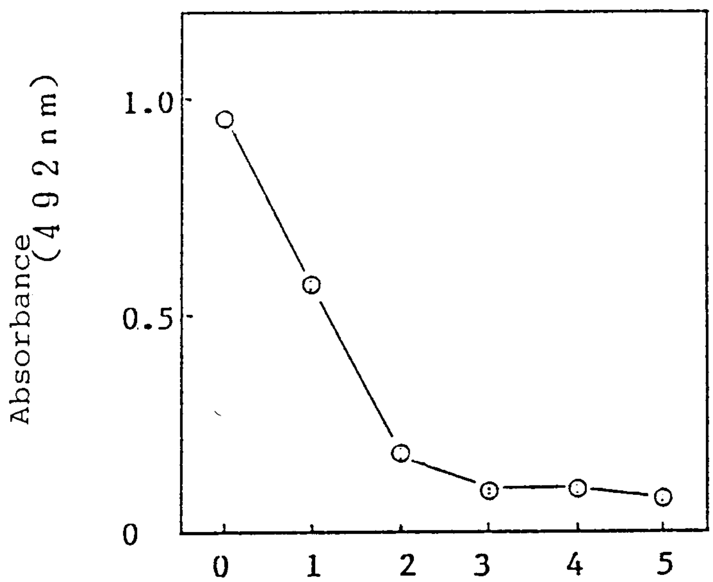


Dilution rate ($3^n$)

Fig. 20

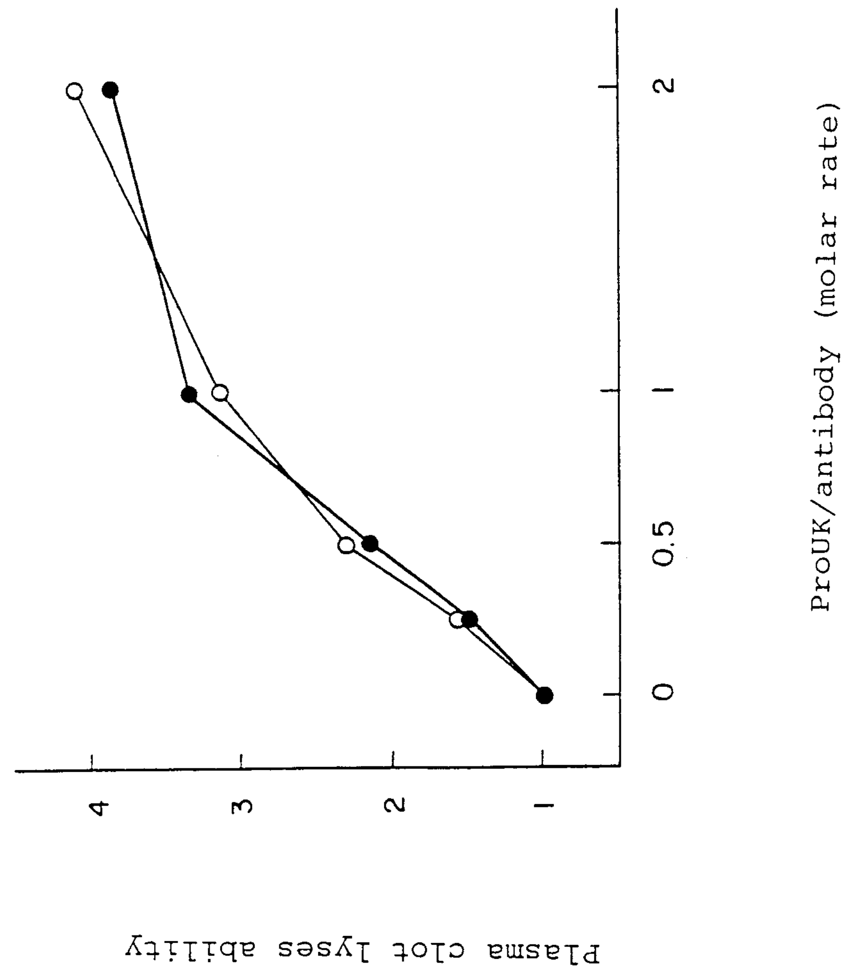
Plasma clot lyses ability
4
3
2
1
0
0.5
1
2
ProUK/antibody (molar rate)

Fig. 21

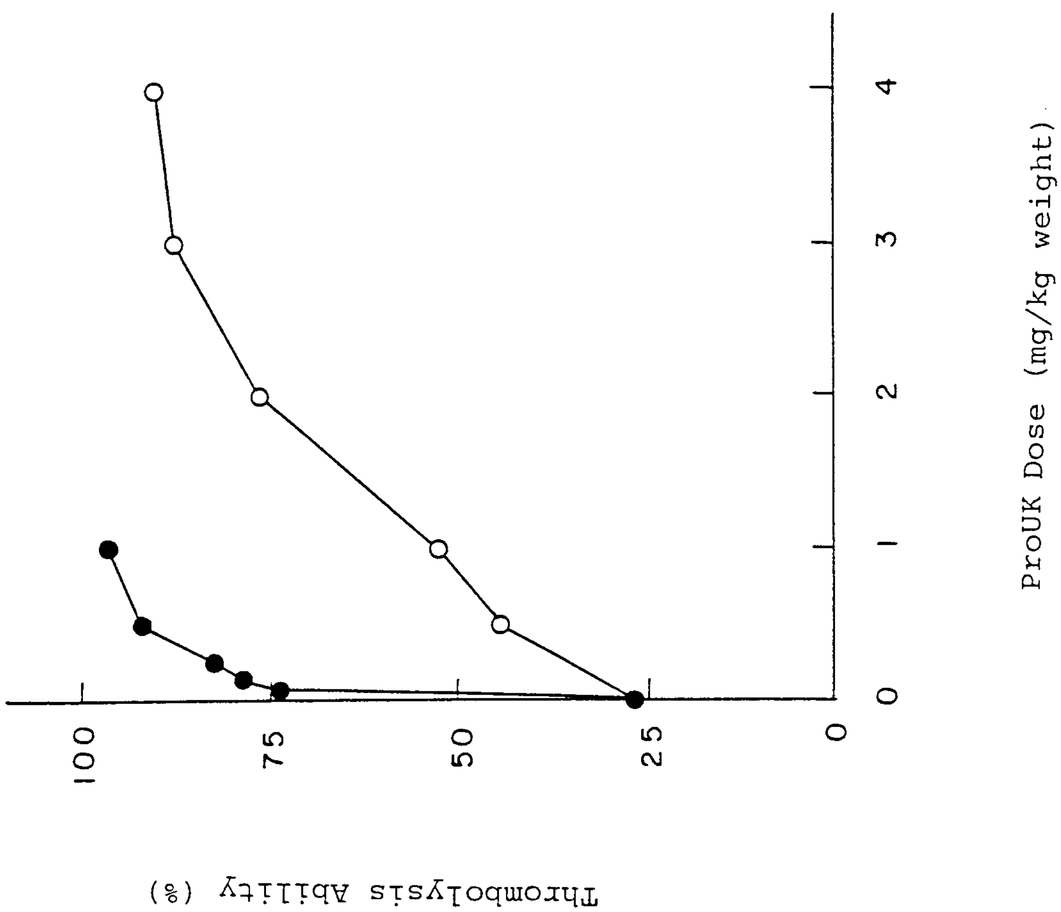
Thrombolysis Ability (%)
100
75
50
25
0
0
1
2
3
4
ProUK Dose (mg/kg weight)